# EUROPEAN PATENT SPECIFICATION

(11) **EP 2 664 676 B1**
(45) Date of publication and mention of the grant of the patent: **17.05.2017**
(21) Application number: 12734228.5
(22) Date of filing: 11.01.2012
(51) Int. Cl.: C12Q 1/68, A23C 9/152

(54) **METHOD FOR SCREENING FOR DIET OR SUBSTANCE PROVIDING PRODUCTION OF MILK HAVING IMMUNOREGULATORY ACTION**
VERFAHREN ZUM SCREENING AUF DIÄT ODER STOFF ZUR ERMÖGLICHUNG DER HERSTELLUNG EINER MILCH MIT IMMUNMODULIERENDER WIRKUNG
PROCÉDÉ DE CRIBLAGE POUR UN RÉGIME ALIMENTAIRE OU SUBSTACE QUI PERMETTENT LA PRODUCTION DE LAIT AYANT UNE ACTIVITÉ IMMUNOMODULATRICE

(30) Priority: 12.01.2011 JP 2011004090
(43) Date of publication of application: 20.11.2013
(73) Proprietor: Morinaga Milk Industry Co., Ltd., Minato-ku Tokyo 108-8384 (JP)
(72) Inventor: IZUMI, Hirohisa, Zama-shi Kanagawa 252-8583 (JP); SHIMIZU, Takashi, Zama-shi Kanagawa 252-8583 (JP); SEKINE, Kazunori, Zama-shi Kanagawa 252-8583 (JP)
(74) Representative: Henderson, Helen Lee
(86) International application number: PCT/JP2012/050312
(87) International publication number: WO 2012/096272

(56) References cited:
- JP-A- 2003 199 598
- ZUBIETA ANA CLAUDIA ET AL: "Effect of suboptimal nutrition during lactation on milk protein gene expression in the rat.", THE JOURNAL OF NUTRITIONAL BIOCHEMISTRY SEP 2006, vol. 17, no. 9, September 2006 (2006-09), pages 604-610, XP002738885, ISSN: 0955-2863
- CATHERINE CLINTON: "Development of the Infant Immune Function and the Effects of Breast Milk", NATURAL MEDICINE JOURNAL, vol. 2, no. 8, 1 January 2010 (2010-01-01) , pages 3-6, XP055113156,
- ADMYRE CHARLOTTE ET AL.: 'Exosomes with Immune Modulatory Features Are Present in Human Breast Milk' J. IMMUNOL. vol. 179, 2007, pages 1969 - 1978, XP055113095
- VALADI HADI ET AL.: 'Exosome-mediated transfer of mRNAs and microRNAs is a novel mechanism of genetic exchange between cells' NATURE CELL BIOLOGY vol. 9, no. 6, 2007, pages 654 - 659, XP003016816
- HATA TAKETOSHI ET AL.: 'Isolation of bovine milk-derived microvesicles carrying mRNAs and microRNAs' BIOCHEM. BIOPHYS. RES. COMMUN. vol. 396, 2010, pages 528 - 533, XP055113144
- CLINTON CATHERINE: 'Development of the infant Immune Function and the Effects of Breast Milk' NATURAL MEDICINE JOURNAL vol. 2, no. 8, 2010, pages 3 - 6, XP055113156
- HIROHISA IZUMI ET AL.: 'Boshikan o Ido suru Bonyuchu Exosome no Igi' EXPERIMENTAL MEDICINE vol. 29, no. 3, February 2011, pages 399 - 403, XP008171074
- KOSAKA NOBUYOSHI ET AL.: 'MicroRNA as a new immune-regulatory agent in breast milk' SILENCE vol. 1, 2010, page 7, XP021070612

## Description

### TECHNICAL FIELD

The present invention relates to a method for screening for a diet providing production of milk having immunoregulatory action, and use thereof. They are useful in the fields of food, feed, and so forth.

### BACKGROUND ART

Exosomes are microvesicles secreted by living body cells or cultured cells, and it is known that miRNAs (microRNAs) and proteins exist therein. As body fluids in which presence of exosomes is confirmed, there are known blood (serum and plasma), breast milk, bronchoalveolar lavage fluid, malignant ascite fluid, synovial fluid, urine, amniotic fluid, sperm, saliva, and so forth. Among these body fluids, only blood serum has been investigated about RNAs so far, and all of the target RNAs are miRNAs. On the other hand, in the researches using cells other than body fluid such as cultured cells, RNAs in microvesicles derived from mast cells and glioblastoma have been investigated, and mRNAs are also investigated in these researches (Non-patent document 1 for all the technical background described in this paragraph).

As for milk, it has been reported that miRNAs are contained in bovine milk, bovine serum, and commercial powdered infant formula (Non-patent document 2), and it has been reported that miRNAs are contained in breast milk and various body fluids other than breast milk (Non-patent document 3). Further, it has been reported that miRNAs in breast milk are involved in immunoregulation (Non-patent document 4).

Furthermore, it has also been reported that miRNAs and mRNAs are contained in microvesicles derived from bovine milk (Non-patent document 5). However, mRNAs investigated in this report are only those coding for 6 kinds of proteins, α-S1-casein, α-S2-casein, β-casein, κ-casein, β-lactoglobulin, and MFG-E8, which are milk proteins, and EF-1α, which is a transcription factor.

miRNAs and mRNAs derived from exosomes in human saliva have recently been investigated, and 509 kinds of mRNAs have been detected by using a microarray (Non-patent document 6). Further, potentiality as a biomarker of renal diseases has been suggested for nucleic acids derived from exosomes in human urine, and these nucleic acids include mRNAs (Non-patent document 7).

Breast milk contains secretory IgA, lactoferrin, lysozyme, cytokines, and so forth, and it is considered that it protects infants from infection, and promotes development of infant's immunity (Non-patent document 8). Actually, it is known that children grown up on breast milk involve a lower risk of infection in the bronchi or intestinal tract compared with children not grown in such a manner. Breast milk contains IgA, lactoferrin, glycoproteins, glycolipids etc., which show antibacterial activities, as well as cytokines, which regulate immunocytes.

As mentioned above, it has been reported that miRNAs involved in immunoregulation exist in milk, and that mRNAs coding for milk proteins and transcription factor are contained in microvesicles derived from bovine milk, but it is not known that mRNAs involved in immunoregulation exist in milk.

### PRIOR ART REFERENCES

### NON-PATENT DOCUMENTS

Non-patent document 1: Simpson R.J., Expert Rev. Proteomics, 6:267-283, 2009
Non-patent Document 2: Chen, X. et al., Cell Res., 20:1128-1137, 2010
Non-patent Document 3: Weber, J.A. et al., Clin. Chem., 56:1733-1741, 2010
Non-patent document 4: Kosaka, N. et al., Silence, 1:7, 2010
Non-patent document 5: Hata, T. et al., Biochem. Biophys. Res. Commun., 396:528-533, 2010
Non-patent document 6: Palanisamy, V. et al., PLoS One, 3(9):e3148, 2008
Non-patent document 7: Miranda. K.C. et al., Kidney Int., 78:191-199, 2010
Non-patent document 8: Goldman, A.S., Breastfeed. Med., 2 (4) : 195-204, 2007

### SUMMARY OF THE INVENTION

### OBJECT TO BE ACHIEVED BY THE INVENTION

An object of the present disclosure is to provide a method for screening for a diet providing production of milk having an immunoregulatory action, a food having an immunoregulatory action, and a method for producing it.

### MEANS FOR ACHIEVING THE OBJECT

The inventors of the present invention conducted researches with paying attention to the fact that breast milk affected maturation of infant's immune system. As a result, they found that mRNAs of immunity-related genes were contained in breast milk, and accomplished the present invention.

The present invention thus provides a method for screening for a diet or a substance providing production of breast milk having an immunoregulatory action, which aims at identifying a diet or a substance that increases or decreases amount of mRNA present in milk of a mammal by using correlation of mRNA profile in the milk and a diet ingested by the mammal or a substance contained in the diet as an index.

In an embodiment of the aforementioned method, the immunoregulatory action is an immunostimulating action, and when either one or both of the following conditions are satisfied, it is determined that the diet or the substance provides production of breast milk having an immunostimulating action:
(A) the mRNA is a mRNA of which amount in milk of a rat increases when a *Bifidobacterium* is orally administered to the rat, and ingestion of the diet increases amount of the mRNA,
(B) the mRNA is a mRNA of which amount in milk of a rat decreases when a *Bifidobacterium* is orally administered to the rat, and ingestion of the diet decreases amount of the mRNA.

In another embodiment of the aforementioned method, the immunoregulatory action is an immunosuppressive action, and when either one or both of the following conditions are satisfied, it is determined that the diet or the substance provides production of breast milk having an immunosuppressive action:
(a) the mRNA is a mRNA of which amount in milk of a rat increases when a *Bifidobacterium* is orally administered to the rat, and ingestion of the diet decreases amount of the mRNA,
(b) the mRNA is a mRNA of which amount in milk of a rat decreases when a *Bifidobacterium* is orally administered to the rat, and ingestion of the diet increases amount of the mRNA.

In an embodiment of the aforementioned method, mRNA profiles in the milk observed before and after ingestion of the diet are compared, and when amount of at least one kind of mRNA in the milk observed after the ingestion is higher than that observed before the ingestion, it is determined that the diet increases the amount of the mRNA in the milk.

In the aforementioned method, mRNA profiles in the milk observed before and after ingestion of the diet are compared, and when amount of a mRNA in the milk observed after the ingestion is 1.5 times or more of that observed before the ingestion, it is determined that the diet increases the amount of the mRNA in the milk.

In another embodiment of the aforementioned method, mRNA profiles in the milk observed before and after ingestion of the diet are compared, and when amount of at least one kind of mRNA in the milk observed after the ingestion is lower than that observed before the ingestion, it is determined that the diet decreases the amount of the mRNA in the milk.

In the aforementioned method, mRNA profiles in the milk observed before and after ingestion of the diet are compared, and when amount of a mRNA observed after the ingestion is 0.67 times or less of that observed before the ingestion, it is determined that the diet decreases the amount of the mRNA in the milk.

Further, in a preferred embodiment of the aforementioned method, the mammal is a human, a rat, or a bovine.

In the aforementioned method, the mRNA profiles comprises amount of mRNA selected from the group consisting of Cyp24a1, Dnm1, Dpysl2, Fam89a, Arhgap22, Fbxl5, LOC304396, Btg2, Csnk1g1, Mll1, Eml4, Nelf, Aldh1l1, Boc, Usp43_predicted, Rassf5, Man2a1, Srgn, Gbf1, RGD1562551, Lrba, P2ry2, Bcl2l1, Rbm14, RGD1307365, Tecpr1, Kctd9, Pkig, RGD1308139, Klhl35, Gab1, Arid5b, Zfp157, Mafk, Trim47, Ddx21, Tiparp, Hspalb, Tcfcp211, Nr3c2, Klf4, Dhx8, Myh14, Mcam, Dusp3, Acer2, L3mbtl3, Fcho2, RT1-N3, Ash1l, Paxip1, Irgq, Plcb1, Sts, Spetex-2D, Zmym3, Anxa1, Rtn4ip1, Gngt1, Srcap, Otud4, RGD1307569, Adpgk, Plk3, Phf1, St14, N4bp1, Naaa, Expi, Fgf7, RGD1562492, Lama2, Ahnak, Sec311, Mks1, Prodh, Bazlb, R3hdm1, Setd5, Rhebl1, Kdm5b, Fbxw8, Epb4.1l4a, Lpin2, Cxcl2, Nr2c2, Ckap4, Ankrd1, Ugcg, Fcgr2b, Sf3a1, Parg, Heatr6, Ctdspl, Tp53bp1, Fam60a, Pik3r2, RGD1305089, Arhgef5, Nek7, Rp2, Vhl, Usp31, Camsap1, RGD1308759, Ldlrap1, RGD1359108, Sp1, Mast4, Rela, Nhlrc3, Intu, Gcap14, Spetex-2B, Cdc42se2, Chd7, Rbms1, Invs, LOC100294508, Kif2a, Exoc6, Zbtb1, Rnd3, Rb1cc1, Sphk1, Ugtla2, Noxo1, Mllt6, Thumpd2, Ptpn4, Thex1, S100a8, Grk5, Trove2, Pcdh17, Med13, LOC684871, RGD1305350, Fam82a2, Rab43, Clip1, Ankrd57, RGD1563375, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargclb, Med1, Casp12, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, RGD1562136, Pde4b, Lkap, Chn1, Dusp16, Pex5, Ns5atp9, Map6, Angptl4, Dnmt3b, Fert2, Gzf1, Plcb4, Ap4m1, Stat3, Rkhd1, LOC691223, Dcp2, Rtn4, Tlr3, Tubb6, Casp8, Nin, Fyn, Tmem77, Maml3, Slc15a4, Sqrdl, Ppp1r9b, Pafah1b1, Def8, Fam164a, Znf746, Bmyc, RGD1305235, Thada, Hip1, Pgrmc2, Sgk3, Baz1a, Foxn3, Map3k12, Efhd2, Pogz, Pcf11, RGD1560523, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrpll, LOC687994, Sptbn1, Dtx31, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, RGD1563437, Paqr5, LOC500684, Ddhd1, Kdm6b, Ube2d1, Rhoc, LOC361100, Cx3cl1, LOC100302372, RGD1311892, Fbxo42, Daam1, Klhl5, Fbxo30, Syt12, Herc1, Dab2ip, Tacc2, Sipa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Rell2, Zfyve16, Cebpd, Tnrc6b, Nsun6, Dennd5a, Fbxw5, Slc25a25, Dtwd1, Hspb1, Homer2, Nhej1, Etnk1, Nol9, Med23, Nup133, Rnd1, Rplp1, Ankfy1, Pik3r4, Plcd1, Cxcl12, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, Il17re, Eif4e3, Dock8, Nolc1, Sult1a1, Ankrd17, Trim2, Nfib, Cd14, S100a10, LOC687105, Ccdc123, RGD1309537, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, RGD1310159, Cdh1, Rnf19a, Pdlim1, Csnk1g3, Hectd1, Taf51, Noc3l, Nfat5, RGD1562529, Atl3, Ptpn9, Nedd41, Wipi1, Gnal, Tle1, Pprc1, Src, Clic1, Secisbp2l, Rnf103, Zfp772, Ogt, Zcchc2, Clasp1, Gtpbp2, Ift172, LOC500625, RGD1309350, Map3k6, Plce1, Nedd4, Cpne3, Prim2, Trafd1, Myef2, Znf532, Tmem50b, Il6ra, Pdcd6ip, LOC500757, Tpm4, Rnasen, Lpin1, Flot1, Cds1, Tnfaip1, Mudeng, Cdc215, Arid2, Znf654, Nat8b, Snx12, Ap1g1, Itsn2, Fam21c, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klhl24, Cox11, Tfip11, Rcan1, Nagk, Ep300, Myst3, Rbm28, Anapc7, Ezh1, Cyp27a1, Irs2, Sdccag1, Foxp1, Dusp18, Hcca2, Fbxl8, Chd1, Tp53bp2, RGD1309995, RGD1310552, Kif5b, Pcaf, Slc25a42, Ocln, RGD1559961, Kifap3, Wtip, Gapvd1, Ankrd42, Supt4h1, Haus1, RGD1310951, Prdm2, Arvcf, Gtf2h1, Rassf6, Pcif1, Arap2, Ube2k, Twf1, Rpl8, Prdx1, Phrf1, Pcm1, Mapk12, Slc1a4, Prl3d4, Trim16, LOC689842, Cacnale, RT1-EC2, Gal, Arnt2, Stk11ip, Sdc3, RGD1563155, Tp53i11, Mpz, Dnmt31, Ache, Cln5, Olr184, Asb7, Rtp3, Peli2, Caskin2, Cass4, Plvap, LOC680711, Nat2, RGD1565498, Hist1h1a, Col22a1, LOC100360528, Htr6, Trim42, Ccdc89, Atp10d, Klri2, LOC100360801, RGD1560019, Ugt2b37, Oprk1, Cyct, Pdzd2, Vom2r31, Sh2d5, Tmem136, Gh1, Ttl, Pgbd5, Zfp583, Ankrd2, Avil, Mcm3ap, Mypn, Frmd4a, Rnf182, Kcng1, Paqr7, RGD1304929, RGD1565100, Pkp1, Nnmt, Csrnp1, Siae, Hoxb6, Pga5, Hoxd9, RGD1308541, Cyth3, Fbp1, Bnc1, Col18a1, Traf3, Sema7a, Rcn3, Ift122, Olr1275, LOC619574, Slc5a4a, Smc2, Cldn18, LOC679462, Pogk, Olr445, Abhd15, LOC688219, RGD1564846, Pias4, Alg8, Nkx2-4, Mmp2, Iqcf1, B9d2, Fam107a, Rarres1, RGD1561276, Wbp2, Wdr93, C1qtnf5, Olr1303, Egfl7, Olr507, Slc32a1, RGD1563516, Id3, LOC691130, Ccdc58, Sox21, Tnfsf12, Qrich2, RGD1308930, LOC307727, B3galnt2, Tob2, Creb311, Klk12, Dqx1, Ncoa2, Slfnl1, Elf2, Gsx2, Hhip, Grm2, Fam170a, Pnma3, Gpc2, Npvf, RGD1308143, Tsen54, Igfbp1, Zdhhc19, Csrnp2, Dcst2, Krt1, Nanos1, Cdh13, Ebi3, Prr3, Rnf43, Btbd17, Olr1626, Trim26, Gata3, Cdkn1c, Hrc, Grasp, Galm, RGD1308106, Samd14, Olr471, Oprd1, Pim1, Il2rb, Ptpn13, Mtus1, RGD1305298, Fhl3, Olr515, Tpsab1, RGD1564961, Hdhd2, Farp1, Ptprs, Hand1, Rpl37a, Tcf15, Neurod2, Usp20, Foxo3, Aldh1l2, RGD1308706, Nov, RGD1566130, Slc34a2, Gzma, Hist1h4b, Iqgap3, Enpp2, Tm7sf2, Exo1, Slc9a1, Rpl37, RGD1565970, Pde12, Eefsec, Dmrta2, LOC498685, Brunol5, Ocm, Olr4, Tmem164, RGD1561843, Flad1, Frmd4b, Marcksl1, Bhlhe22, Yif1b, Ttc38, Phyh, Mfsd6, LOC257642, Fam189b, Sned1, LOC100362849, Cd3d, Slc25a29, Cd7, RGD1563281, Grb7, Ccdc147, Stat2, LOC688228, Ppp3ca, LOC688241, Shisa4, Atp8b1, Krtap1-5, Cd3g, Txnl4b, Ranbp10, Dynlt3, Atp2b2, Trub1, Zhx3, Rexo4, Snapc2, LOC685031, Rad541, Vps37c, Hmga1, Olr1572, Furin, RT1-CE5, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, LOC287010, Arhgap26, LOC311352, Znrf4, Msh5, Tmed7, Gpd1l, Ankrd37, Cox4i2, Msl3l2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, RGD1563148, Vegfb, Lpl, Chaf1a, Srrd, Steap1, Fkbp11, Slc39a3, Zfp53, Bcan, Foxc2, Jph1, Ccl27, Pdia3, Gabrq, Surf6, Runx3, Abcb6, Cdk2, LOC687813, and Porcn.

Further, in the aforementioned method, the mRNA of which amount in milk of a rat increases when a *Bifidobacterium* is orally administered to the rat is selected from the group consisting of Cyp24a1, Dnm1, Dpysl2, Fam89a, Arhgap22, Fbxl5, LOC304396, Btg2, Csnk1g1, Mll1, Eml4, Nelf, Aldh1l1, Boc, Usp43_predicted, Rassf5, Man2a1, Srgn, Gbf1, RGD1562551, Lrba, P2ry2, Bcl2l1, Rbm14, RGD1307365, Tecpr1, Kctd9, Pkig, RGD1308139, Klhl35, Gab1, Arid5b, Zfp157, Mafk, Trim47, Ddx21, Tiparp, Hspalb, Tcfcp211, Nr3c2, Klf4, Dhx8, Myh14, Mcam, Dusp3, Acer2, L3mbtl3, Fcho2, RT1-N3, Ash1l, Paxip1, Irgq, Plcb1, Sts, Spetex-2D, Zmym3, Anxa1, Rtn4ip1, Gngt1, Srcap, Otud4, RGD1307569, Adpgk, Plk3, Phf1, St14, N4bp1, Naaa, Expi, Fgf7, RGD1562492, Lama2, Ahnak, Sec311, Mks1, Prodh, Bazlb, R3hdm1, Setd5, Rhebl1, Kdm5b, Fbxw8, Epb4.1l4a, Lpin2, Cxcl2, Nr2c2, Ckap4, Ankrd1, Ugcg, Fcgr2b, Sf3a1, Parg, Heatr6, Ctdspl, Tp53bp1, Fam60a, Pik3r2, RGD1305089, Arhgef5, Nek7, Rp2, Vhl, Usp31, Camsap1, RGD1308759, Ldlrap1, RGD1359108, Sp1, Mast4, Rela, Nhlrc3, Intu, Gcap14, Spetex-2B, Cdc42se2, Chd7, Rbms1, Invs, LOC100294508, Kif2a, Exoc6, Zbtb1, Rnd3, Rb1cc1, Sphk1, Ugtla2, Noxo1, Mllt6, Thumpd2, Ptpn4, Thex1, S100a8, Grk5, Trove2, Pcdh17, Med13, LOC684871, RGD1305350, Fam82a2, Rab43, Clip1, Ankrd57, RGD1563375, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargclb, Med1, Casp12, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, RGD1562136, Pde4b, Lkap, Chn1, Dusp16, Pex5, Ns5atp9, Map6, Angptl4, Dnmt3b, Fert2, Gzf1, Plcb4, Ap4m1, Stat3, Rkhd1, LOC691223, Dcp2, Rtn4, Tlr3, Tubb6, Casp8, Nin, Fyn, Tmem77, Maml3, Slc15a4, Sqrdl, Ppp1r9b, Pafah1b1, Def8, Fam164a, Znf746, Bmyc, RGD1305235, Thada, Hip1, Pgrmc2, Sgk3, Baz1a, Foxn3, Map3k12, Efhd2, Pogz, Pcf11, RGD1560523, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrpll, LOC687994, Sptbn1, Dtx31, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, RGD1563437, Paqr5, LOC500684, Ddhd1, Kdm6b, Ube2d1, Rhoc, LOC361100, Cx3cl1, LOC100302372, RGD1311892, Fbxo42, Daam1, Klhl5, Fbxo30, Syt12, Herc1, Dab2ip, Tacc2, Sipa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Rell2, Zfyve16, Cebpd, Tnrc6b, Nsun6, Dennd5a, Fbxw5, Slc25a25, Dtwd1, Hspb1, Homer2, Nhej1, Etnk1, Nol9, Med23, Nup133, Rnd1, Rplp1, Ankfy1, Pik3r4, Plcd1, Cxcl12, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, Il17re, Eif4e3, Dock8, Nolc1, Sult1a1, Ankrd17, Trim2, Nfib, Cd14, S100a10, LOC687105, Ccdc123, RGD1309537, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, RGD1310159, Cdh1, Rnf19a, Pdlim1, Csnk1g3, Hectd1, Taf51, Noc3l, Nfat5, RGD1562529, Atl3, Ptpn9, Nedd41, Wipi1, Gnal, Tle1, Pprc1, Src, Clic1, Secisbp2l, Rnf103, Zfp772, Ogt, Zcchc2, Clasp1, Gtpbp2, Ift172, LOC500625, RGD1309350, Map3k6, Plce1, Nedd4, Cpne3, Prim2, Trafd1, Myef2, Znf532, Tmem50b, Il6ra, Pdcd6ip, LOC500757, Tpm4, Rnasen, Lpin1, Flot1, Cds1, Tnfaip1, Mudeng, Cdc215, Arid2, Znf654, Nat8b, Snx12, Ap1g1, Itsn2, Fam21c, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klhl24, Cox11, Tfip11, Rcan1, Nagk, Ep300, Myst3, Rbm28, Anapc7, Ezh1, Cyp27a1, Irs2, Sdccag1, Foxp1, Dusp18, Hcca2, Fbxl8, Chd1, Tp53bp2, RGD1309995, RGD1310552, Kif5b, Pcaf, Slc25a42, Ocln, RGD1559961, Kifap3, Wtip, Gapvd1, Ankrd42, Supt4h1, Haus1, RGD1310951, Prdm2, Arvcf, Gtf2h1, Rassf6, Pcif1, Arap2, Ube2k, Twf1, Rpl8, Prdx1, Phrf1, Pcm1, and Mapk12

The aforementioned mRNAs are mRNAs corresponding to the genes mentioned in Tables 1 to 14 mentioned later. In the tables, the gene names mentioned in the columns of "Gene Symbol" and "Entrez Gene ID" are the gene names used in the Entrez Gene database of NCBI (National Center for Biotechnology Information), and the gene names mentioned in the column of "Gene Name" are the generic gene names. The same shall apply to Tables 15 to 22 mentioned later.

Further, in the aforementioned method, the mRNA of which amount in milk of a rat decreases when a *Bifidobacterium* is orally administered to the rat is selected from the group consisting of Slc1a4, Prl3d4, Trim16, LOC689842, Cacnale, RT1-EC2, Gal, Arnt2, Stk11ip, Sdc3, RGD1563155, Tp53i11, Mpz, Dnmt31, Ache, Cln5, Olr184, Asb7, Rtp3, Peli2, Caskin2, Cass4, Plvap, LOC680711, Nat2, RGD1565498, Hist1h1a, Col22a1, LOC100360528, Htr6, Trim42, Ccdc89, Atp10d, Klri2, LOC100360801, RGD1560019, Ugt2b37, Oprk1, Cyct, Pdzd2, Vom2r31, Sh2d5, Tmem136, Gh1, Ttl, Pgbd5, Zfp583, Ankrd2, Avil, Mcm3ap, Mypn, Frmd4a, Rnf182, Kcng1, Paqr7, RGD1304929, RGD1565100, Pkp1, Nnmt, Csrnp1, Siae, Hoxb6, Pga5, Hoxd9, RGD1308541, Cyth3, Fbp1, Bnc1, Col18a1, Traf3, Sema7a, Rcn3, Ift122, Olr1275, LOC619574, Slc5a4a, Smc2, Cldn18, LOC679462, Pogk, Olr445, Abhd15, LOC688219, RGD1564846, Pias4, Alg8, Nkx2-4, Mmp2, Iqcf1, B9d2, Fam107a, Rarres1, RGD1561276, Wbp2, Wdr93, C1qtnf5, Olr1303, Egfl7, Olr507, Slc32a1, RGD1563516, Id3, LOC691130, Ccdc58, Sox21, Tnfsf12, Qrich2, RGD1308930, LOC307727, B3galnt2, Tob2, Creb311, Klk12, Dqx1, Ncoa2, Slfnl1, Elf2, Gsx2, Hhip, Grm2, Fam170a, Pnma3, Gpc2, Npvf, RGD1308143, Tsen54, Igfbp1, Zdhhc19, Csrnp2, Dcst2, Krt1, Nanos1, Cdh13, Ebi3, Prr3, Rnf43, Btbd17, Olr1626, Trim26, Gata3, Cdkn1c, Hrc, Grasp, Galm, RGD1308106, Samd14, Olr471, Oprd1, Pim1, Il2rb, Ptpn13, Mtus1, RGD1305298, Fhl3, Olr515, Tpsab1, RGD1564961, Hdhd2, Farp1, Ptprs, Hand1, Rpl37a, Tcf15, Neurod2, Usp20, Foxo3, Aldh1l2, RGD1308706, Nov, RGD1566130, Slc34a2, Gzma, Hist1h4b, Iqgap3, Enpp2, Tm7sf2, Exo1, Slc9a1, Rpl37, RGD1565970, Pde12, Eefsec, Dmrta2, LOC498685, Brunol5, Ocm, Olr4, Tmem164, RGD1561843, Flad1, Frmd4b, Marcksl1, Bhlhe22, Yif1b, Ttc38, Phyh, Mfsd6, LOC257642, Fam189b, Sned1, LOC100362849, Cd3d, Slc25a29, Cd7, RGD1563281, Grb7, Ccdc147, Stat2, LOC688228, Ppp3ca, LOC688241, Shisa4, Atp8b1, Krtap1-5, Cd3g, Txnl4b, Ranbp10, Dynlt3, Atp2b2, Trub1, Zhx3, Rexo4, Snapc2, LOC685031, Rad541, Vps37c, Hmga1, Olr1572, Furin, RT1-CE5, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, LOC287010, Arhgap26, LOC311352, Znrf4, Msh5, Tmed7, Gpd1l, Ankrd37, Cox4i2, Msl3l2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, RGD1563148, Vegfb, Lpl, Chaf1a, Srrd, Steap1, Fkbp11, Slc39a3, Zfp53, Bcan, Foxc2, Jph1, Ccl27, Pdia3, Gabrq, Surf6, Runx3, Abcb6, Cdk2, LOC687813, and Porcn.

The aforementioned mRNAs are mRNAs corresponding to the genes mentioned in Tables 15 to 22 mentioned later.

The present invention also provides a method for producing milk or a dairy product having an immunoregulatory action, which comprises the step of giving a diet or a substance identified to increase or decrease amount of mRNA in milk of a mammal by the aforementioned screening method to a mammal (except for human), and the step of collecting milk of the mammal.

In an embodiment of the aforementioned method, the immunoregulatory action is an immunostimulating action, and the diet or the substance is one determined to provide production of breast milk having an immunostimulating action.

In another embodiment of the aforementioned method, the immunoregulatory action is an immunosuppressive action, and the diet or the substance is one determined to provide production of breast milk having an immunosuppressive action.

The present invention also provides a method for producing a composition for oral ingestion having an immunostimulating action, which comprises adding a mRNA of which amount in milk of a rat increases when a *Bifidobacterium* is orally administered to the rat to a base for a composition for oral ingestion.

In the aforementioned method, the mRNA is selected from the group consisting of Cyp24a1, Dnm1, Dpysl2, Fam89a, Arhgap22, Fbxl5, LOC304396, Btg2, Csnk1g1, Mll1, Eml4, Nelf, Aldh1l1, Boc, Usp43_predicted, Rassf5, Man2a1, Srgn, Gbf1, RGD1562551, Lrba, P2ry2, Bcl2l1, Rbm14, RGD1307365, Tecpr1, Kctd9, Pkig, RGD1308139, Klhl35, Gab1, Arid5b, Zfp157, Mafk, Trim47, Ddx21, Tiparp, Hspalb, Tcfcp211, Nr3c2, Klf4, Dhx8, Myh14, Mcam, Dusp3, Acer2, L3mbtl3, Fcho2, RT1-N3, Ash1l, Paxip1, Irgq, Plcb1, Sts, Spetex-2D, Zmym3, Anxa1, Rtn4ip1, Gngt1, Srcap, Otud4, RGD1307569, Adpgk, Plk3, Phf1, St14, N4bp1, Naaa, Expi, Fgf7, RGD1562492, Lama2, Ahnak, Sec311, Mks1, Prodh, Bazlb, R3hdm1, Setd5, Rhebl1, Kdm5b, Fbxw8, Epb4.1l4a, Lpin2, Cxcl2, Nr2c2, Ckap4, Ankrd1, Ugcg, Fcgr2b, Sf3a1, Parg, Heatr6, Ctdspl, Tp53bp1, Fam60a, Pik3r2, RGD1305089, Arhgef5, Nek7, Rp2, Vhl, Usp31, Camsap1, RGD1308759, Ldlrap1, RGD1359108, Sp1, Mast4, Rela, Nhlrc3, Intu, Gcap14, Spetex-2B, Cdc42se2, Chd7, Rbms1, Invs, LOC100294508, Kif2a, Exoc6, Zbtb1, Rnd3, Rb1cc1, Sphk1, Ugtla2, Noxo1, Mllt6, Thumpd2, Ptpn4, Thex1, S100a8, Grk5, Trove2, Pcdh17, Med13, LOC684871, RGD1305350, Fam82a2, Rab43, Clip1, Ankrd57, RGD1563375, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargclb, Med1, Casp12, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, RGD1562136, Pde4b, Lkap, Chn1, Dusp16, Pex5, Ns5atp9, Map6, Angptl4, Dnmt3b, Fert2, Gzf1, Plcb4, Ap4m1, Stat3, Rkhd1, LOC691223, Dcp2, Rtn4, Tlr3, Tubb6, Casp8, Nin, Fyn, Tmem77, Maml3, Slc15a4, Sqrdl, Ppp1r9b, Pafah1b1, Def8, Fam164a, Znf746, Bmyc, RGD1305235, Thada, Hip1, Pgrmc2, Sgk3, Baz1a, Foxn3, Map3k12, Efhd2, Pogz, Pcf11, RGD1560523, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrpll, LOC687994, Sptbn1, Dtx31, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, RGD1563437, Paqr5, LOC500684, Ddhd1, Kdm6b, Ube2d1, Rhoc, LOC361100, Cx3cl1, LOC100302372, RGD1311892, Fbxo42, Daam1, Klhl5, Fbxo30, Syt12, Herc1, Dab2ip, Tacc2, Sipa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Rell2, Zfyve16, Cebpd, Tnrc6b, Nsun6, Dennd5a, Fbxw5, Slc25a25, Dtwd1, Hspb1, Homer2, Nhej1, Etnk1, Nol9, Med23, Nup133, Rnd1, Rplp1, Ankfy1, Pik3r4, Plcd1, Cxcl12, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, Il17re, Eif4e3, Dock8, Nolc1, Sult1a1, Ankrd17, Trim2, Nfib, Cd14, S100a10, LOC687105, Ccdc123, RGD1309537, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, RGD1310159, Cdh1, Rnf19a, Pdlim1, Csnk1g3, Hectd1, Taf51, Noc3l, Nfat5, RGD1562529, Atl3, Ptpn9, Nedd41, Wipi1, Gnal, Tle1, Pprc1, Src, Clic1, Secisbp2l, Rnf103, Zfp772, Ogt, Zcchc2, Clasp1, Gtpbp2, Ift172, LOC500625, RGD1309350, Map3k6, Plce1, Nedd4, Cpne3, Prim2, Trafd1, Myef2, Znf532, Tmem50b, Il6ra, Pdcd6ip, LOC500757, Tpm4, Rnasen, Lpin1, Flot1, Cds1, Tnfaip1, Mudeng, Cdc215, Arid2, Znf654, Nat8b, Snx12, Ap1g1, Itsn2, Fam21c, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klh124, Cox11, Tfip11, Rcan1, Nagk, Ep300, Myst3, Rbm28, Anapc7, Ezh1, Cyp27a1, Irs2, Sdccag1, Foxp1, Dusp18, Hcca2, Fbxl8, Chd1, Tp53bp2, RGD1309995, RGD1310552, Kif5b, Pcaf, Slc25a42, Ocln, RGD1559961, Kifap3, Wtip, Gapvd1, Ankrd42, Supt4h1, Haus1, RGD1310951, Prdm2, Arvcf, Gtf2h1, Rassf6, Pcif1, Arap2, Ube2k, Twf1, Rpl8, Prdx1, Phrf1, Pcm1, and Mapk12.

The present invention also provides a method for producing a composition for oral ingestion having an immunosuppressive action, which comprises adding a mRNA of which amount in milk of a rat decreases when a *Bifidobacterium* is orally administered to the rat to a base for a composition for oral ingestion.

In the aforementioned method, the mRNA is selected from the group consisting of Slcla4, Prl3d4, Trim16, LOC689842, Cacnale, RT1-EC2, Gal, Arnt2, Stk11ip, Sdc3, RGD1563155, Tp53i11, Mpz, Dnmt31, Ache, Cln5, Olr184, Asb7, Rtp3, Peli2, Caskin2, Cass4, Plvap, LOC680711, Nat2, RGD1565498, Hist1h1a, Col22a1, LOC100360528, Htr6, Trim42, Ccdc89, Atp10d, Klri2, LOC100360801, RGD1560019, Ugt2b37, Oprk1, Cyct, Pdzd2, Vom2r31, Sh2d5, Tmem136, Gh1, Ttl, Pgbd5, Zfp583, Ankrd2, Avil, Mcm3ap, Mypn, Frmd4a, Rnf182, Kcng1, Paqr7, RGD1304929, RGD1565100, Pkp1, Nnmt, Csrnp1, Siae, Hoxb6, Pga5, Hoxd9, RGD1308541, Cyth3, Fbp1, Bnc1, Col18a1, Traf3, Sema7a, Rcn3, Ift122, Olr1275, LOC619574, Slc5a4a, Smc2, Cldn18, LOC679462, Pogk, Olr445, Abhd15, LOC688219, RGD1564846, Pias4, Alg8, Nkx2-4, Mmp2, Iqcf1, B9d2, Fam107a, Rarres1, RGD1561276, Wbp2, Wdr93, C1qtnf5, Olr1303, Egf17, Olr507, Slc32a1, RGD1563516, Id3, LOC691130, Ccdc58, Sox21, Tnfsf12, Qrich2, RGD1308930, LOC307727, B3galnt2, Tob2, Creb311, Klk12, Dqx1, Ncoa2, Slfnl1, Elf2, Gsx2, Hhip, Grm2, Fam170a, Pnma3, Gpc2, Npvf, RGD1308143, Tsen54, Igfbp1, Zdhhc19, Csrnp2, Dcst2, Krt1, Nanos1, Cdh13, Ebi3, Prr3, Rnf43, Btbd17, Olr1626, Trim26, Gata3, Cdkn1c, Hrc, Grasp, Galm, RGD1308106, Samd14, Olr471, Oprd1, Pim1, I12rb, Ptpn13, Mtus1, RGD1305298, Fh13, Olr515, Tpsab1, RGD1564961, Hdhd2, Farp1, Ptprs, Hand1, Rpl37a, Tcf15, Neurod2, Usp20, Foxo3, Aldh1l2, RGD1308706, Nov, RGD1566130, Slc34a2, Gzma, Hist1h4b, Iqgap3, Enpp2, Tm7sf2, Exo1, Slc9a1, Rpl37, RGD1565970, Pde12, Eefsec, Dmrta2, LOC498685, Brunol5, Ocm, Olr4, Tmem164, RGD1561843, Flad1, Frmd4b, Marcksl1, Bhlhe22, Yif1b, Ttc38, Phyh, Mfsd6, LOC257642, Fam189b, Sned1, LOC100362849, Cd3d, Slc25a29, Cd7, RGD1563281, Grb7, Ccdc147, Stat2, LOC688228, Ppp3ca, LOC688241, Shisa4, Atp8b1, Krtap1-5, Cd3g, Txnl4b, Ranbp10, Dynlt3, Atp2b2, Trub1, Zhx3, Rexo4, Snapc2, LOC685031, Rad541, Vps37c, Hmga1, Olr1572, Furin, RT1-CE5, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, LOC287010, Arhgap26, LOC311352, Znrf4, Msh5, Tmed7, Gpd11, Ankrd37, Cox4i2, Msl312, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, RGD1563148, Vegfb, Lpl, Chaf1a, Srrd, Steap1, Fkbp11, Slc39a3, Zfp53, Bean, Foxc2, Jph1, Ccl27, Pdia3, Gabrq, Surf6, Runx3, Abcb6, Cdk2, LOC687813, and Porcn.

In a preferred embodiment of the method for producing a composition for oral ingestion, the composition is a food for infants or a food for little children.

In a preferred embodiment of the method for producing a composition for oral ingestion, the food for infants or the food for little children is infant formula or follow-on formula.

### MODES FOR CARRYING OUT THE INVENTION

The method of the present invention is a method for screening for a diet or a substance providing production of breast milk having an immunoregulatory action, which comprises identifying a diet or a substance that increases or decreases amount of mRNA present in milk of a mammal by using correlation of mRNA profile in the milk and a diet ingested by the mammal or a substance contained in the diet as an index.

In an embodiment of the aforementioned method, the immunoregulatory action is an immunostimulating action, and when either one or both of the following conditions are satisfied, it is determined that the diet or the substance provides production of breast milk having an immunostimulating action:
(A) the mRNA is a mRNA of which amount in milk of a rat increases when a *Bifidobacterium* is orally administered to the rat, and ingestion of the diet increases amount of the mRNA,
(B) the mRNA is a mRNA of which amount in milk of a rat decreases when a *Bifidobacterium* is orally administered to the rat, and ingestion of the diet decreases amount of the mRNA.

In another embodiment of the aforementioned method, the immunoregulatory action is an immunosuppressive action, and when either one or both of the following conditions are satisfied, it is determined that the diet or the substance provides production of breast milk having an immunosuppressive action:
(a) the mRNA is a mRNA of which amount in milk of a rat increases when a *Bifidobacterium* is orally administered to the rat, and ingestion of the diet decreases amount of the mRNA,
(b) the mRNA is a mRNA of which amount in milk of a rat decreases when a *Bifidobacterium* is orally administered to the rat, and ingestion of the diet increases amount of the mRNA.

The present invention is based on a concept that an immunoregulatory action is expected to be obtained by oral administration of mRNA, because of the novel finding that mRNAs relating to immunoregulation are contained in milk, and the fact that breast milk promotes development of immunity in infants ingesting the breast milk (for example, Breastfeed Med., 2(4):195-204, 2007). And, on the basis of a prediction that mRNA profile in milk is affected by diet, it was thought to identify a diet or an active ingredient contained in it that could increase or decrease amount of mRNA present in milk.

The immunoregulatory action defined for the screening method, milk, dairy product, and so forth of the present invention includes, for example, both an action of enhancing immunopotentiating action, which functions for the purpose of "defense" against external attacks (immunostimulating action), and an immunosuppressive action suppressively functioning against overresponse by the immunity, i.e., allergic responses, autoimmune diseases, chronic inflammations etc., in which "hyperimmunoreaction" adversely affect living organisms.

The terms "immunostimulating action" and "immunosuppressive action" are used in a relative meaning. When an immunopotentiating action usually observed for breast milk of a certain mammal is enhanced after ingestion of the diet or the substance, the breast milk has an immunostimulating action, and when the immunopotentiating action is decreased after ingestion of the diet or the substance, the breast milk has an immunosuppressive action. When the immunopotentiating action observed after ingestion of the diet or the substance by a mammal is enhanced compared with that observed before the ingestion, the breast milk of the mammal has an immunostimulating action, and when the immunopotentiating action is decreased compared with that observed before the ingestion, the breast milk has an immunosuppressive action.

The correlation of mRNA profile in milk of a mammal and a diet ingested by the mammal or a substance contained in the diet can be investigated, for example, as follows.

Milk is collected from a mammal that ingested a diet, and mRNA profile in the milk is examined.

The mammal is not particularly limited, and examples include human, bovine, water buffalo, goat, ovine, swine, ape, dog, cat, rat, mouse, hamster, guinea pig, and so forth. The mammal is preferably human or bovine.

In the present invention, the mRNA profile consists of type and amount of mRNA. The mRNA may consist of one kind of mRNA, or arbitrary two or more kinds of mRNAs. Type of mRNA is not particularly limited, so long as those existing in milk and involved in immunoregulation are chosen. As described above, it is known that breast milk promotes development of immunity in infants who ingest it (for example, Breastfeed Med., 2(4):195-204, 2007). Therefore, it is suggested that mRNAs contained in milk include those involved in immune functions. Further, as shown in the examples, it has been demonstrated that mRNAs contained in milk include those of which amount is increased by administration of a *Bifidobacterium* having an immunoregulatory action, and those of which amount is decreased by the same. This fact means that mRNAs contained in milk include those functioning in an immunostimulating manner, and those functioning in an immunosuppressive manner.

Type of mRNA is not particularly limited, so long as those existing in milk and involved in immunoregulation are chosen. Specific examples include, for example, mRNA of which amount in milk of a rat increases when a *Bifidobacterium* is orally administered to the rat, and mRNA of which amount in milk of a rat decreases when a *Bifidobacterium* is orally administered to the rat.

Examples of mRNA of which amount in milk of a rat increases when a *Bifidobacterium* is orally administered to the rat include those mentioned in Tables 1 to 14 mentioned later.

Examples of mRNA of which amount in milk of a rat decreases when a *Bifidobacterium* is orally administered to the rat include those mentioned in Tables 15 to 22 mentioned later.

In this specification, mRNA is specified by a name of corresponding gene, i.e., the gene used as a template for transcription of the mRNA.

A certain gene and a protein encoded by it may have different sequences depending on type of organism or due to individual difference. Genes showing high sequence homology and coding for proteins having the same functions or analogous functions are often regarded as the same genes or analogous genes as homologues or orthologues. In the present invention, mRNAs derived from the same or analogous genes are regarded as the same mRNAs. Therefore, even when "mRNA" in the expression "mRNA profile in milk of a mammal" and corresponding "mRNA" of which amount in milk of a rat increases or decreases when a *Bifidobacterium* is orally administered to the rat have different sequences, they may be regarded as the same mRNA, so long as they show high sequence homology and code for proteins having the same or analogous functions.

mRNA is not limited to mRNA having a known sequence, but mRNA may contain substitution, deletion, insertion, addition, or inversion of one or several nucleotides, so long as the encoded protein has function the same as or analogous to that of a protein encoded by a known sequence. Specific examples of such mRNA include, for example, mRNA having a nucleotide sequence showing a homology of 80% or more, preferably 90% or more, more preferably 95% or more, still more preferably 98% or more, to a known mRNA.

The amount of mRNA may be an absolute amount or a relative amount. The relative amount may be a relative amount based on an average amount in a certain mammal, or may be a relative amount observed after ingestion of a diet based on the amount observed before the ingestion. For the measurement of the amount of mRNA, methods usually used for measurement of mRNA amount such as quantitative reverse transcription PCR (qRT-PCR) can be employed. The amount of mRNA can also be measured by the microarray method. As for extraction of mRNA from milk, methods usually used for extraction of mRNA can be employed, and a commercially available mRNA isolation kit can also be used.

Further, amount of mRNA present in milk can also be indirectly measured by measuring expression amount of the mRNA in mammary glandular cells.

Correlation of mRNA profile in milk of a mammal and a diet ingested by the mammal or a substance contained in the diet is examined. The correlation of the mRNA profile in milk of a mammal and a diet ingested by the mammal or a substance contained in the diet refers to correlation of the mRNA profile and presence or absence of the diet or the substance or amount of the diet or the substance. For example, if amounts of one or more kinds of mRNAs in milk of a mammal which has ingested a certain diet or substance are higher or lower than those observed in the mammal which has not ingested the diet or the substance, the diet or the substance and the mRNA profile have positive or negative correlation, respectively. Further, also when amounts of one or more kinds of mRNAs in milk of a mammal which has ingested a certain diet or substance are higher than those observed in the mammal which has not ingested the diet or the substance, and at the same time, amounts of other one or more kinds of mRNAs in milk of a mammal which has ingested a certain diet or substance are lower than those observed in the mammal which has not ingested the diet or the substance, the diet or the substance and the mRNA profile have correlation. Further, if ingestion of a certain diet or substance does not affect mRNA profile, the substance and the mRNA profile do not correlate with each other.

Specifically, for example, when mRNA profiles in the milk observed before and after ingestion of the diet are compared, and amount of a certain kind of mRNA observed after the ingestion is higher than that observed before the ingestion, it is determined that the diet increases the amount of the mRNA in the milk. The degree of increase in amount of mRNA in the milk observed after the ingestion of the diet is 1.5 times or more, preferably 2 times or more, more preferably 5 times or more, still more preferably 10 times or more, of that observed before the ingestion.

Further, when mRNA profiles in the milk observed before and after ingestion of the diet are compared, and amount of a certain kind of mRNA observed after the ingestion is lower than that observed before the ingestion, it is determined that the diet decreases the amount of the mRNA in the milk. The degree of decrease in amount of mRNA in the milk observed after the ingestion of the diet is 0.67 times or less, preferably 0.5 times or less, more preferably 0.2 times or less, still more preferably 0.1 times or less, of that observed before the ingestion.

Furthermore, measurement of mRNA profile before ingestion of a diet is not indispensable, and correlation of a diet and amount of mRNA can also be examined by comparing mRNA profile measured after ingestion of a diet with ordinary mRNA profile of an objective mammal measured beforehand.

mRNA of which correlation is investigated may consist of a single kind of mRNA, or arbitrary two or more kinds of mRNAs.

In another embodiment, mRNA profile in milk and mRNA profile in serum or plasma are compared, and if amount of mRNA contained in both of milk and serum or plasma is increased by ingestion of the diet at a higher degree in milk compared with that observed in serum or plasma, it is determined that the diet increases amount of the mRNA present in milk. The degree of increase in amount of mRNA in milk is 1.5 times or more, preferably 2 times or more, more preferably 5 times or more, still more preferably 10 times or more, of that observed in serum or plasma.

Further, when mRNA profile in milk and mRNA profile in serum or plasma are compared, if amount of a mRNA contained in both of milk and serum or plasma is decreased by ingestion of the diet at a larger degree in milk compared with that observed in serum or plasma, it is determined that the diet decreases amount of the mRNA present in milk. The degree of decrease in amount of mRNA in milk compared with that in serum or plasma is 1.5 times or more, preferably 2 times or more, more preferably 5 times or more, still more preferably 10 times or more. For example, when amount of a certain mRNA decreases to 0.5 times in serum or plasma and decreases to 0.3 times in milk, degree of the decrease in milk is 1.67 (0.5/0.3) times higher than that in serum or plasma.

In addition, when there is a factor that increases or decreases amount of mRNA in milk, and increase or decrease of the amount of the mRNA is suppressed by ingestion of a certain diet, the diet has an action of decreasing or increasing the amount of the mRNA.

The diet may consist of a single substance or may be a composition, so long as it can be orally ingested. Further, "before ingestion" and "after ingestion" may mean "before and after one time of ingestion of diet", or "before and after two or more times of ingestion of diet". Further, two or more times of ingestion of diet may be two or more times of ingestion of the same diet, or ingestion of two or more kinds of diets.

The diet may be ingested according to a planned scheme or freely ingested. In the latter case, correlation of the diet and mRNA profile in milk can be examined by hearing content of ingested diet in the case of human. When the diet is ingested or administered according to a planned scheme, the diet can be considered as a "test sample". The diet may be a usual diet or a usual diet containing a test substance. Amount of diet to be ingested, time of ingestion, number of times of ingestion and so forth are not particularly limited.

If a diet that increases amount of mRNA in milk is chosen, a substance that is contained in the diet and increases amount of the mRNA in milk can be identified in the same manner as that mentioned above. Further, if a diet that decreases amount of mRNA in milk is chosen, a substance that is contained in the diet and decreases amount of the mRNA in milk can be identified in the same manner as that mentioned above.

If a diet or a substance that increases or decreases amount of mRNA in milk is identified, a diet that increases or decreases amount of the mRNA in milk can be designed.

That is, a diet or a substance contained in the diet of which ingestion increases amount of mRNA of the same kind as that of mRNA of which amount in milk of a rat increases when a *Bifidobacterium* is orally administered to the rat, among mRNAs in milk, is preferred for production of milk having an immunostimulating action. Further, a diet or a substance contained in the diet of which ingestion decreases amount of mRNA of the same kind as that of mRNA of which amount in milk of a rat decreases when a *Bifidobacterium* is orally administered to the rat, among mRNAs in milk, is also preferred for production of milk having an immunostimulating action. Further, it is considered that these diets or substances are unsuitable for production of milk having an immunosuppressive action.

On the other hand, a diet or a substance contained in the diet of which ingestion decreases amount of mRNA of the same kind as that of mRNA of which amount in milk of a rat increases when a *Bifidobacterium* is orally administered to the rat, among mRNAs in milk, is preferred for production of milk having an immunosuppressive action. Further, a diet or a substance contained in the diet of which ingestion increases amount of mRNA of the same kind as that of mRNA of which amount in milk of a rat decreases when a *Bifidobacterium* is orally administered to the rat, among mRNAs in milk, is also preferred for production of milk having an immunosuppressive action. Further, it is considered that these diets or substances are unsuitable for production of milk having an immunostimulating action.

Screening for a diet or a substance providing production of breast milk having an immunoregulatory action, or a diet or a substance unsuitable for production of breast milk having an immunoregulatory action can be performed as described above. As shown in the examples described later, when a *Bifidobacterium* (*Bifidobacterium longum*) was orally administered to rats, amounts of 562 kinds of mRNAs increased, and amounts of 325 kinds of mRNAs decreased.

It is known that *Bifidobacteria* function as probiotics, and have, in particular, an immunoregulatory action. Therefore, the fact that the administration of the *Bifidobacterium* increased amounts of mRNAs in milk also supports the involvement of mRNAs in milk in immunoregulation. Demonstration of change in amounts of mRNAs in milk induced by administration of the *Bifidobacterium,* i.e., correlation of the *Bifidobacterium* and mRNA profile, shows that the screening method of the present invention is feasible. Further, although there were also mRNAs of which amounts in milk were not changed by administration of the *Bifidobacterium,* a possibility that amounts of those mRNAs may be increased or decreased by another kind of diet or a substance contained therein is not denied.

As probiotic functions of *Bifidobacteria,* there are known prophylaxis or amelioration of respiratory tract infection, acute infectious diarrhea, antibiotic-associated diarrhea, *Clostridium dificile*-associated diarrhea, necrotizing enterocolitis, traveler's diarrhea, *Helicobacter pylori* infection, and so forth (The Journal of Nutrition, 2010 Mar;140(3):698S-712S. Epub 2010 Jan 27). It is suggested that mRNA of which amount in milk is increased by administration of *Bifidobacteria* not only regulates immunity, but also exhibits functions similar to the aforementioned probiotic functions in mammals that ingested them.

By giving a diet or a substance that increases amount of mRNA in milk functioning in an immunostimulating manner chosen as described above to a mammal, and collecting milk from the mammal, milk having an immunostimulating action or milk of which immunostimulating action is enhanced can be obtained. Further, by reducing or avoiding ingestion by a mammal of a diet or a substance that decreases amount of mRNA in milk functioning in an immunostimulating manner chosen as described above, an immunostimulating action of milk can be enhanced, or decrease of an immunostimulating action can be prevented. Ingestion of a diet or a substance that increases or decreases amount of mRNA in milk, and reduction or avoidance of ingestion of a diet or a substance that decreases or increases amount of mRNA in milk may be used in combination.

Further, by giving a diet or a substance that increases amount of mRNA in milk functioning in an immunosuppressive manner chosen as described above to a mammal, and collecting milk from the mammal, milk having an immunosuppressive action or milk of which immunostimulating action is decreased can be obtained. Further, by reducing or avoiding ingestion by a mammal of a diet or a substance that decreases amount of mRNA in milk functioning in an immunosuppressive manner chosen as described above, an immunosuppressive action of milk can be enhanced, or decrease of an immunosuppressive action can be prevented. Ingestion of a diet or a substance that decreases or increases amount of mRNA in milk, and reduction or avoidance of ingestion of a diet or a substance that increases or decreases amount of mRNA in milk may be used in combination.

By processing milk having an immunoregulatory action obtained as described above, a dairy product having an immunoregulatory action can be produced.

Type of the dairy product is not particularly limited, so long as mRNA can exist in it with maintaining the functions thereof, and examples include processed milk, infant formula, milk beverages, powdered infant formula, fermented milk, cream, butter, cheese, ice cream, and so forth. As the dairy product, a dairy product for infants or little children is preferred.

According to the present invention, there was demonstrated presence in milk of mRNAs, especially mRNAs which have been known to participate in enhancement of immunity, such as development of immunity, antiallergy, anti-inflammation, and defense against infection. In addition, it is well known that breast milk gives an immunostimulating action to an infant who ingested it. Therefore, it is rationally predicted that mRNA participating in immunoregulation can regulate immunity of organism such as human who ingested it. Therefore, it is considered that transfer of various mRNAs from a mother to an infant is extremely significant for, in particular, infants having an underdeveloped immune system.

Another aspect of the present invention is a method for producing a composition for oral ingestion having an immunostimulating action, which comprises adding a mRNA of which amount in milk of a rat increases when a *Bifidobacterium* is orally administered to the rat to a base for a composition for oral ingestion. Examples of the mRNA include those mentioned in Tables 1 to 14 mentioned later.

Still another aspect of the present invention is a method for producing a composition for oral ingestion having an immunosuppressive action, which comprises adding a mRNA of which amount in milk of a rat decreases when a *Bifidobacterium* is orally administered to the rat to a base for a composition for oral ingestion. Examples of the mRNA include those mentioned in Tables 15 to 22 mentioned later.

The mRNA may consist of a single kind of mRNA or arbitrary two or more kinds of mRNAs.

The base for composition for oral ingestion is not particularly limited so long as an orally ingestible or administrable base in which mRNA can exist with maintaining functions thereof is chosen, and examples include foods, drinks, drug bases, feeds, and so forth.

Foods may be in any form, and include drinks. Foods include foods for adults, foods for infants, foods for little children, and so forth.

Examples of the foods for adults include enteral nutrients, fluid diets such as concentrated fluid diets, nutritional supplementary foods, and so forth.

Examples of the foods for infants or the foods for little children include, for example, modified milks (for example, infant formula, infant formula for low birth weight infants, follow-on formula, etc. as well as infant formula for allergic infants, non-lactose milk, special milk for inborn errors of metabolism infants, etc., and dried milk prepared from these), powders for supplement of breast milk or powdered infant formula, baby food, and so forth.

The infant formula referred to here are foods produced by using milk or dairy products as main raw materials, and adding nutrients required for infants, and are mainly used as alternative food for breast milk in infancy, and as alternative food for breast milk or nutritional complementary food in childhood. Other examples thereof include foods produced for the purpose of contributing to nutritional ingestion suitable for infants with a specific inherent or acquired disease.

mRNA is relatively resistant to freeze-thaw, low pH such as acidic condition of pH 2, and RNases (Hata, T. et al., Biochem. Biophys. Res. Commun., 396:528-533, 2010), and thus is suitable as an active ingredient to be added to foods. The stability at a low pH suggests that mRNA molecules are resistant to the infant's intragastric environment, and can be absorbed by the intestinal tract, which is one of the main immune organs of infants, and thus they can affect the immune system of infants. Further, storage and freeze-thaw of breast milk do not denature mRNA, and this is nutritionally important for low birth weight infants and hospitalized infants, who are usually given cryopreserved breast milk. Furthermore, the resistance of mRNA to RNases suggests that mRNA may exist in a complex such as exosome and microvesicle in breast milk. Similarly, when mRNA is added to a composition for oral ingestion, mRNA may be micellized by using, for example, lipid bilayer or the like.

Although content of mRNA in the composition for oral ingestion is not particularly limited, and may be appropriately chosen, it is, for example, 10 to 10,000 ng/ml, preferably 20 to 10,000 ng/ml, more preferably 50 to 10,000 ng/ml, in total. Further, amount of mRNA to be ingested is, for example, 5 µg to 120 mg/day, preferably 10 µg to 120 mg/day, more preferably 25 µg to 120 mg/day, in total.

mRNA can be prepared by, for example, gene engineering using a cell introduced with an expression vector that expresses a corresponding gene.

Further, mRNA can also be prepared by chemical synthesis. That is, mRNA can be obtained by synthesizing a sense strand and an antisense strand and annealing them.

Further, mRNA can also be prepared from breast milk or a fractionation product thereof containing mRNA. For example, mRNA that functions in an immunostimulating manner, for example, a mRNA of which amount in milk of a rat increases when a *Bifidobacterium* is orally administered to the rat, can be collected from breast milk of a mammal given a diet or a substance that increases amount of the mRNA in milk. Further, mRNA that functions in an immunosuppressive manner, for example, a mRNA of which amount in milk of a rat decreases when a *Bifidobacterium* is orally administered to the rat, can be collected from breast milk of a mammal given a diet or a substance that increases amount of the mRNA in milk. mRNA may be a purified product or a crude product. mRNA that functions in an immunostimulating manner may contain a small amount of mRNA that functions in an immunosuppressive manner, so long as the immunostimulating action of the mRNA is not degraded. Further, mRNA that functions in an immunosuppressive manner may contain a small amount of mRNA that functions in an immunostimulating manner, so long as the immunosuppressive action of the mRNA is not degraded.

When the composition for oral ingestion of the present invention is a pharmaceutical agent, the composition can be prepared by combining mRNA with pharmaceutically acceptable carriers for oral administration. The form of the pharmaceutical preparation is not particularly limited, and examples include tablet, pill, powder, solution, suspension, emulsion, granule, capsule, syrup, and so forth. For the formulation, additives widely used for usual pharmaceutical agents as pharmaceutical carriers for oral administration such as excipients, binders, disintegrating agents, lubricants, stabilizers, corrigents, diluents, and surfactants can be used. Further, unless the effect of the present invention is degraded, mRNA may be used together with another drug having an immunoregulatory action.

Although amount of mRNA contained in the pharmaceutical agent is not particularly limited, and can be appropriately chosen, it is, for example, 2 µg/kg to 2 mg/kg, preferably 4 µg/kg to 2 mg/kg, more preferably 10 µg/kg to 2 mg/kg, in total.

When the composition for oral ingestion is a food, it may be for any of various uses utilizing an immunostimulating action. Examples of the use include, for example, uses as foods suitable for persons showing reduced resistance, uses as foods or drinks useful for reduction and elimination of risk factors of various diseases caused by immune depression, and so forth.

The foods or drinks of the present invention can be marketed as foods attached with an indication describing that the foods or drinks are used for immunoregulation.

The aforementioned term "indication" includes all actions for informing consumers the aforementioned use, and any indications reminding or analogizing the aforementioned use fall within the scope of the "indication" of the present invention regardless of purpose, content, objective article, medium etc. of the indication. However, the indication is preferably made with an expression that allows consumers to directly recognize the aforementioned use. Specific examples include actions of indicating the aforementioned use on goods or packages of goods relating to the food of the present invention, actions of assigning, delivering, displaying for the purpose of assigning or delivering or importing such goods or packages of goods on which the aforementioned use is indicated, displaying or distributing advertisements, price lists or business papers relating the goods on which the aforementioned use is indicated, or providing information including those as contents with indicating the aforementioned use by an electromagnetic method (Internet etc.) and so forth.

The indication is preferably an indication approved by the administration etc. (for example, an indication in a form based on an approval, which is qualified on the basis of any of various legal systems provided by the administration), and it is particularly preferably an indication on advertisement materials at the sales spots such as packages, containers, catalogs, pamphlets and POPs (point of purchase advertising), other documents and so forth.

Examples of the indication further include, for example, indications as health food, functional food, enteric nutritive food, food for special dietary uses, food with nutrient function claims, quasi-drug and so forth as well as indications approved by the Ministry of Health, Labor and Welfare, for example, indications approved on the basis of the system of food for specified health uses and similar systems. Examples of the latter include indications as food for specified health uses, indications as food for specified health uses with qualified health claims, indications of influence on body structures and functions, indications of reduction of disease risk claims and so forth, and more precisely, typical examples include indications as food for specified health uses (especially indications of use for health) provided in the enforcement regulations of Health Promotion Law (Japanese Ministry of Health, Labor and Welfare, Ministerial ordinance No. 86, April 30, 2003) and similar indications.

As shown in the examples, rat milk contained many kinds of mRNAs derived from genes coding for hormones such as growth hormone, cholecystokinin, and gastrin, growth factors such as epidermal growth factor, fibroblast growth factor, platelet-derived growth factor, and vascular endothelial growth factor, enzymes such as fatty acid synthase, lipoprotein lipase, and acyl-CoA synthase, and so forth. It is considered that these proteins have an action of promoting growth of children (baby mammals). Therefore, the present disclosure can be similarly applied to screening for a diet or a substance that provides production of breast milk having an enhanced action of promoting growth of children (baby mammals), a method for producing milk or a dairy product having such an action, and a method for producing a composition for oral ingestion having such an action.

### EXAMPLES

Hereafter, the present invention will be further specifically explained with reference to examples. However, the present invention is not limited to the following examples.

### Example 1: Analysis of mRNA in rat milk

Three F344 rats on day 14 of gestation were purchased. All the purchased rats gave birth on day 21 of gestation, and they were milked 2, 9 and 16 days after the delivery under ether anesthesia to collect milk. Further, they were dissected 21 days after the delivery, which corresponded to the weaning day, and blood was collected from the inferior vena cava.

Each milk sample was centrifuged at 1,200 x g and 4°C for 10 minutes, and the lipid layer and cell debris were removed.

Then, each sample was centrifuged twice at 21,500 x g and 4°C for 30 minutes, further centrifuged once under the same conditions for 1 hour to remove the casein fraction, and passed through membranes of 0.65 µm, 0.45 µm and 0.22 µm to remove cell debris and insoluble matter and thereby obtain a milk serum sample.

The blood obtained by the blood collection was centrifuged at 1,200 x g and 4°C for 10 minutes, and passed through membranes of 0.65 µm, 0.45 µm and 0.22 µm to remove cell debris and insoluble matter and thereby obtain a blood serum sample.

Total RNAs were obtained from the obtained milk serum sample and the blood serum sample by using miRNeasy Mini Kit (Qiagen). Since the blood serum sample contained only a small amount of RNA, the blood sera of three rats were combined, and the total RNA was obtained from the combined blood serum.

After quality of each obtained RNA solution was confirmed by using BioAnalyzer, 100 ng of RNA was used to synthesize cRNA by using Low RNA Input Liner Amplification Kit (Agilent Technologies). At the time of this synthesis, CPT labeled with a cyanine dye (Cyanine 3-CPT) was incorporated into cRNA to attain fluorescence labeling of cRNA. The labeled cRNA was added to a hybridization buffer, allowed to hybridize on Whole Rat Genome Oligo Microarray (4 x 44 K, Agilent Technologies) for 17 hours, washed, and then imaged with Agilent Microarray Scanner (Agilent Technologies), and the fluorescent signals of the spots were numerically represented by using Feature Extraction Software. The results were analyzed by using GeneSpring GX11.0 (Agilent Technologies).

As a result, in the milk serum sample, mRNAs corresponding to 10,948 kinds of probes were detected, and in the blood serum sample, mRNAs corresponding to 2,361 kinds of probes were detected. The mRNAs detected in the milk serum sample included many mRNAs derived from genes coding for proteins relating to the immune system, such as cytokines, chemokines, and antimicrobial peptides. In fact, when biological interpretation was performed by using Ingenuity Pathway Analysis (Ingenuity Systems), it was demonstrated that many mRNAs relating to pathways of "infection mechanism" or "infectious disease" were included. An action of defending children (baby mammals) from infection is known for milk, and interestingly, the defense from infection has also been suggested on the mRNA level. In addition, many mRNAs derived from genes coding for proteins that promote growth of children (baby mammals) such as hormones, growth factors, and enzymes were also contained.

### Example 2: Identification of diet or substance providing production of milk having immunoregulatory action

SD rats on day 9 to 10 of gestation were purchased, and during the period from day 13 to day 20 of gestation, a suspension in which cells of a *Bifidobacterium, Bifidobacterium longum* BB536 (ATCC BAA-999), were suspended in PBS (phosphate buffered saline) (1 x 10⁹ cfu/ml) was orally administered everyday to the rats of test group (n = 3) in a volume of 1 ml/day per rat.

Further, 1 ml/day per rat of PBS was administered everyday to the rats of control group (n = 3). The B. *longum* ATCC BAA-999 strain can be purchased from American Type Culture Collection (Address: 12301 Parklawn Drive, Rockville, Maryland 20852, United States of America).

All the rats gave birth on day 21 of gestation, and they were milked under ether anesthesia one day after the birth. Each obtained milk sample was centrifuged at 1,200 x g and 4°C for 10 minutes, and the lipid layer and cell debris were removed.

Then, each sample was centrifuged twice at 21,500 x g and 4°C for 30 minutes, further centrifuged once under the same conditions for 1 hour to remove the casein fraction, and passed through membranes of 0.65 µm, 0.45 µm and 0.22 µm to remove cell debris and insoluble matter and thereby obtain a milk serum sample.

Total RNA was obtained from the obtained milk serum sample by using miRNeasy Mini Kit (Qiagen). By using 100 ng of the obtained RNA sample, detection of mRNAs and analysis of expression amounts thereof were conducted in the same manner as that of Example 1.

As a result, number of probes corresponding to mRNAs confirmed in the test group and the control group was 10,239.

Further, the expression amounts in the *Bifidobacterium longum* BB536 strain-administered group and the control group were compared, and evaluated by the Mann-Whitney U-test. As a result, at a probability level of P < 0.05, number of mRNAs that increased 1.5 times or more was 562, and number of mRNAs that decreased to 0.67 times or less was 325. These mRNAs and rate of change (RC) in expression amounts thereof are shown in Tables 1 to 22. The mRNAs of which expression amounts increased are mentioned in Tables 1 to 14, and the mRNAs of which expression amounts decreased are mentioned in Tables 15 to 22.
[Table 1]

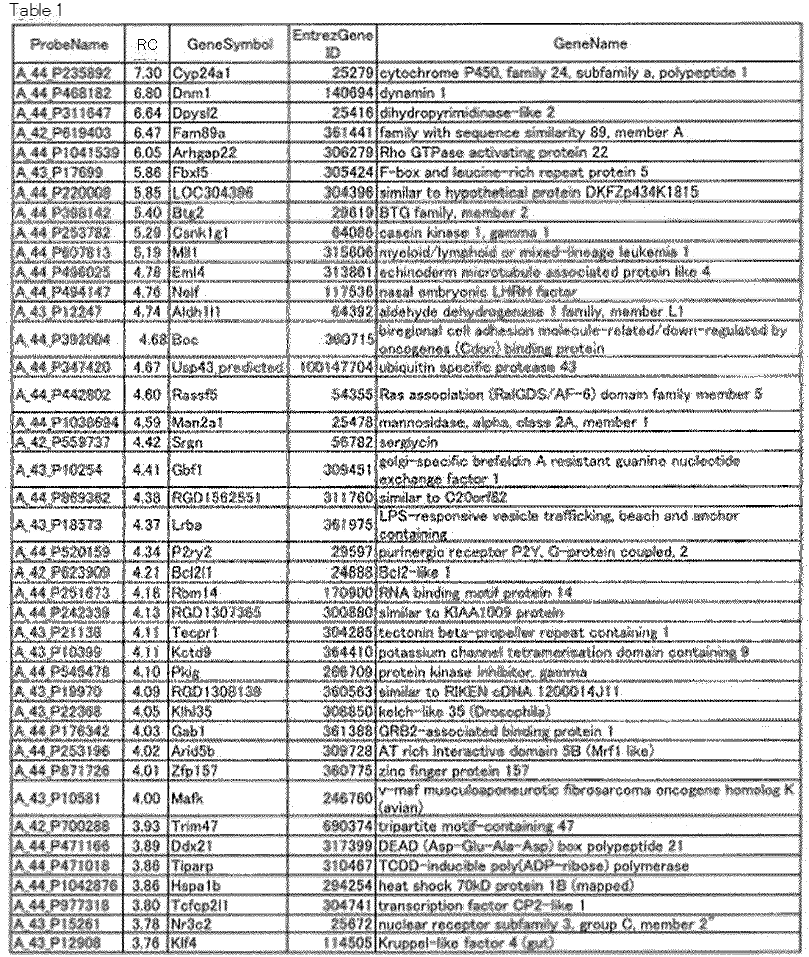

[Table 2]

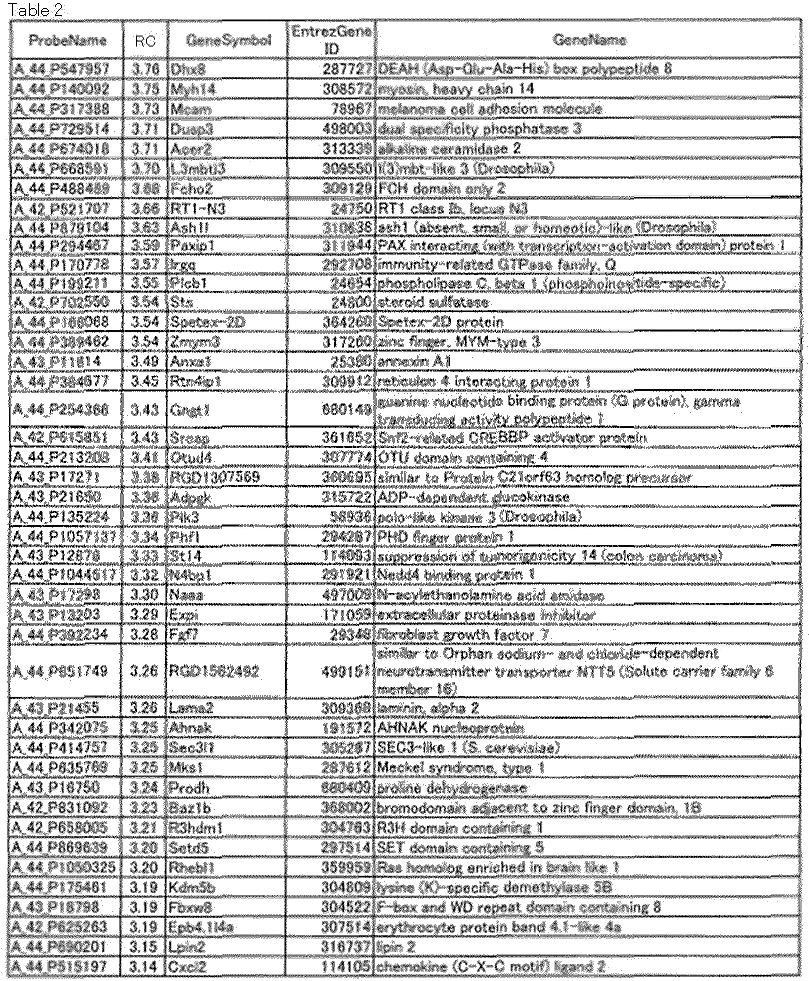

[Table 3]

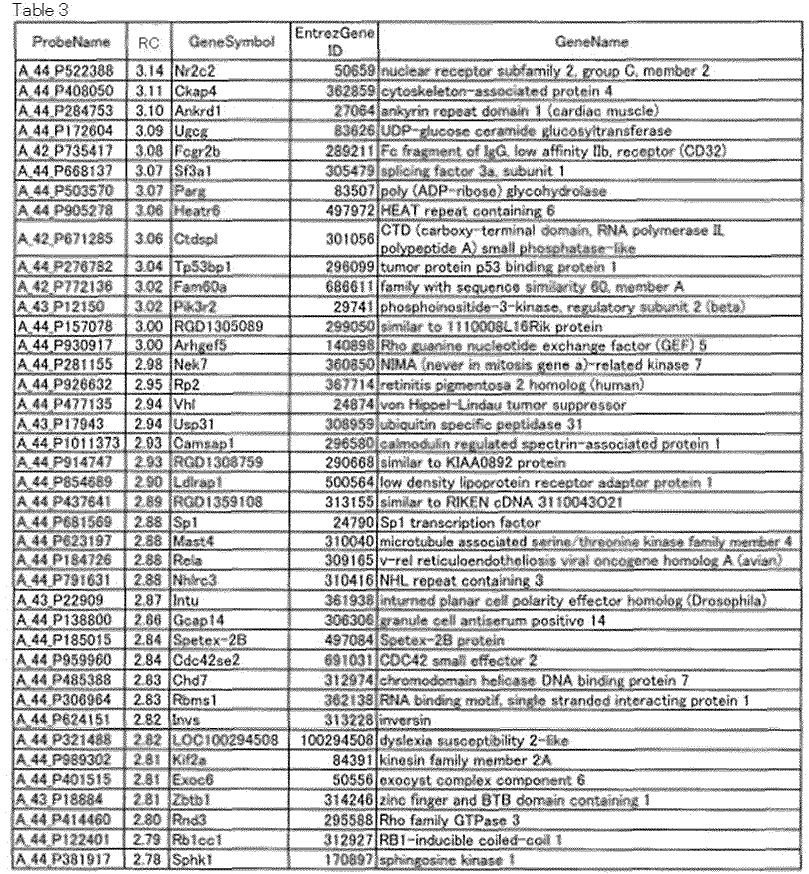

[Table 4]

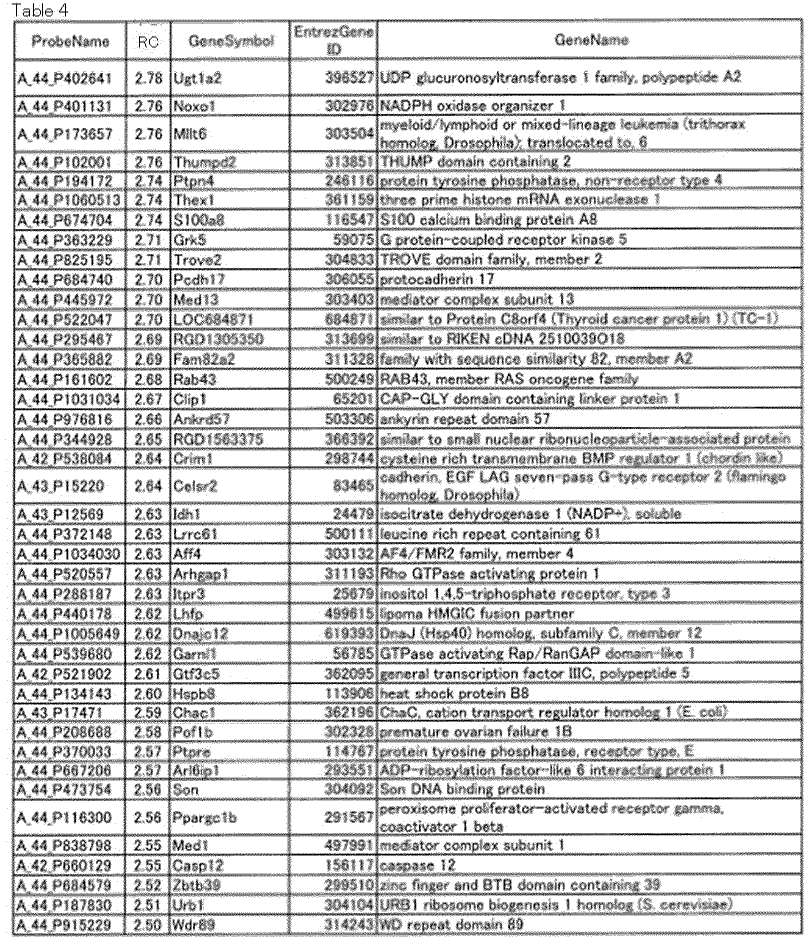

[Table 5]

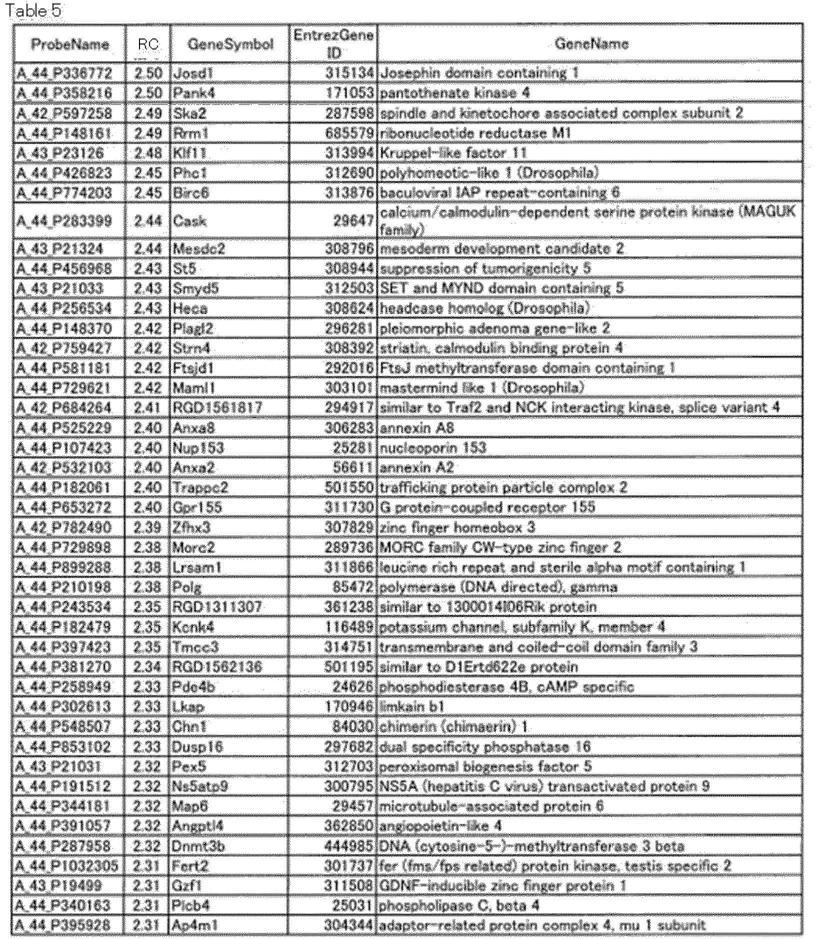

[Table 6]

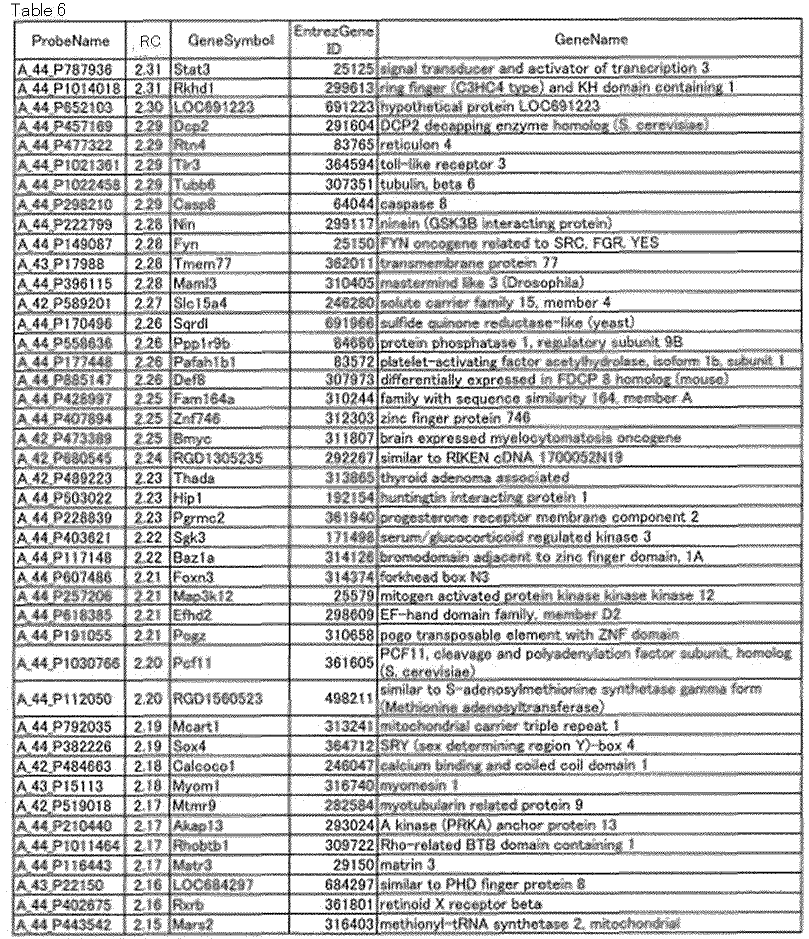

[Table 7]

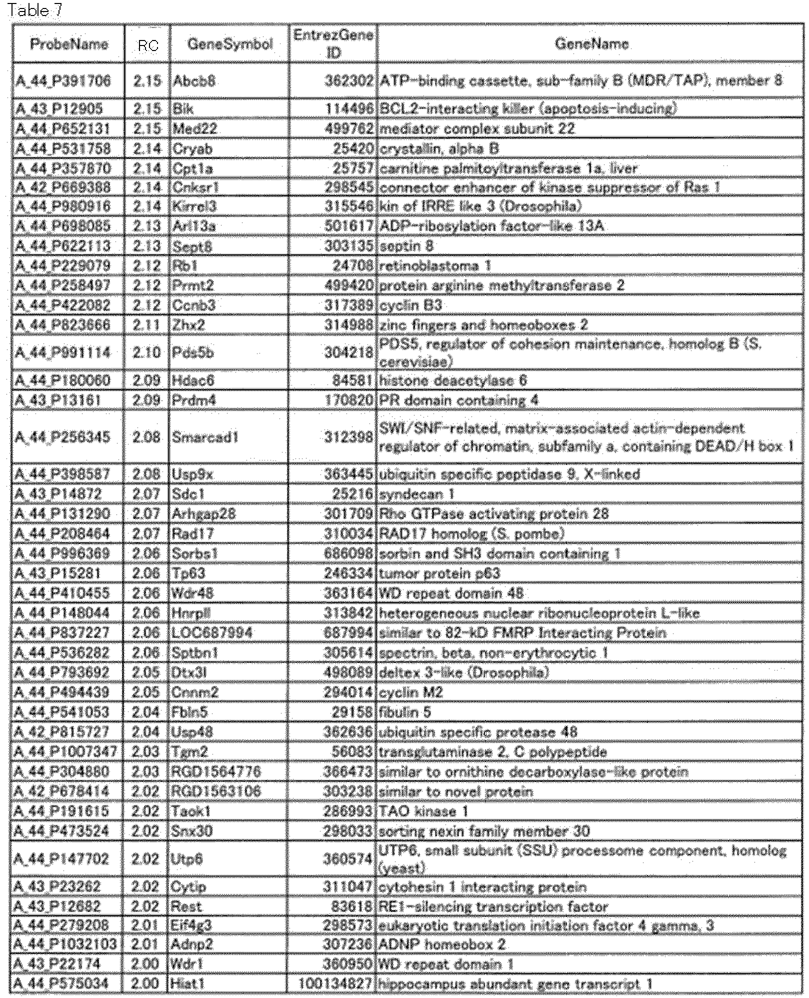

[Table 8]

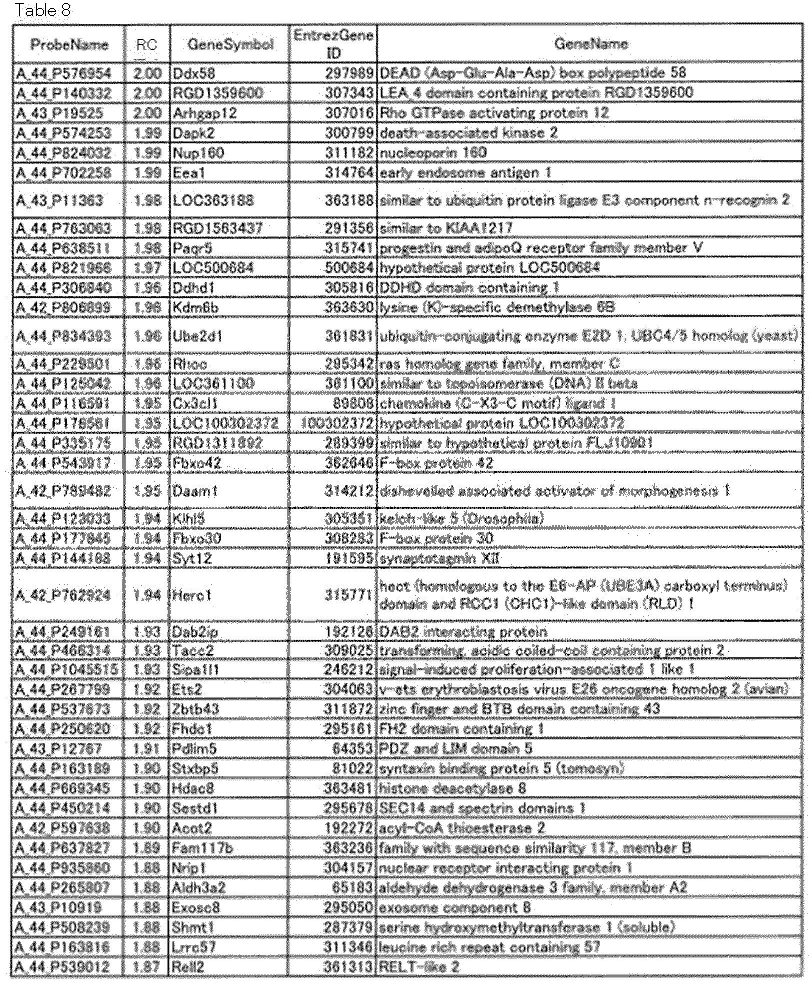

[Table 9]

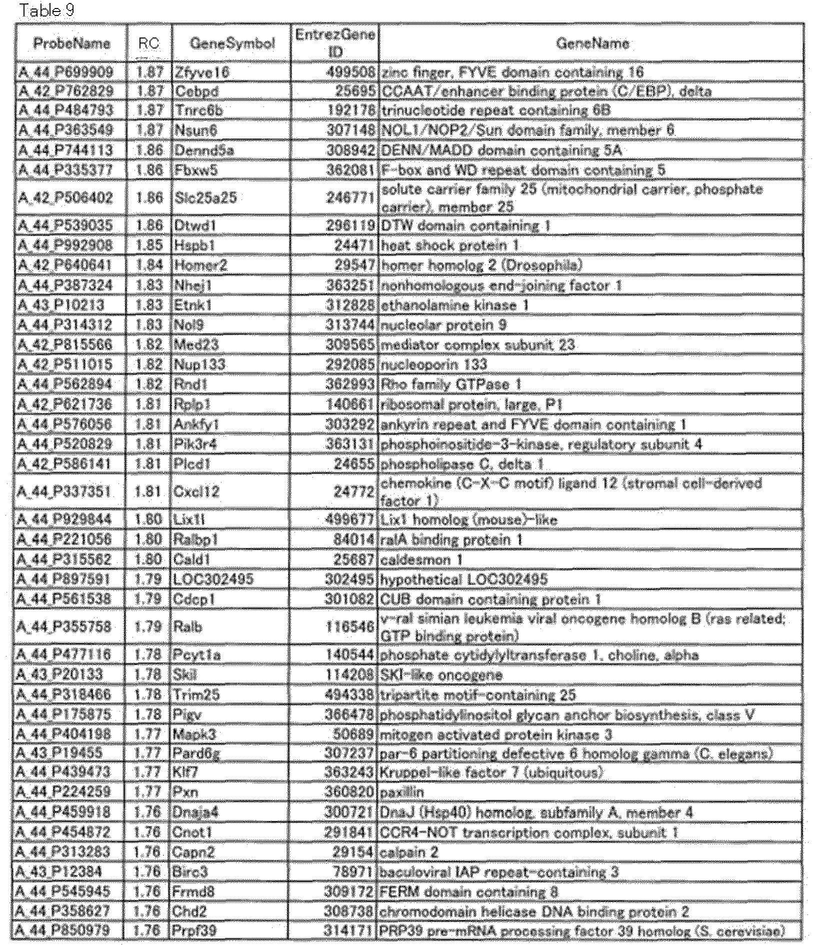

[Table 10]

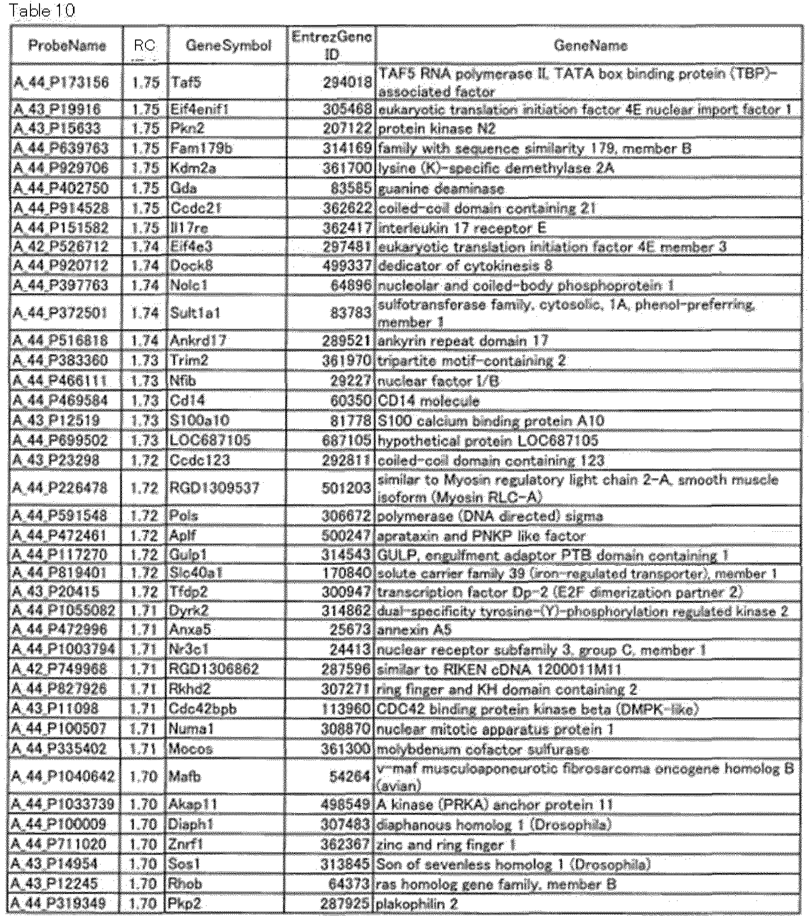

[Table 11]

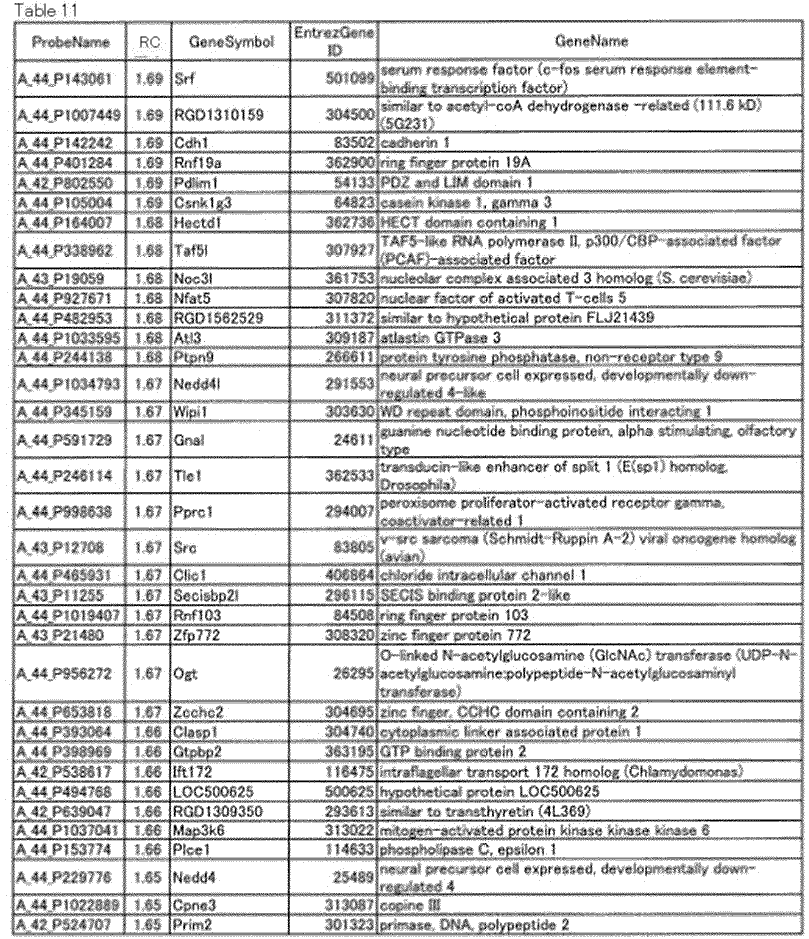

[Table 12]

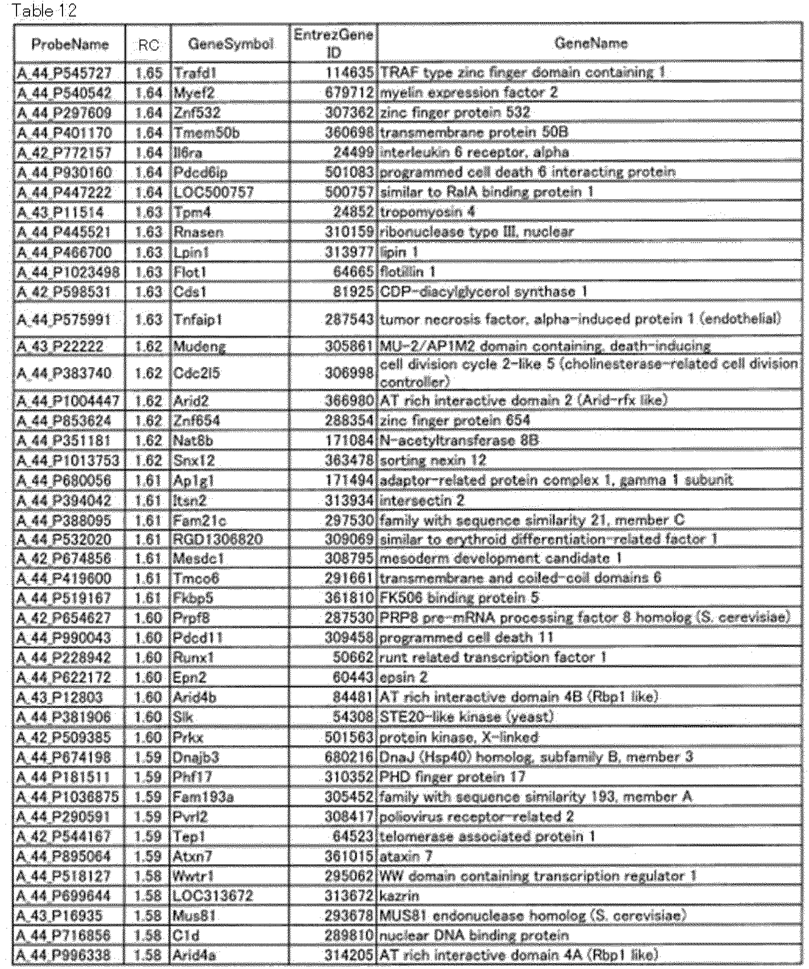

[Table 13]

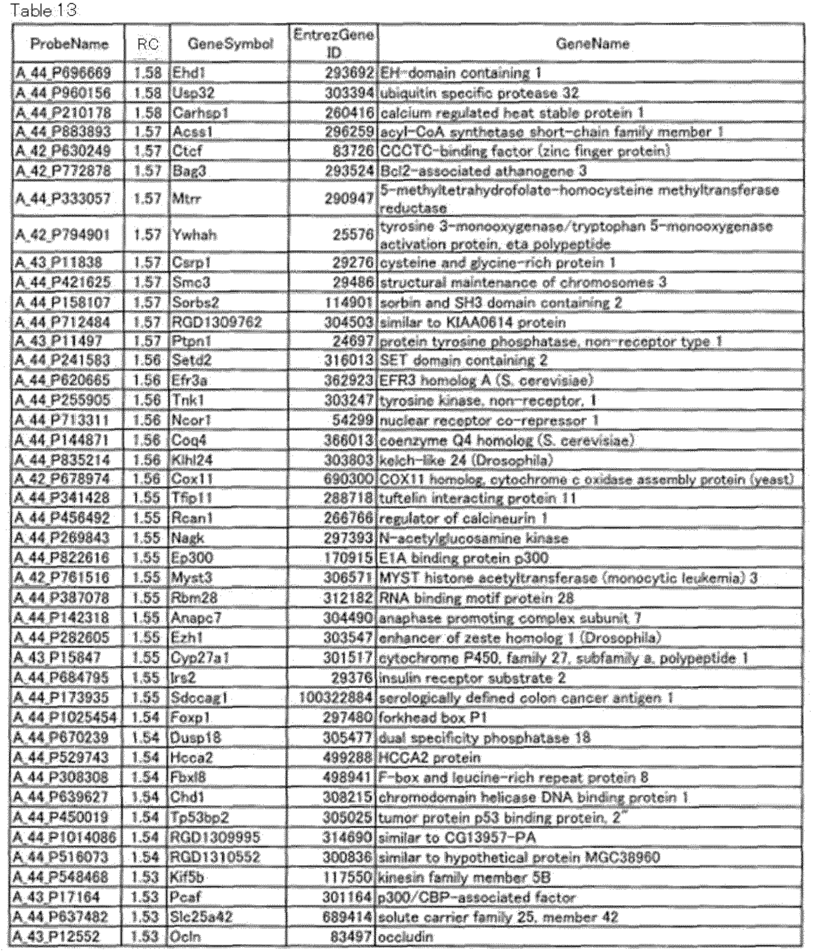

[Table 14]

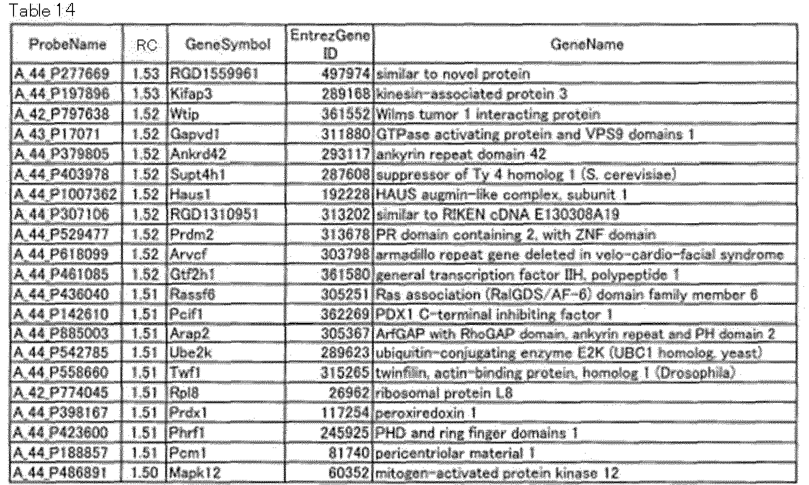

[Table 15]

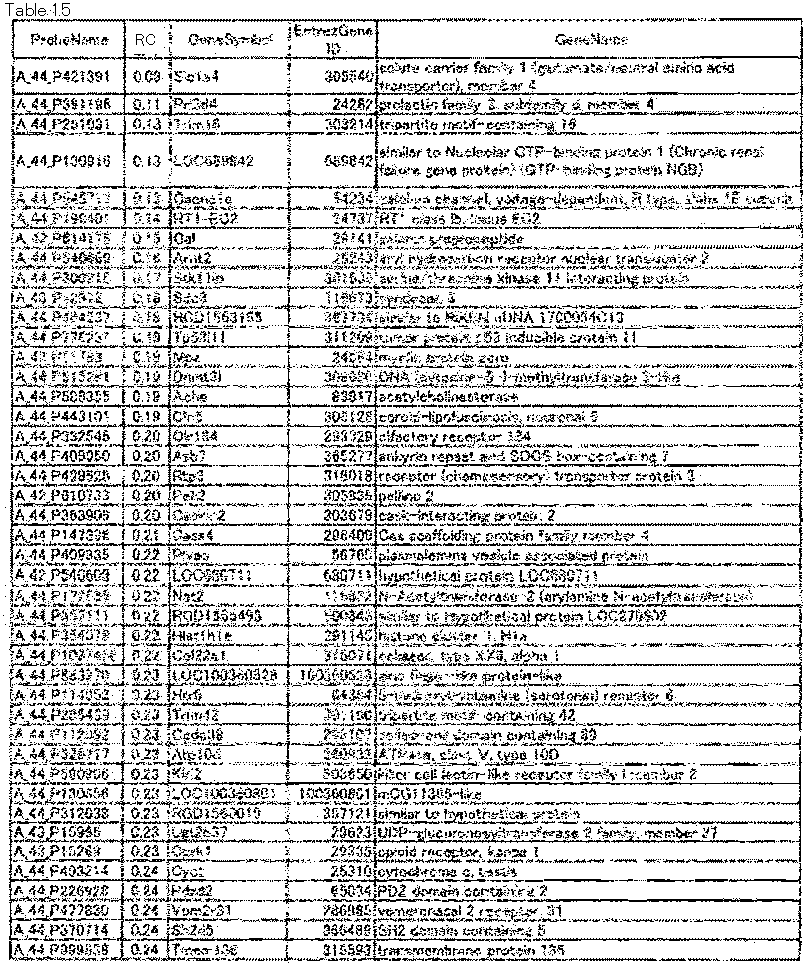

[Table 16]

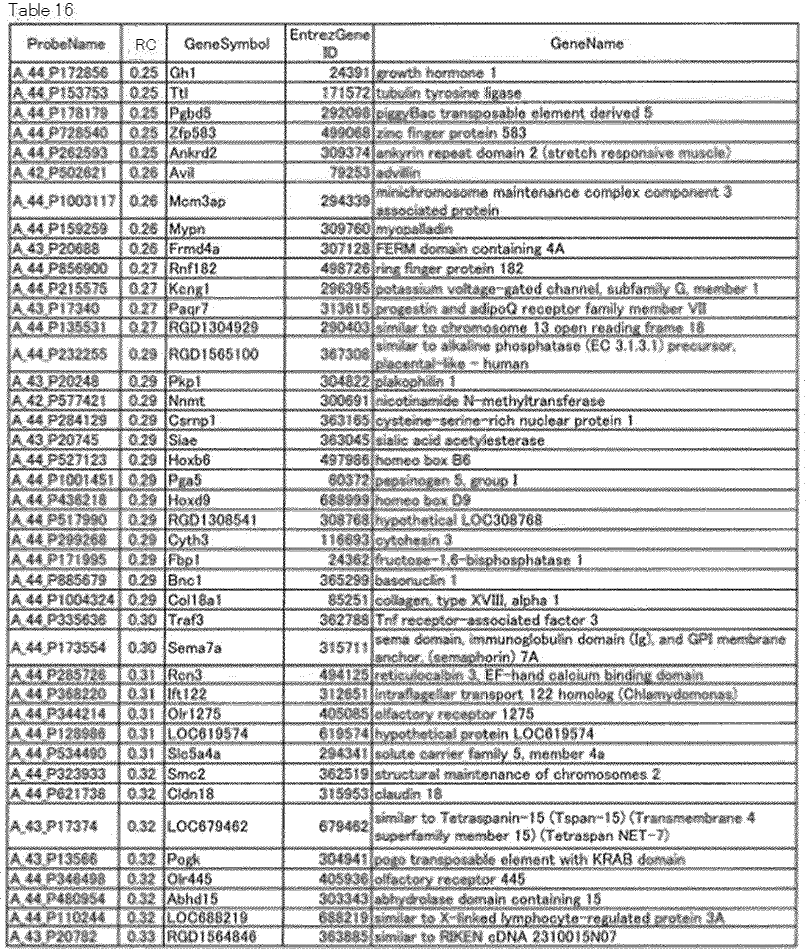

[Table 17]

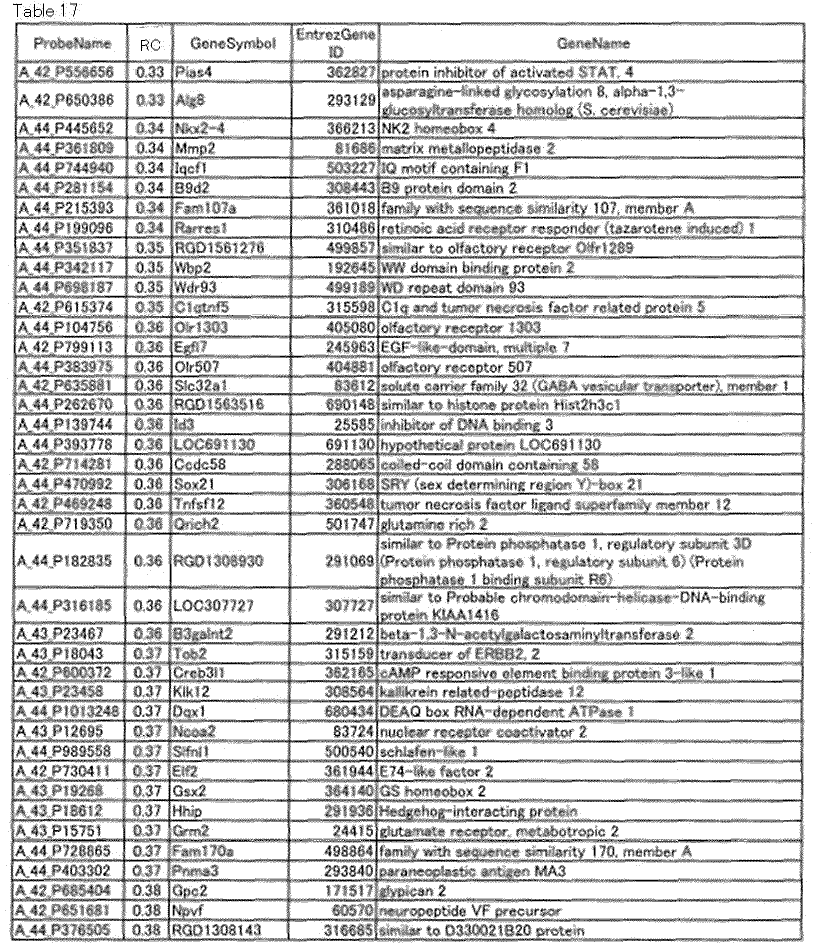

[Table 18]

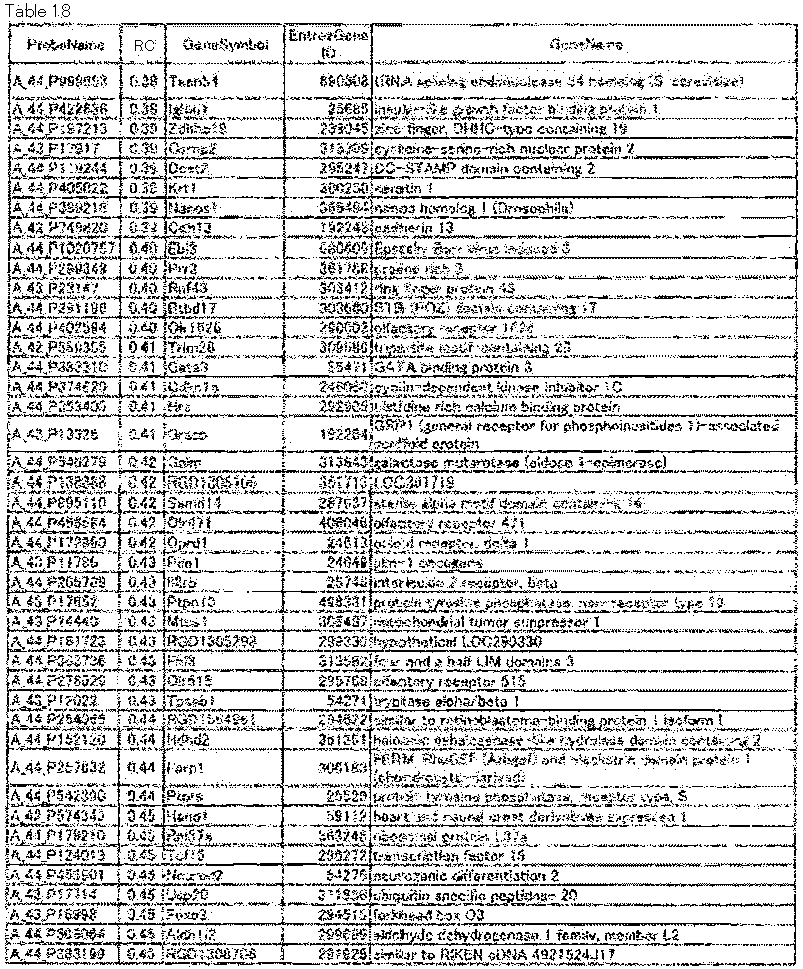

[Table 19]

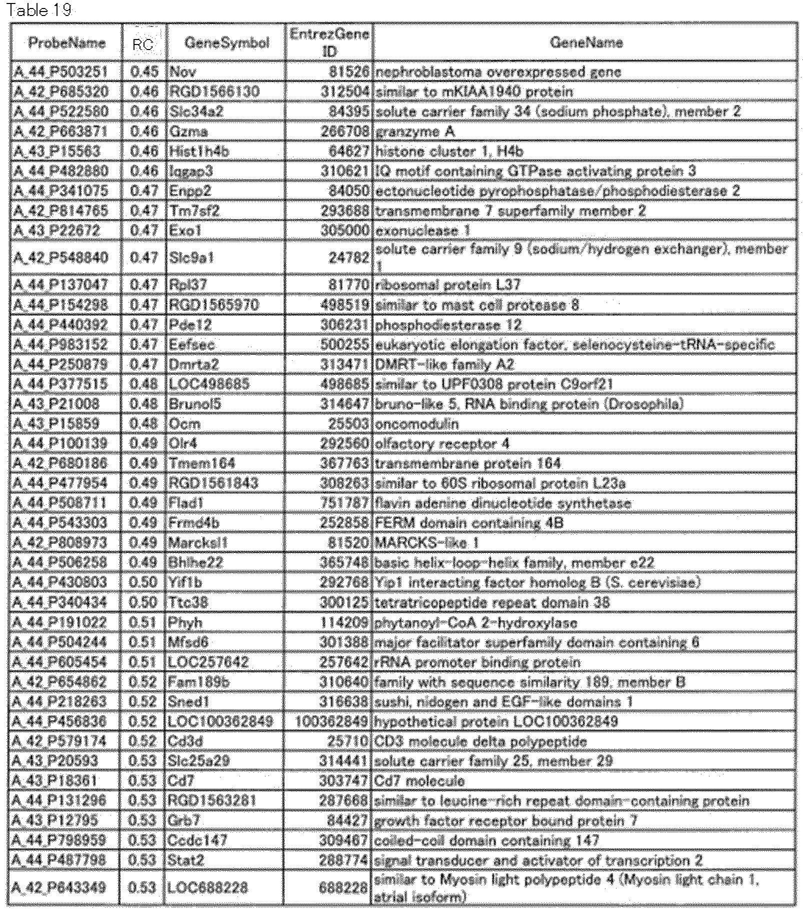

[Table 20]

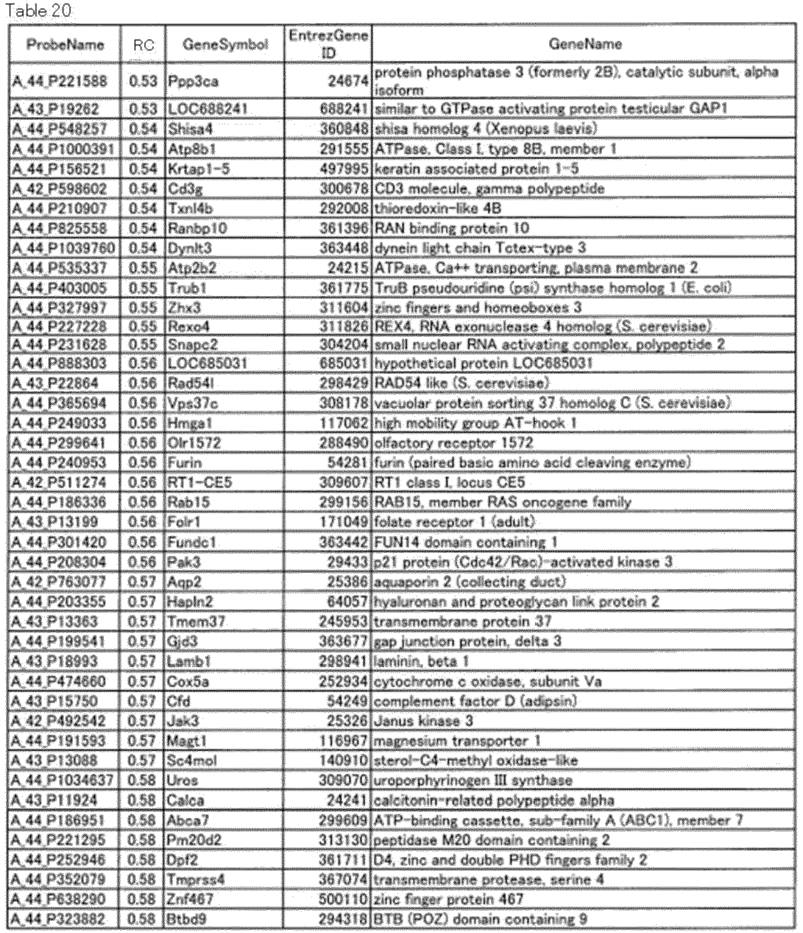

[Table 21]

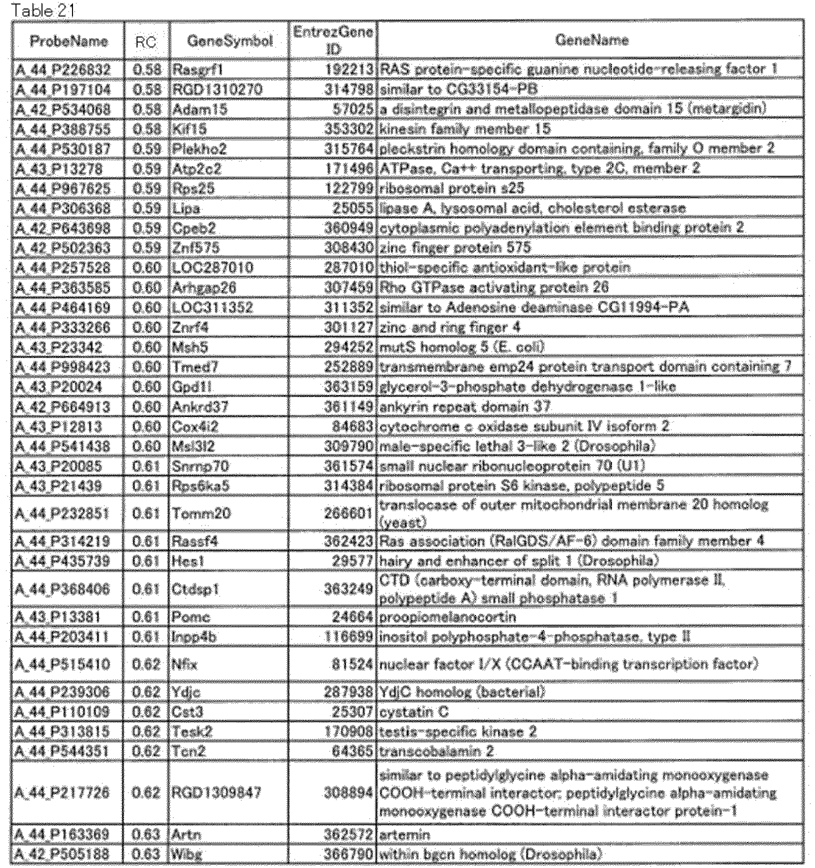

[Table 22]

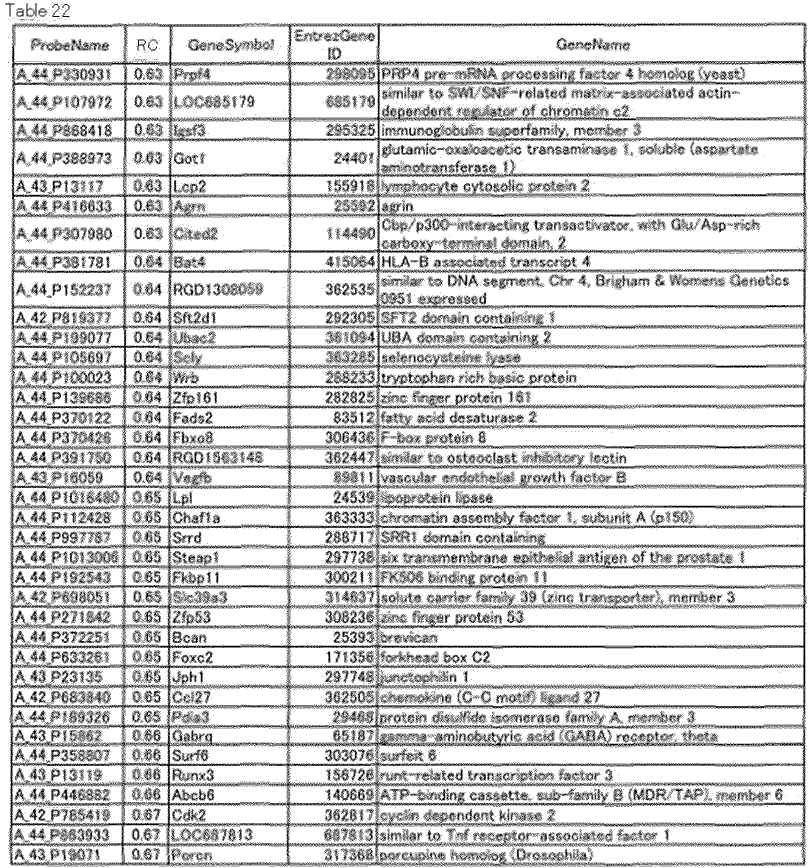

The mRNAs of which expression amounts increased included those derived from genes coding for cytokine receptors such as Il6ra and Il-17re, which are receptors of IL-6 and IL-17 playing important roles in defense against infection, and genes coding for chemokines such as Cxcl2 and Cxcl12, which induce migration of neutrophils to function for the defense against infection, and Cx3cl1, which induce migration of cells expressing CX3CR1 (monocytes, NK cells, T cells, etc.) to function for the defense against infection. It is considered that increase in expression of these genes contributes to immunostimulation.

Further, the mRNAs of which expression amounts increased also included those derived for genes coding for such proteins as Cd14, which is considered to bind with LPS (lipopolysaccharide) of Gram-negative bacteria to serve as a cofactor of TLR4, thereby suppress IgE and exhibit an antiallergic action, and Tlr3, which is mainly expressed in cells responsible for natural immunity such as dendritic cells and macrophages, and recognizes double stranded RNAs derived from viruses to function for the defense against infection. It is considered that increase of expression of Cd14 contributes to suppression of allergy.

On the other hand, the mRNAs of which expression amounts decreased included those derived for genes coding for Cc127, which is known to increase in serum of atopic dermatitis patients, and Gata3, which is a Th2 cell-specific transcription factor. It is considered that decrease of expression of such genes contributes to suppression of allergy.

The *Bifidobacterium longum* BB536 is known to have an antiallergic action, and it has been suggested that this action is not based on enhancement of the Th1 reaction like lactic acid bacteria, but based on suppression of the Th2 reaction (Takahashi, N. et al., Clin. Exp. Immunol., 145:130-138, 2006). It is said that neonates are generally in a Th2-dominant state, and it is considered that continuous bias of Th1/Th2 balance to the Th2 side is the cause of onset of allergy. Therefore, it is considered that such suppression of the Th2 reaction as mentioned above promotes normal development of immunity in infants.

In addition, many mRNAs derived from genes coding for proteins that promote growth of children (baby mammals), for example, hormones such as growth hormone, cholecystokinin and gastrin, growth factors such as epidermal growth factor, fibroblast growth factor, platelet-derived growth factor, and vascular endothelial growth factor, enzymes such as fatty acid synthase, lipoprotein lipase, and acyl-CoA synthase, and so forth were also contained.

### INDUSTRIAL APPLICABILITY

According to the present invention, a diet or a component contained therein that provides production of milk having an immunoregulatory action can be screened for. Further, a method for producing a dairy product having an immunoregulatory action is provided by the present invention. The composition for oral ingestion of the present disclosure has an immunostimulating action or an immunosuppressive action, and is useful especially for infants.

## Claims

1. A method for screening for a diet or a substance providing production of breast milk having an immunostimulating action, which comprises identifying a diet or a substance that increases or decreases amount of mRNA present in milk of a mammal by using correlation of mRNA profile in the milk and a diet ingested by the mammal or a substance contained in the diet as an index,
wherein the mRNA profiles-comprises amount of mRNA selected from the group consisting of Cyp24a1, Dnm1, Dpysl2, Fam89a, Arhgap22, Fbxl5,Btg2, Csnklg1, Mll1, Eml4, Nelf, Aldh1l1, Boc, Usp43_predicted, Rassf5, Man2a1, Srgn, Gbf1, RGD1562551, Lrba, P2ry2, Bcl211, Rbm14, RGD1307365, Tecpr1, Kctd9, Pkig, RGD1308139, Klhl35, Gab1, Arid5b, Mafk, Trim47, Ddx21, Tiparp, Hspalb, Nr3c2, Klf4, Dhx8, Myh14, Mcam, Dusp3, Acer2, L3mbtl3, Fcho2, Ash1l, Paxip1, Irgq, Plcb1, Sts, Zmym3, Anxa1, Rtn4ip1, Gngt1, Srcap, Otud4, RGD1307569, Adpgk, Plk3, Phf1, St14, N4bp1, Naaa, Fgf7, Lama2, Ahnak, Sec3l1, Mks1, Prodh, Bazlb, R3hdm1, Setd5, Rhebl1, Kdm5b, Fbxw8, Epb4.114a, Lpin2, Cxcl2, Nr2c2, Ckap4, Ankrd1, Ugcg, Fcgr2b, Sf3a1, Parg, Heatr6, Ctdspl, Tp53bp1, Fam60a, Pik3r2, Arhgef5, Nek7, Rp2, Vhl, Usp31, Camsap1, RGD1308759, Ldlrap1, Sp1, Mast4, Rela, Nhlrc3, Intu, Gcap14, Cdc42se2, Chd7, Rbms1, Invs, Kif2a, Exoc6, Zbtb1, Rnd3, Rb1cc1, Sphk1, Noxo1, Mllt6, Thumpd2, Ptpn4, Thex1, S100a8, Grk5, Trove2, Pcdh17, Med13, Fam82a2, Rab43, Clip1, Ankrd57, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargclb, Med1, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5,Map6, Angptl4, Dnmt3b, Gzf1, Plcb4, Ap4m1, Stat3, Rkhd1, Dcp2, Rtn4, Tlr3, Tubb6, Casp8, Nin, Fyn, Tmem77, Maml3, Slc15a4, Sqrdl, Ppplr9b, Pafahlb1, Def8, Fam164a, Znf746, RGD1305235, Thada, Hip1, Pgrmc2, Sgk3, Baz1a, Foxn3, Map3k12, Efhd2, Pogz, Pcf11, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrpll, Sptbn1, Dtx3l, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, Paqr5, Ddhd1, Kdm6b, Ube2d1, Rhoc, LOC361100, Cx3c11, Fbxo42, Daam1, Klhl5, Fbxo30, Syt12, Herc1, Dab2ip, Tacc2, Sipa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Re112, Zfyve16, Cebpd, Tnrc6b, Nsun6, Dennd5a, Fbxw5, Slc25a25, Dtwd1, Hspb1, Homer2, Nhej1, Etnk1, No19, Med23, Nup133, Rnd1, Rplp1, Ankfy1, Pik3r4, Plcd1, Cxcl12, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, Il17re, Eif4e3, Dock8, Nolc1, Sult1a1, Ankrd17, Trim2, Nfib, Cd14, S100a10, LOC68710S, Ccdc123, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, Cdh1, Rnf19a, Pdlim1, Csnklg3, Hectd1, Taf5l, Noc3l, Nfat5, RGD1562529, Atl3, Ptpn9, Nedd4l, Wipi1, Gnal, Tle1, Pprc1, Src, Clic1, Secisbp21, Rnf103, Ogt, Zcchc2, Clasp1, Gtpbp2, Ift172, Map3k6, Plce1, Nedd4, Cpne3, Prim2, Trafd1, Myef2, Znf532, Tmem50b, Il6ra, Pdcd6ip, Tpm4, Rnasen, Lpin1, Flot1, Cds1, Tnfaip1, Mudeng, Cdc2l5, Arid2, Znf654, Nat8b, Snx12, Ap1g1, Itsn2, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klhl24, Cox11, Tfip11, Rcan1, Nagk, Myst3, Rbm28, Anapc7, Ezh1, Cyp27a1, Irs2, Sdccag1, Foxp1, Dusp18, Hcca2, Fbxl8, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, Twf1, Rpl8, Prdx1, Phrf1, Pcm1, Mapk12, Slc1a4, Trim16, Cacnale, Gal, Arnt2, Stk11ip, Sdc3, Tp53i11, Mpz, Dnmt31, Ache, Cln5, Asb7, Rtp3, Peli2, Caskin2, Cass4, Plvap, Histlhla, Col22a1, Htr6, Trim42, Ccdc89, Atp10d, Oprk1, Cyct, Pdzd2, Sh2d5, Tmem136, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbp1, Bnc1, Col18a1, Traf3, Sema7a, Rcn3, Ift122, Smc2, Cldn18, LOC679462, Pogk, Abhd15, Pias4, Alg8, Nkx2-4, Mmp2, Iqcf1, B9d2, Fam107a, Rarres1, Wbp2, Wdr93, C1qtnf5, Egfl7, Slc32a1, Id3, Ccdc58, Sox21, Tnfsf12, Qrich2, RGD1308930, B3galnt2, Tob2, Creb311, Klk12, Dqx1, Ncoa2, Slfnl1, Elf2, Gsx2, Hhip, Grm2, Fam170a, Gpc2, Npvf, Tsen54, Igfbp1, Zdhhc19, Csrnp2, Dcst2, Krt1, Nanos1, Cdh13, Ebi3, Prr3, Rnf43, Btbd17, Trim26, Gata3, Cdkn1c, Hrc, Grasp, Galm, Samd14, Oprd1, Pim1, Il2rb Ptpn13, Mtus1, Fhl3, Hdhd2, Farp1, Ptprs, Hand1, Rpl37a, Tcf15, Neurod2, Usp20, Foxo3, Aldh1l2, Nov, RGD1566130, Slc34a2, Gzma, Iqgap3, Enpp2, Tm7sf2, Exo1, Slc9a1, Rpl37,Pde12, Eefsec, Dmrta2, LOC498685, Brunol5, Tmem164, Flad1, Frmd4b, Marcksl1, Bhlhe22, Yif1b, Ttc38, Phyh, Mfsd6, Fam189b, Sned1,Cd3d, Slc25a29, Cd7, RGD1563281, Grb7, Ccdc147, Stat2, Ppp3ca, LOC688241, Shisa4, Atp8b1, Cd3g, Txnl4b, Ranbp10, Dynlt3, Atp2b2, Trub1, Zhx3, Rexo4, Snapc2, Rad541, Vps37c, Hmga1, Furin, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpdl1, Ankrd37, Cox4i2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, Vegfb, Lpl, Chaf1a, Srrd, Steap1, Fkbp11, Slc39a3, Bcan, Foxc2, Jph1, Cc127, Pdia3, Gabrq, Surf6, Runx3, Abcb6, Cdk2, LOC687813, and Porcn, and
when either one or both of the following conditions are satisfied, it is determined that the diet or the substance provides production of breast milk having an immunostimulating action:
(A) the mRNA is a mRNA of which amount in milk of a rat increases when a *Bifidobacterium* is orally administered to the rat, and when mRNA profiles in the milk observed before and after ingestion of the diet are compared, amount of at least one kind of mRNA in the milk observed after the ingestion is 1.5 times or more of that observed before the ingestion,
(B) the mRNA is a mRNA of which amount in milk of a rat decreases when a *Bifidobacterium* is orally administered to the rat, and when mRNA profiles in the milk observed before and after ingestion of the diet are compared, amount of at least one kind of mRNA in the milk observed after the ingestion is 0.67 times or less of that observed before the ingestion.

2. A method for screening for a diet or a substance providing production of breast milk having an immunosuppressive action, which comprises identifying a diet or a substance that increases or decreases amount of mRNA present in milk of a mammal by using correlation of mRNA profile in the milk and a diet ingested by the mammal or a substance contained in the diet as an index,
wherein the mRNA profiles comprises amount of mRNA selected from the group consisting of Cyp24a1, Dnm1, Dpysl2, Fam89a, Arhgap22, Fbxl5,Btg2, Csnk1g1, Mll1, Eml4, Nelf, Aldh1l1, Boc, Usp43_predicted, Rassf5, Man2a1, Srgn, Gbf1, RGD1562551, Lrba, P2ry2, Bcl2l1, Rbm14, RGD1307365, Tecpr1, Kctd9, Pkig, RGD1308139, Klhl35, Gab1, Arid5b, Mafk, Trim47, Ddx21, Tiparp, Hspalb, Nr3c2, Klf4, Dhx8, Myh14, Mcam, Dusp3, Acer2, L3mbtl3, Fcho2, Ash1l, Paxip1, Irgq, Plcb1, Sts, Zmym3, Anxa1, Rtn4ip1, Gngt1, Srcap, Otud4, RGD1307569, Adpgk, Plk3, Phf1, St14, N4bp1, Naaa, Fgf7, Lama2, Ahnak, Sec3l1, Mks1, Prodh, Bazlb, R3hdm1, Setd5, Rhebl1, Kdm5b, Fbxw8, Epb4.1l4a, Lpin2, Cxcl2, Nr2c2, Ckap4, Ankrd1, Ugcg, Fcgr2b, Sf3a1, Parg, Heatr6, Ctdspl, Tp53bp1, Fam60a, Pik3r2, Arhgef5, Nek7, Rp2, Vhl, Usp31, Camsap1, RGD1308759, Ldlrap1, Sp1, Mast4, Rela, Nhlrc3, Intu, Gcap14, Cdc42se2, Chd7, Rbms1, Invs, Kif2a, Exoc6, Zbtb1, Rnd3, Rb1cc1, Sphk1, Noxo1, Mllt6, Thumpd2, Ptpn4, Thex1, S100a8, Grk5, Trove2, Pcdh17, Med13, Fam82a2, Rab43, Clip1, Ankrd57, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargclb, Med1, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5,Map6, Angptl4, Dnmt3b, Gzf1, Plcb4, Ap4m1, Stat3, Rkhd1, Dcp2, Rtn4, Tlr3, Tubb6, Casp8, Nin, Fyn, Tmem77, Maml3, Slc15a4, Sqrdl, Ppplr9b, Pafah1b1, Def8, Fam164a, Znf746, RGD1305235, Thada, Hip1, Pgrmc2, Sgk3, Baz1a, Foxn3, Map3k12, Efhd2, Pogz, Pcf11, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrpll, Sptbn1, Dtx3l, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, Paqr5, Ddhd1, Kdm6b, Ube2d1, Rhoc, LOC361100, Cx3c11, Fbxo42, Daam1, Klhl5, Fbxo30, Syt12, Herc1, Dab2ip, Tacc2, Sipa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Re112, Zfyve16, Cebpd, Tnrc6b, Nsun6, Dennd5a, Fbxw5, Slc25a25, Dtwd1, Hspb1, Homer2, Nhej1, Etnk1, No19, Med23, Nup133, Rnd1, Rplp1, Ankfy1, Pik3r4, Plcd1, Cxcl12, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, Il17re, Eif4e3, Dock8, Nolc1, Sult1a1, Ankrd17, Trim2, Nfib, Cd14, S100a10, LOC687105, Ccdc123, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, Cdh1, Rnf19a, Pdlim1, Csnklg3, Hectd1, Taf5l, Noc3l, Nfat5, RGD1562529, Atl3, Ptpn9, Nedd4l, Wipi1, Gnal, Tle1, Pprc1, Src, Clic1, Secisbp21, Rnf103, Ogt, Zcchc2, Clasp1, Gtpbp2, Ift172, Map3k6, Plce1, Nedd4, Cpne3, Prim2, Trafd1, Myef2, Znf532, Tmem50b, Il6ra, Pdcd6ip, Tpm4, Rnasen, Lpin1, Flot1, Cds1, Tnfaip1, Mudeng, Cdc2l5, Arid2, Znf654, Nat8b, Snx12, Ap1g1, Itsn2, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klhl24, Cox11, Tfip11, Rcan1, Nagk, Myst3, Rbm28, Anapc7, Ezh1, Cyp27a1, Irs2, Sdccag1, Foxp1, Dusp18, Hcca2, Fbxl8, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, Twf1, Rpl8, Prdx1, Phrf1, Pcm1, Mapk12, Slcla4, Trim16, Cacnale, Gal, Arnt2, Stk11ip, Sdc3, Tp53i11, Mpz, Dnmt31, Ache, Cln5, Asb7, Rtp3, Peli2, Caskin2, Cass4, Plvap, Histlhla, Col22a1, Htr6, Trim42, Ccdc89, Atp10d, Oprk1, Cyct, Pdzd2, Sh2d5, Tmem136, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbp1, Bnc1, Col18a1, Traf3, Sema7a, Rcn3, Ift122, Smc2, Cldn18, LOC679462, Pogk, Abhd15, Pias4, Alg8, Nkx2-4, Mmp2, Iqcf1, B9d2, Fam107a, Rarres1, Wbp2, Wdr93, C1qtnf5, Egfl7, Slc32a1, Id3, Ccdc58, Sox21, Tnfsf12, Qrich2, RGD1308930, B3galnt2, Tob2, Creb311, Klk12, Dqx1, Ncoa2, Slfnl1, Elf2, Gsx2, Hhip, Grm2, Fam170a, Gpc2, Npvf, Tsen54, Igfbp1, Zdhhc19, Csrnp2, Dcst2, Krt1, Nanos1, Cdh13, Ebi3, Prr3, Rnf43, Btbd17, Trim26, Gata3, Cdkn1c, Hrc, Grasp, Galm, Samd14, Oprd1, Pim1, Il2rb Ptpn13, Mtus1, Fhl3, Hdhd2, Farp1, Ptprs, Hand1, Rpl37a, Tcf15, Neurod2, Usp20, Foxo3, Aldh1l2, Nov, RGD1566130, Slc34a2, Gzma, Iqgap3, Enpp2, Tm7sf2, Exo1, Slc9a1, Rpl37,Pde12, Eefsec, Dmrta2, LOC498685, Brunol5, Tmem164, Flad1, Frmd4b, Marcksl1, Bhlhe22, Yif1b, Ttc38, Phyh, Mfsd6, Fam189b, Sned1,Cd3d, Slc25a29, Cd7, RGD1563281, Grb7, Ccdc147, Stat2, Ppp3ca, LOC688241, Shisa4, Atp8b1, Cd3g, Txnl4b, Ranbp10, Dynlt3, Atp2b2, Trub1, Zhx3, Rexo4, Snapc2, Rad541, Vps37c, Hmga1, Furin, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpdl1, Ankrd37, Cox4i2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, Vegfb, Lpl, Chaf1a, Srrd, Steap1, Fkbp11, Slc39a3, Bcan, Foxc2, Jph1, Ccl27, Pdia3, Gabrq, Surf6, Runx3, Abcb6, Cdk2, LOC687813, and Porcn, and
when either one or both of the following conditions are satisfied, it is determined that the diet or the substance provides production of breast milk having an immunosuppressive action:
(a) the mRNA is a mRNA of which amount in milk of a rat increases when a *Bifidobacterium* is orally administered to the rat, and when mRNA profiles in the milk observed before and after ingestion of the diet are compared, amount of at least one kind of mRNA in the milk observed after the ingestion is 0.67 times or less of that observed before the ingestion,
(b) the mRNA is a mRNA of which amount in milk of a rat decreases when a *Bifidobacterium* is orally administered to the rat, and when mRNA profiles in the milk observed before and after ingestion of the diet are compared, amount of at least one kind of mRNA in the milk observed after the ingestion is 1.5 times or more of that observed before the ingestion.

3. The method according to claim 1 or 2, wherein the mammal is a human, a rat, or a bovine.

4. The method according to any one of claims 1 to 3, wherein the mRNA of which amount in milk of a rat increases when a *Bifidobacterium* is orally administered to the rat is selected from the group consisting of Cyp24a1, Dnm1, Dpysl2, Fam89a, Arhgap22, Fbxl5,Btg2, Csnk1g1, Mll1, Eml4, Nelf, Aldh1l1, Boc, Usp43_predicted, Rassf5, Man2a1, Srgn, Gbf1, RGD1562551, Lrba, P2ry2, Bcl2l1, Rbm14, RGD1307365, Tecpr1, Kctd9, Pkig, RGD1308139, Klhl35, Gab1, Arid5b, Mafk, Trim47, Ddx21, Tiparp, Hspalb, Nr3c2, Klf4, Dhx8, Myh14, Mcam, Dusp3, Acer2, L3mbtl3, Fcho2, Ash1l, Paxip1, Irgq, Plcb1, Sts, Zmym3, Anxa1, Rtn4ip1, Gngt1, Srcap, Otud4, RGD1307569, Adpgk, Plk3, Phf1, St14, N4bp1, Naaa, Fgf7, Lama2, Ahnak, Sec3l1, Mks1, Prodh, Bazlb, R3hdm1, Setd5, Rhebl1, Kdm5b, Fbxw8, Epb4.1l4a, Lpin2, Cxcl2, Nr2c2, Ckap4, Ankrd1, Ugcg, Fcgr2b, Sf3a1, Parg, Heatr6, Ctdspl, Tp53bp1, Fam60a, Pik3r2, Arhgef5, Nek7, Rp2, Vhl, Usp31, Camsap1, RGD1308759, Ldlrap1, Sp1, Mast4, Rela, Nhlrc3, Intu, Gcap14, Cdc42se2, Chd7, Rbms1, Invs, Kif2a, Exoc6, Zbtb1, Rnd3, Rb1cc1, Sphk1, Noxo1, Mllt6, Thumpd2, Ptpn4, Thex1, S100a8, Grk5, Trove2, Pcdh17, Med13, Fam82a2, Rab43, Clip1, Ankrd57, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargclb, Med1, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5,Map6, Angptl4, Dnmt3b, Gzf1, Plcb4, Ap4m1, Stat3, Rkhd1, Dcp2, Rtn4, Tlr3, Tubb6, Casp8, Nin, Fyn, Tmem77, Maml3, Slc15a4, Sqrdl, Ppplr9b, Pafah1b1, Def8, Fam164a, Znf746, RGD1305235, Thada, Hip1, Pgrmc2, Sgk3, Baz1a, Foxn3, Map3k12, Efhd2, Pogz, Pcf11, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrpll, Sptbn1, Dtx3l, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, Paqr5, Ddhd1, Kdm6b, Ube2d1, Rhoc, LOC361100, Cx3c11, Fbxo42, Daam1, Klhl5, Fbxo30, Syt12, Herc1, Dab2ip, Tacc2, Sipa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Re112, Zfyve16, Cebpd, Tnrc6b, Nsun6, Dennd5a, Fbxw5, Slc25a25, Dtwd1, Hspb1, Homer2, Nhej1, Etnk1, No19, Med23, Nup133, Rnd1, Rplp1, Ankfy1, Pik3r4, Plcd1, Cxcl12, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, Il17re, Eif4e3, Dock8, Nolc1, Sult1a1, Ankrd17, Trim2, Nfib, Cd14, S100a10, LOC687105, Ccdc123, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, Cdh1, Rnf19a, Pdlim1, Csnklg3, Hectd1, Taf5l, Noc3l, Nfat5, RGD1562529, Atl3, Ptpn9, Nedd4l, Wipi1, Gnal, Tle1, Pprc1, Src, Clic1, Secisbp21, Rnf103, Ogt, Zcchc2, Clasp1, Gtpbp2, Ift172, Map3k6, Plce1, Nedd4, Cpne3, Prim2, Trafd1, Myef2, Znf532, Tmem50b, Il6ra, Pdcd6ip, Tpm4, Rnasen, Lpin1, Flot1, Cds1, Tnfaip1, Mudeng, Cdc2l5, Arid2, Znf654, Nat8b, Snx12, Ap1g1, Itsn2, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klhl24, Cox11, Tfip11, Rcan1, Nagk, Myst3, Rbm28, Anapc7, Ezh1, Cyp27a1, Irs2, Sdccag1, Foxp1, Dusp18, Hcca2, Fbxl8, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, Twf1, Rp18, Prdx1, Phrf1, Pcm1, and Mapk12.

5. The method according to any one of claims 1 to 3, wherein the mRNA of which amount in milk of a rat decreases when a *Bifidobacterium* is orally administered to the rat is selected from the group consisting of Slcla4, Trim16, Cacnale, Gal, Arnt2, Stk11ip, Sdc3, Tp53i11, Mpz, Dnmt31, Ache, Cln5, Asb7, Rtp3, Peli2, Caskin2, Cass4, Plvap, Histlhla, Col22a1, Htr6, Trim42, Ccdc89, Atp10d, Oprk1, Cyct, Pdzd2, Sh2d5, Tmem136, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbp1, Bnc1, Col18a1, Traf3, Sema7a, Rcn3, Ift122, Smc2, Cldn18, LOC679462, Pogk, Abhd15, Pias4, Alg8, Nkx2-4, Mmp2, Iqcf1, B9d2, Fam107a, Rarres1, Wbp2, Wdr93, C1qtnf5, Egfl7, Slc32a1, Id3, Ccdc58, Sox21, Tnfsf12, Qrich2, RGD1308930, B3galnt2, Tob2, Creb311, Klk12, Dqx1, Ncoa2, Slfnl1, Elf2, Gsx2, Hhip, Grm2, Fam170a, Gpc2, Npvf, Tsen54, Igfbp1, Zdhhc19, Csrnp2, Dcst2, Krt1, Nanos1, Cdh13, Ebi3, Prr3, Rnf43, Btbd17, Trim26, Gata3, Cdkn1c, Hrc, Grasp, Galm, Samd14, Oprd1, Pim1, Il2rb Ptpn13, Mtus1, Fhl3, Hdhd2, Farp1, Ptprs, Hand1, Rpl37a, Tcf15, Neurod2, Usp20, Foxo3, Aldh1l2, Nov, RGD1566130, Slc34a2, Gzma, Iqgap3, Enpp2, Tm7sf2, Exo1, Slc9a1, Rpl37,Pde12, Eefsec, Dmrta2, LOC498685, Brunol5, Tmem164, Flad1, Frmd4b, Marcksl1, Bhlhe22, Yif1b, Ttc38, Phyh, Mfsd6, Fam189b, Sned1,Cd3d, Slc25a29, Cd7, RGD1563281, Grb7, Ccdc147, Stat2, Ppp3ca, LOC688241, Shisa4, Atp8b1, Cd3g, Txnl4b, Ranbp10, Dynlt3, Atp2b2, Trub1, Zhx3, Rexo4, Snapc2, Rad541, Vps37c, Hmga1, Furin, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpdl1, Ankrd37, Cox4i2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, Vegfb, Lpl, Chaf1a, Srrd, Steap1, Fkbp11, Slc39a3, Bcan, Foxc2, Jph1, Cc127, Pdia3, Gabrq, Surf6, Runx3, Abcb6, Cdk2, LOC687813, and Porcn.

6. A method for producing a composition for oral ingestion having an immunostimulating action, comprising the step of adding a mRNA of which amount in milk of a rat increases when a *Bifidobacterium* is orally administered to the rat to a base for a composition for oral ingestion, wherein the mRNA is selected from the group consisting of Cyp24a1, Dnm1, Dpysl2, Fam89a, Arhgap22, Fbxl5,Btg2, Csnk1g1, Mll1, Eml4, Nelf, Aldh1l1, Boc, Usp43_predicted, Rassf5, Man2a1, Srgn, Gbf1, RGD1562551, Lrba, P2ry2, Bcl2l1, Rbm14, RGD1307365, Tecpr1, Kctd9, Pkig, RGD1308139, Klhl35, Gab1, Arid5b, Mafk, Trim47, Ddx21, Tiparp, Hspalb, Nr3c2, Klf4, Dhx8, Myh14, Mcam, Dusp3, Acer2, L3mbtl3, Fcho2, Ash1l, Paxip1, Irgq, Plcb1, Sts, Zmym3, Anxa1, Rtn4ip1, Gngt1, Srcap, Otud4, RGD1307569, Adpgk, Plk3, Phf1, St14, N4bp1, Naaa, Fgf7, Lama2, Ahnak, Sec3l1, Mks1, Prodh, Bazlb, R3hdm1, Setd5, Rhebl1, Kdm5b, Fbxw8, Epb4.1l4a, Lpin2, Cxcl2, Nr2c2, Ckap4, Ankrd1, Ugcg, Fcgr2b, Sf3a1, Parg, Heatr6, Ctdspl, Tp53bp1, Fam60a, Pik3r2, Arhgef5, Nek7, Rp2, Vhl, Usp31, Camsap1, RGD1308759, Ldlrap1, Sp1, Mast4, Rela, Nhlrc3, Intu, Gcap14, Cdc42se2, Chd7, Rbms1, Invs, Kif2a, Exoc6, Zbtb1, Rnd3, Rb1cc1, Sphk1, Noxo1, Mllt6, Thumpd2, Ptpn4, Thex1, S100a8, Grk5, Trove2, Pcdh17, Med13, Fam82a2, Rab43, Clip1, Ankrd57, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargclb, Med1, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5,Map6, Angptl4, Dnmt3b, Gzf1, Plcb4, Ap4m1, Stat3, Rkhd1, Dcp2, Rtn4, Tlr3, Tubb6, Casp8, Nin, Fyn, Tmem77, Maml3, Slc15a4, Sqrdl, Ppplr9b, Pafah1b1, Def8, Fam164a, Znf746, RGD1305235, Thada, Hip1, Pgrmc2, Sgk3, Baz1a, Foxn3, Map3k12, Efhd2, Pogz, Pcf11, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrpll, Sptbn1, Dtx3l, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, Paqr5, Ddhd1, Kdm6b, Ube2d1, Rhoc, LOC361100, Cx3cl1, Fbxo42, Daam1, Klhl5, Fbxo30, Syt12, Herc1, Dab2ip, Tacc2, Sipa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Rell2, Zfyve16, Cebpd, Tnrc6b, Nsun6, Dennd5a, Fbxw5, Slc25a25, Dtwd1, Hspb1, Homer2, Nhej1, Etnk1, Nol9, Med23, Nup133, Rnd1, Rplp1, Ankfy1, Pik3r4, Plcd1, Cxcl12, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, Il17re, Eif4e3, Dock8, Nolc1, Sult1a1, Ankrd17, Trim2, Nfib, Cd14, S100a10, LOC687105, Ccdc123, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, Cdh1, Rnf19a, Pdlim1, Csnklg3, Hectd1, Taf51, Noc3l, Nfat5, RGD1562529, Atl3, Ptpn9, Nedd4l, Wipi1, Gnal, Tle1, Pprc1, Src, Clic1, Secisbp2l, Rnf103, Ogt, Zcchc2, Clasp1, Gtpbp2, Ift172, Map3k6, Plce1, Nedd4, Cpne3, Prim2, Trafd1, Myef2, Znf532, Tmem50b, Il6ra, Pdcd6ip, Tpm4, Rnasen, Lpin1, Flot1, Cds1, Tnfaip1, Mudeng, Cdc2l5, Arid2, Znf654, Nat8b, Snx12, Ap1g1, Itsn2, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klhl24, Cox11, Tfip11, Rcan1, Nagk, Myst3, Rbm28, Anapc7, Ezh1, Cyp27a1, Irs2, Sdccag1, Foxp1, Dusp18, Hcca2, Fbxl8, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, Twf1, Rpl8, Prdx1, Phrf1, Pcm1, and Mapk12.

7. A method for producing a composition for oral ingestion having an immunosuppressive action, comprising the step of adding a mRNA of which amount in milk of a rat decreases when a *Bifidobacterium* is orally administered to the rat to a base for a composition for oral ingestion, wherein the mRNA is selected from the group consisting of Slc1a4, Trim16, Cacnale, Gal, Arnt2, Stk11ip, Sdc3, Tp53i11, Mpz, Dnmt3l, Ache, Cln5, Asb7, Rtp3, Peli2, Caskin2, Cass4, Plvap, Histlhla, Col22a1, Htr6, Trim42, Ccdc89, Atp10d, Oprk1, Cyct, Pdzd2, Sh2d5, Tmem136, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbp1, Bnc1, Col18a1, Traf3, Sema7a, Rcn3, Ift122, Smc2, Cldn18, LOC679462, Pogk, Abhd15, Pias4, Alg8, Nkx2-4, Mmp2, Iqcf1, B9d2, Fam107a, Rarres1, Wbp2, Wdr93, C1qtnf5, Egfl7, Slc32a1, Id3, Ccdc58, Sox21, Tnfsf12, Qrich2, RGD1308930, B3galnt2, Tob2, Creb3l1, Klk12, Dqx1, Ncoa2, Slfnl1, Elf2, Gsx2, Hhip, Grm2, Fam170a, Gpc2, Npvf, Tsen54, Igfbp1, Zdhhc19, Csrnp2, Dcst2, Krt1, Nanos1, Cdh13, Ebi3, Prr3, Rnf43, Btbd17, Trim26, Gata3, Cdkn1c, Hrc, Grasp, Galm, Samd14, Oprd1, Pim1, Il2rb, Ptpn13, Mtus1, Fhl3, Hdhd2, Farp1, Ptprs, Hand1, Rpl37a, Tcf15, Neurod2, Usp20, Foxo3, Aldh1l2, Nov, RGD1566130, Slc34a2, Gzma, Iqgap3, Enpp2, Tm7sf2, Exo1, Slc9a1, Rpl37,Pde12, Eefsec, Dmrta2, LOC498685, Brunol5, Tmem164, Flad1, Frmd4b, Marcksl1, Bhlhe22, Yif1b, Ttc38, Phyh, Mfsd6, Fam189b, Sned1,Cd3d, Slc25a29, Cd7, RGD1563281, Grb7, Ccdc147, Stat2, Ppp3ca, LOC688241, Shisa4, Atp8bl, Cd3g, Txnl4b, Ranbp10, Dynlt3, Atp2b2, Trub1, Zhx3, Rexo4, Snapc2, Rad54l, Vps37c, Hmga1, Furin, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpd1l, Ankrd37, Cox4i2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, Vegfb, Lpl, Chaf1a, Srrd, Steap1, Fkbp11, Slc39a3, Bcan, Foxc2, Jph1, Ccl27, Pdia3, Gabrq, Surf6, Runx3, Abcb6, Cdk2, LOC687813, and Porcn.

8. The method according to claim 6 or 7, wherein the composition for oral ingestion is a food for infants or a food for little children.

9. The method according to claim 8, wherein the food for infants or the food for little children is infant formula or follow-on formula.

## Patentansprüche

1. Verfahren zum Screenen auf ein Nahrungsmittel oder eine Substanz, das/die die Herstellung von Muttermilch mit immunstimulierender Wirkung ermöglicht, umfassend das Identifizieren eines Nahrungsmittels oder einer Substanz, die die Menge an mRNA, die in der Milch eines Säugers vorhanden ist, erhöht oder verringert, durch Verwendung der Korrelation des mRNA-Profils in der Milch und einem Nahrungsmittel, das von dem Säuger eingenommen wird, oder einer Substanz, die in dem Nahrungsmittel als Index enthalten ist,
wobei das mRNA-Profile eine Menge an mRNA umfasst, die ausgewählt ist aus der Gruppe bestehend aus Cyp24a1, Dnm1, Dpys12, Fam89a, Arhgap22, Fbx15,Btg2,
Csnk1g1, Mll1, Eml4, Nelf, Aldh111, Boc, Usp43_predicted, Rassf5, Man2a1, Srgn, Gbf1, RGD1562551, Lrba, P2ry2, Bcl2l1, Rbm14, RGD1307365, Tecpr1, Kctd9, Pkig, RGD1308139, Klhl35, Gab1, Arid5b, Mafk, Trim47, Ddx2l, Tiparp, Hspalb, Nr3c2, Klf4, Dhx8, Myhl4, Mcam, Dusp3, Acer2, L3mbtl3, Fcho2, Ash1l, Paxip1, Irgq, Plcb1, Sts, Zmym3, Anxal, Rtn4ip1, Gngt1, Srcap, Otud4, RGD1307569, Adpgk, Plk3, Phf1, St14, N4bp1, Naaa, Fgf7, Lama2, Ahnak, Sec3l1, Mks1, Prodh, Baz1b, R3hdm1, Setd5, Rhebl1, Kdm5b, Fbxw8, Epb4.1l4a, Lpin2, Cxcl2, Nr2c2, Ckap4, Ankrd1, Ugcg, Fcgr2b, Sf3a1, Parg, Heatr6, Ctdsp1, Tp53bp1, Fam60a, Pik3r2, Arhgef5, Nek7, Rp2, Vhl, Usp31, Camsap1, RGD1308759, Ldlrap1, Sp1, Mast4, Rela, Nhlrc3, Intu, Gcap14, Cdc42se2, Chd7, Rbms1, Invs, Kif2a, Exoc6, Zbtb1, Rnd3, Pb1cc1, Sphk1, Noxo1, Mllt6, Thumpd2, Ptpn4, Thex1, S100a8, Grk5, Trove2, Pcdh17, Med13, Fam82a2, Rab43, Clip1, Ankrd57, Crim1, Celsr2, Idh1, Lrrc61, Aff4, Arhgap1, Itpr3, Lhfp, Dnajc12, Garnl1, Gtf3c5, Hspb8, Chacl, Pof1b, Ptpre, Arl6ip1, Son, Ppargc1b, Med1, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5,Map6, Angptl4, Dnmt3b, Gzf1, Plcb4, Ap4m1, Stat3, Rkhd1, Dcp2, Rtn4, Tlr3, Tubb6, Casp8, Nin, Fyn, Tmem77, Maml3, Slc15a4, Sqrdl, Ppplr9b, Pafah1b1, Def8, Fam164a, Znf746, RGD1305235, Thada, Hip1, Pgrmc2, Sgk3, Baz1a, Foxn3, Map3k12, Efhd2, Pogz, Pcf11, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrpll, Sptbn1, Dtx3l, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, Paqr5, Ddhd1, Kdm6b, Ube2d1, Rhoc, LOC361100, Cx3cl1, Fbxo42, Daam1, Klhl5, Fbxo30, Syt12, Herc1, Dab2ip, Tacc2, Sipa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Rell2, Zfyve16, Cebpd, Tnrc6b, Nsun6, Dennd5a, Fbxw5, Slc25a25, Dtwd1, Hspb1, Homer2, Nhej1, Etnk1, Nol9, Med23, Nup133, Rnd1, Rp1p1, Ankfy1, Pik3r4, Plcd1, Cxc112, Lix11, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, Il17re, Eif4e3, Dock8, Nolc1, Sult1a1, Ankrd17, Trim2, Nfib, Cd14, S100a10, LOC6B7105, Ccdc123, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, Cdh1, Rnf19a, Pdlim1, Csnk1g3, Hectd1, Taf5l, Noc3l, Nfat5, RGD1562529, Atl3, Ptpn9, Nedd4l, Wipi1, Gnal, Tle1, Pprc1, Src, Clic1, Secisbp2l, Rnf103, Ogt, Zcchc2, Clasp1, Gtpbp2, Ift172, Map3k6, Plce1, Nedd4, Cpne3, Prim2, Trafd1, Myef2, Znf532, Tmem50b, I16ra, Pdcd6ip, Tpm4, Rnasen, Lpin1, Flot1, Cds1, Tnfaip1, Mudeng, Cdc2l5, Arid2, Znf654, Nat8b, Snx12, Ap1g1, Itsn2, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klh124, Cox11, Tfip11, Rcan1, Nagk, Myst3, Rbm28, Anapc7, Ezh1, Cyp27a1, Irs2, Sdccag1, Foxp1, Dusp18, Hcca2, Fbx18, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, Twf1, Rp18, Prdx1, Phrf1, Pcm1, Mapk12, Slc1a4, Trim16, Cacnale, Gal, Arnt2, Stk11ip, Sdc3, Tp53i11, Mpz, Dnmt31, Ache, C1n5, Asb7, Rtp3, Peli2, Caskin2, Cass4, Plvap, Hist1h1a, Col22a1, Htr6, Trim42, Ccdc89, Atp10d, Oprk1, Cyct, Pdzd2, Sh2d5, Tmem136, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbp1, Bnc1, Col18a1, Traf3, Sema7a, Rcn3, Ift122, Smc2, Cldn18, LOC679462, Pogk, Abhd15, Pias4, Alg8, Nkx2-4, Mmp2, Iqcf1, B9d2, Fam107a, Rarres1, Wbp2, Wdr93, C1qtnf5, Egfl7, Slc32a1, Id3, Ccdc58, Sox21, Tnfsf12, Qrich2, RGD1308930, B3galnt2, Tob2, Creb311, K1k12, Dqx1, Ncoa2, Slfnl1, Elf2, Gsx2, Hhip, Grm2, Fam170a, Gpc2, Npvf, Tsen54, Igfbp1, Zdhhc19, Csrnp2, Dcst2, Krt1, Nanos1, Cdh13, Ebi3, Prr3, Rnf43, Btbd17, Trim26, Gata3, Cdkn1c, Hrc, Grasp, Galm, Samd14, Oprd1, Pim1, Il2rb, Ptpn13, Mtus1, Fhl3, Hdhd2, Farpl, Ptprs, Hand1, Rpl37a, Tcf15, Neurod2, Usp20, Foxo3, Aldh112, Nov, RGD1566130, Slc34a2, Gzma, Iqgap3, Enpp2, Tm7sf2, Exo1, Slc9a1, Rpl37,Pde12, Eefsec, Dmrta2, LOC498685, Bruno15, Tmem164, Flad1, Frmd4b, Marcksl1, Bhlhe22, Yiflb, Ttc38, Phyh, Mfsd6, Fam189b, Sned1,Cd3d, Slc25a29, Cd7, RGD1563281, Grb7, Ccdc147, Stat2, Ppp3ca, LOC688241, Shisa4, Atp8b1, Cd3g, Txn14b, Ranbp10, Dynlt3, Atp2b2, Trub1, Zhx3, Rexo4, Snapc2, Rad54l, Vps37c, Hmga1, Furin, Rab15, Folr1, Fundc1, Park3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpd1l, Ankrd37, Cox4i2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, Vegfb, Lp1, Chaf1a, Srrd, Steap1, Fkbp11, Slc39a3, Bcan, Foxc2, Jph1, Ccl27, Pdia3, Gabrq, Surf6, Runx3, Abcb6, Cdk2, LOC687813,
und Porcn, und
wenn entweder eine oder beide der folgenden Bedingungen erfüllt ist/sind, bestimmt wird, dass das Nahrungsmittel oder die Substanz die Herstellung von Muttermilch mit immunstimulierender Wirkung ermöglicht:
(A) die mRNA eine mRNA ist, deren Menge in der Milch einer Ratte zunimmt, wenn der Ratte ein *Bifidobakterium* oral verabreicht wird, und wenn die mRNA-Profile in der Milch, die vor und nach Einnahme des Nahrungsmittels beobachtet werden, verglichen werden, die Menge von mindestens einer Art von mRNA in der Milch, die nach der Einnahme beobachtet wird, 1,5 mal so viel oder mehr ist als die Menge, die vor der Einnahme beobachtet wird,
(B) die mRNA eine mRNA ist, deren Menge in der Milch einer Ratte abnimmt, wenn der Ratte ein *Bifidobakterium* oral verabreicht wird, und wenn die mRNA-Profile in der Milch, die vor und nach Einnahme des Nahrungsmittels beobachtet werden, verglichen werden, die Menge von mindestens einer Art von mRNA in der Milch, die nach der Einnahme beobachtet wird, 0,67 mal so viel oder weniger ist als die Menge, die vor der Einnahme beobachtet wird.

2. Verfahren zum Screenen auf ein Nahrungsmittel oder eine Substanz, das/die die Herstellung von Muttermilch mit immunsuppressiver Wirkung ermöglicht, umfassend das Identifizieren eines Nahrungsmittels oder einer Substanz, die die Menge an mRNA, die in der Milch eines Säugers vorhanden ist, erhöht oder verringert, durch Verwendung der Korrelation des mRNA-Profils in der Milch und einem Nahrungsmittel, das von dem Säuger aufgenommen wird, oder einer Substanz, die in dem Nahrungsmittel als Index enthalten ist,
wobei das mRNA-Profil eine mRNA-Menge umfasst, die ausgewählt ist aus der Gruppe bestehend aus Cyp24a1, Dnm1, Dpys12, Fam89a, Arhgap22, Fbx15, Btg2,
Csnk1g1, Mll1, Eml4, Nelf, Aldh111, Bloc, Usp43_predicted, Rassf5, Man2a1, Srgn, Gbf1, RGD1562551, Lrba, P2ry2, Bcl211, Rbm14, RGD1307365, Tecpr1, Kctd9, Pkig, RGD1308139, Klhl35, Gab1, Arid5b, Mafk, Trim47, Ddx21, Tiparp, Hspa1b, Nr3c2, Klf4, Dhx8, Myh14, Mcam, Dusp3, Acer2, L3mbtl3, Fcho2, Ash1l, Paxip1, Irgq, Plcb1, Sts, Zmym3, Anxa1, Rtn4ip1, Gngt1, Srcap, Otud4, RGD1307569, Adpgk, Plk3, Phf1, St14, N4bp1, Naaa, Fgf7, Lama2, Ahnak, Sec3l1, Mks1, Prodh, Baz1b, R3hdm1, Setd5, Rhebl1, Kdm5b, Fbxw8, Epb4.114a, Lpin2, Cxcl2, Nr2c2, Ckap4, Ankrd1, Ugcg, Fcgr2b, Sf3a1, Parg, Heatr6, Ctdspl, Tp53bp1, Fam60a, Pik3r2, Arhgef5, Nek7, Rp2, Vhl, Usp31, Camsap1, RGD1308759, Ldlrap1, Sp1, Mast4, Rela, Nhlrc3, Intu, Gcap14, Cdc42se2, Chd7, Rbms1, Invs, Kif2a, Exoc6, Zbtb1, Rnd3, Rb1cc1, Sphk1, Noxo1, Mllt6, Thumpd2, Ptpn4, Thex1, S100a8, Grk5, Trove2, Pcdh17, Med13, Fam82a2, Rab43, Clip1, Ankrd57, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargc1b, Med1, Zbtb39, Urb1, Wdr89, Josd1, Pank4, Ska2, Rrm1, K1f11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5,Map6, Angptl4, Dnmt3b, Gzf1, Plcb4, Ap4m1, Stat3, Rkhd1, Dcp2, Rtn4, Tlr3, Tubb6, Casp8, Nin, Fyn, Tmem77, Maml3, Slc15a4, Sqrdl, Ppp1r9b, Pafah1b1, Def8, Fam164a, Znf746, RGD1305235, Thada, Hip1, Pgrmc2, Sgk3, Baz1a, Foxn3, Map3k12, Efhd2, Pogz, Pcf11, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrp11, Sptbn1, Dtx31, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, Paqr5, Ddhd1, Kdm6b, Ube2d1, Rhoc, LOC361100, Cx3cl1, Fbxo42, Daam1, Klhl5, Fbxo30, Syt12, Herc1, Dab2ip, Tacc2, Sipall1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Rell2, Zfyve16, Cebpd, Tnrc6b, Nsun6, Dennd5a, Fbxw5, Slc25a25, Dtwd1, Hspb1, Homer2, Nhej1, Etnk1, Nol9, Med23, Nup133, Rnd1, Pplp1, Ankfy1, Pik3r4, Plcd1, Cxc112, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, I117re, Eif4e3, Dock8, Nolc1, Sult1a1, Ankrd17, Trim2, Nfib, Cd14, S100a10, LOC687105, Ccdc123, Pols, Aplf, Gu1p1, S1c40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, Cdh1, Rnf19a, Pdlim1, Csnk1g3, Hectd1, Taf51, Noc31, Nfat5, RGD1562529, At13, Ptpn9, Nedd41, Wipi1, Gna1, Tle1, Pprc1, Src, Clic1, Secisbp21, Rnf103, Ogt, Zcchc2, Clasp1, Gtpbp2, Ift172, Map3k6, Plce1, Nedd4, Cpne3, Prim2, Trafd1, Myef2, Znf532, Tmem50b, Il6ra, Pdcd6ip, Tpm4, Rnasen, Lpin1, Flot1, Cds1, Tnfaip1, Mudeng, Cdc215, Arid2, Znf654, Nat8b, Snx12, Ap1g1, Itsn2, RGD1306820, Mesdc1, Tmco6, Fkbp5, Prpf8, Pdcd11, Runx1, Epn2, Arid4b, Slk, Prkx, Dnajb3, Phf17, Fam193a, Pvr12, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klh124, Cox11, Tfip11, Rcan1, Nagk, Myst3, Rbm28, Anapc7, Ezh1, Cyp27a1, Irs2, Sdccag1, Foxp1, Dusp18, Hcca2, Fbxl8, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, Twf1, Rp18, Prdx1, Phrf1, Pcm1, Mark12, Slc1a4, Trim16, Cacnale, Gal, Arnt2, Stk11ip, Sdc3, Tp53i11, Mpz, Dnmt31, Ache, Cln5, Asb7, Rtp3, Peli2, Caskin2, Cass4, Plvap, Hist1h1a, Col22a1, Htr6, Trim42, Ccdc89, Atp10d, Oprk1, Cyct, Pdzd2, Sh2d5, Tmeml36, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbp1, Bnc1, Col18a1, Traf3, Sema7a, Rcn3, Ift122, Smc2, Cldn18, LOC679462, Pogk, Abhd15, Pias4, Alg8, Nkx2-4, Mmp2, Iqcf1, B9d2, Fam107a, Parres1, Wbp2, Wdr93, Clqtnf5, Egf17, Slc32a1, Id3, Ccdc58, Sox21, Tnfsf12, Qrich2, RGD1308930, B3galnt2, Tob2, Creb311, Klk12, Dqx1, Ncoa2, S1fn11, Elf2, Gsx2, Hhip, Grm2, Fam170a, Gpc2, Npvf, Tsen54, Igfbp1, Zdhhc19, Csrnp2, Dcst2, Krt1, Nanos1, Cdh13, Ebi3, Prr3, Rnf43, Btbd17, Trim26, Data3, Cdkn1c, Hrc, Grasp, Ga1m, Samd14, Oprd1, Pim1, Il2rb, Ptpn13, Mtus1, Fh13, Hdhd2, Farp1, Ptprs, Hand1, Rp137a, Tcf15, Neurod2, Usp20, Foxo3, Aldh1l2, Nov, RGD1566130, Slc34a2, Gzma, Iqgap3, Enpp2, Tm7sf2, Exo1, Slc9a1, Rp137, Pde12, Eefsec, Dmrta2, LOC498685, Bruno15, Tmem164, Flad1, Frmd4b, Marcks11, Bhlhe22, Yif1b, Ttc38, Phyh, Mfsd6, Fam189b, Sned1, Cd3d, S1c25a29, Cd7, RGD1563281, Grb7, Ccdc147, Stat2, Ppp3ca, LOC688241, Shisa4, Atp8b1, Cd3g, Txn14b, Ranbp10, Dynlt3, Atp2b2, Trubl, Zhx3, Rexo4, Snapc2, Rand541, Vps37c, Hmga1, Furin, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpd11, Ankrd37, Cox4i2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fades2, Fbxo8, Vegfb, Lpl, Chaf1a, Srrd, Steap1, Fkbp11, Slc39a3, Bcan, Foxc2, Jph1, Ccl27, Pdia3, Gabrq, Surf6, Runx3, Abcb6, Cdk2, LOC687813,
und Porcn, und
wenn entweder eine oder beide der folgenden Bedingungen erfüllt ist/sind, bestimmt wird, dass das Nahrungsmittel oder die Substanz die Herstellung von Muttermilch mit immunstimulierender Wirkung ermöglicht:
(a) die mRNA eine mRNA ist, deren Menge in der Milch einer Ratte zunimmt, wenn der Ratte ein *Bifidobakterium* oral verabreicht wird, und wenn die mRNA-Profile in der Milch, die vor und nach Einnahme des Nahrungsmittels beobachtet werden, verglichen werden, die Menge von mindestens einer Art von mRNA in der Milch, die nach der Einnahme beobachtet wird, 0,67 mal so viel oder weniger ist, als die Menge, die vor der Einnahme beobachtet wird,
(b) die mRNA eine mRNA ist, deren Menge in der Milch einer Ratte abnimmt, wenn der Ratte ein *Bifidobakterium* oral verabreicht wird, und wenn die mRNA-Profile in der Milch, die vor und nach Einnahme des Nahrungsmittels beobachtet werden, verglichen werden, die Menge von mindestens einer Art von mRNA in der Milch, die nach der Einnahme beobachtet wird, 1,5 mal so viel oder mehr ist, als die Menge, die vor der Einnahme beobachtet wird.

3. Verfahren nach Anspruch 1 oder 2, wobei der Säuger ein Mensch, eine Ratte oder ein Rind ist.

4. Verfahren nach einem der Ansprüche 1 bis 3, wobei die mRNA, deren Menge in der Milch einer Ratte zunimmt, wenn der Ratte ein *Bifidobakterium* oral verabreicht wird, ausgewählt ist aus der Gruppe bestehend aus Cyp24a1,
Dnm1, Dpysl2, Fam89a, Arhgap22, Fbx15, Btg2, Csnk1g1, Ml11, Em14, Nelf, Aldh1l1, Bloc, Usp43_predicted, Rassf5, Man2a1, Srgn, Gbf1, RGD1562551, Lrba, P2ry2, Bsl211, Rbm14, RGD1307365, Tecpr1, Kctd9, Pkig, RGD1308139, Klh135, Gab1, Arid5b, Mafk, Trim47, Ddx21, Tiparp, Hspa1b, hFr3ca2, Klf4, Dhx8, Myh14, Mcam, Dusp3, Acer2, L3mbtl3, Echo2, Ash11, Paxip1, Irgq, Plcb1, Sts, Zmym3, Gna1, Rtn4ipl, Gngt1, Srcap, Otud4, RGD1307569, Adpgk, Plk3, Phf1, St14, N4bp1, Naaa, Fgf7, Lama2, Ahnak, Sec311, Mks1, Prodh, Baz1b, R3hdm1, Setd5, Rhebl1, Kdm5b, Fbxw8, Epb4.114a, Lpin2, Cxcl2, Nr2c2, Ckap4, Ankrd1, Ugcg, Fcgr2b, Sf3a1, Parg, Heatr6, Ctdspl, Tp53bp1, Fam60a, Pik3r2, Arhgef5, Nek7, Rp2, Vh1, Usp31, Camsap1, RGD1308759, Ld1rap1, Sp1, Mast4, Re1a, Nh1rc3, Intu, Gcap14, Cdc42se2, Chd7, Rbms1, Invs, Kif2a, Exoc6, Zbtb1, Rind3, Rb1cc1, Sphk1, Noxo1, M11t6, Thumpd2, Ptpn4, Thex1, S100a8, Grk5, Trove2, Pcdh17, Med13, Fam82a2, Rab43, Clip1, Ankrd57, Crim1, Celsr2, rdh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargc1b, Med1, Zbtb39, Urb1, Wdr89, Josd1, Pank4, Ska2, Rrm1, Flf11, Phc1, Birc6, Cask, Mesdc2, St5, Smyd5, Heca, Plag12, Stern4, Ftsjd1, Mam11, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5, Map6, Angptl4, Dnmt3b, Gzf1, Plcb4, Ap4m1, Stat3, Rkhd1, Dcp2, Rtn4, Tlr3, Tubb6, Casp8, Nin, Fyn, Tmem77, Mam13, Slc15a4, Sqrd1, Ppp1r9b, Pafah1b1, Def8, Fam164a, Znf746, RGD1305235, Thada, Hip1, Pgrmc2, Sgk3, Baz1a, Eoxn3, Map3k12, Efhd2, Pogz, Pcf11, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Korbs1, Tp63, Wdr48, Hnrpl1, Sptbn1, Dtx31, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Mup160, Eea1, LOC363188, Paqr5, Ddhd1, Kdm6b, Ube2dl, Rhoc, LOC361100, Cx3c11, Fbxo42, Daam1, Klhl5, Fbxo30, Syt12, Herc1, Dab2ip, Tacc2, Sipa111, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Rell2, Zfyve16, Cebpd, Tnrc6b, Nsun6, Dennd5a, Fbxw5, Slc25a25, Dtwd1, Hspb1, Homer2, Nhej1, Etnk1, Nol9, Med23, Nup133, Rnd1, Rplp1, Ankfy1, Pik3r4, P1cd1, Cxcl12, Lix11, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, Il17re, Eif4e3, Dock8, Nolc1, Sult1a1, Ankrd17, Trim2, Nfib, Cdl4, S100a10, LOC687105, Ccdc123, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, Cdh1, Rnf19a, Pdlim1, Csnk1g3, Hectd1, Taf51, Noc31, Nfat5, RGD1562529, At13, Ptpn9, Nedd41, Wipi1, Gna1, Tle1, Pprc1, Src, Clic1, Secisbp21, Rnf103, Ogt, Zcchc2, Clasp1, Gtpbp2, Ift172, Map3k6, Plce1, Nedd4, Cpne3, Prim2, Trafd1, Myef2, Znf532, Tmem50b, I16ra, Pdcd6ip, Tpm4, Rnasen, Lpin1, Flot1, Cds1, Tnfaip1, Mudeng, Cdc215, Arid2, Znf654, Nat8b, Snx12, Ap1g1, Itsn2, RGD1306820, Mesdc1, Tmco6, Fkbp5, Prpf8, Pdcd11, Runx1, Epn2, Arid4b, S1k, Prkx, Dnajb3, Phf17, Fam193a, Perl2, Tep1, Atxn7, Mwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klhl24, Coxl1, Tfipl1, Rcan1, Nagk, Myst3, Rbm28, Anapc7, Ezh1, Cyp27a1, Irs2, Sdccag1, Foxp1, Dusp18, Hcca2, Fbx18, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, **Twf1, Rp18, Prdx1, Phrf1, Pcm1 und Mapk12.**

5. **Verfahren nach einem der Ansprüche 1 bis 3, wobei die mRNA, deren Menge in der Milch einer Ratte abnimmt, wenn der Ratte ein *Bifidobakterium* oral verabreicht wird, ausgewählt ist aus der Gruppe bestehend aus Slc1a4,**
Trim16, Cacnale, Ga1, Arnt2, Stk11ip, Sdc3, Tp53i11, Mpz, Dnmt31, Ache, Cln5, Asb7, Rtp3, PeLi2, Caskin2, Cass4, Plvap, Hist1hla, Col22a1, Htr6, Trim42, Ccdc89, Atp10d, Oprk1, Cyct, Pdzd2, Sh2d5, Tmem136, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbp1, Bnc1, Col18a1, Traf3, Sema7a, Rcn3, Ift122, Smc2, Cldnl8, LOC679462, Pogk, Abhd15, Pias4, Alg8, Nkx2-4, Mmp2, Iqcf1, B9d2, Fam107a, Rarres1, Wbp2, Wdr93, Clqtnf5, Egf17, Slc32a1, Id3, Ccdc58, Sox21, Tnfsf12, Qrich2, RGD1308930, B3galnt2, Tob2, Creb311, K1k12, Dqx1, Ncoa2, Slfn11, Elf2, Gsx2, Hhip, Grm2, Fam170a, Gpc2, Npvf, Tsen54, Igfbp1, Zdhhc19, Csrnp2, Dcst2, Krt1, Nanos1, Cdh13, Ebi3, Prr3, Rnf43, Btbd17, Trim26, Gata3, Cdkn1c, Hrc, Grasp, Galm, Samd14, Oprd1, Pim1, I12rb, Ptpn13, Mtus1, Fhl3, Hdhd2, Farp1, Ptprs, Hand1, Rpl37a, Tcf15, Neurod2, Usp20, Foxo3, Aldh112, Nov, RGD1566130, Slc34a2, Gzma, Iqgap3, Enpp2, Tm7sf2, Exo1, Slc9a1, Rpl37, Pde12, Eefsec, Dmrta2, LOC498685, Brunol5, Tmem164, Flad1, Frmd4b, Marcksl1, Bhlhe22, Yif1b, Ttc38, Phyh, Mfsd6, Fam189b, Sned1, Cd3d, Slc25a29, Cd7, RGD1563281, Grb7, Ccdcl47, Stat2, Ppp3ca, LOC688241, Shisa4, Atp8b1, Cd3g, Txn14b, Ranbp10, Dynlt3, Atp2b2, Trub1, Zhx3, Rexo4, Snapc2, Rad541, Vps37c, Hmga1, Furin, Rab15, Folr1, Fundc1, Pak3, Aqp2, Hapln2, Tmem37, Gjd3, Lambl, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpd1l, Ankrd37, Cox4i2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, Vegfb, Lpl, Chaf1a, Srrd, Steapl, Fkbp11, Slc39a3, Bcan, Foxc2, Jph1, Ccl27, Pdia3, Gabrq, Surf6, Runx3, Abcb6, Cdk2, LOC687813 und Porcn.

6. Verfahren zur Herstellung einer Zusammensetzung zur oralen Einnahme mit immunstimulierender Wirkung, umfassend den Schritt des Hinzufügens von mRNA, deren Menge in Milch einer Ratte zunimmt, wenn der Ratte ein *Bifidobakterium* oral verabreicht wird, zu einer Grundlage für eine Zusammensetzung zur oralen Einnahme, wobei die mRNA ausgewählt ist aus der Gruppe bestehend aus Cyp24a1, Dnm1, Dpysl2, Fam89a, Arhgap22, Fbx15,Btg2, Csnk1g1, Mll1, Eml4, Nelf, Aldhll1, Boc, Usp43_predicted, Rassf5, Man2a1, Srgn, Gbfl, RGD1562551, Lrba, P2ry2, Bcl2l1, Rbm14, RGD1307365, Tecpr1, Kctd9, Pkig, RGD1308139, Klh135, Gab1, Arid5b, Mark, Trim47, Ddx21, Tiparp, Hspa1b, Nr3c2, Klf4, Dhx8, Myh14, Mcam, Dusp3, Acer2, L3mbtl3, Fcho2, Ash1l, Paxip1, Irgq, Plcb1, Sts, Zmym3, Anxa1, Rtn4ip1, Gngt1, Srcap, Otud4, RGD1307569, Adpgk, Plk3, Phf1, St14, N4bp1, Naaa, Fgf7, Lama2, Ahnak, Sec3l1, Mks1, Prodh, Baz1b, R3hdm1, Setd5, Rhebl1, Kdm5b, Fbxw8, Epb4.114a, Lpin2, Cxcl2, Nr2c2, Ckap4, Ankrd1, Ugcg, Fcgr2b, Sf3a1, Parg, Heatr6, Ctdspl, Tp53bp1, Fam60a, Pik3r2, Arhgef5, Nek7, Rp2, Vhl, Usp31, Camsap1, RGD1308759, Ldlrap1, Sp1, Mast4, Rela, Nhlrc3, Intu, Gcap14, Cdc42se2, Chd7, Rbms1, Invs, Kif2a, Exoc6, Zbtb1, Rnd3, Rb1cc1, Sphk1, Noxo1, Mllt6, Thumpd2, Ptpn4, Thex1, S100a8, Grk5, Trove2, Pcdh17, Med13, Fam82a2, Rab43, Clip1, Ankrd57, Criml, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargclb, Med1, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Mam11, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5, Map6, Angpt14, Dnmt3b, Gzf1, Plcb4, Ap4m1, Stat3, Rkhd1, Dcp2, Rtn4, Tlr3, Tubb6, Casp8, Nin, Fyn, Tmem77, Maml3, Slc15a4, Sqrdl, Ppp1r9b, Pafah1b1, Def8, Fam164a, Znf746, RGD1305235, Thada, Hip1, Pgrmc2, Sgk3, Baz1a, Foxn3, Map3k12, Efhd2, Pogz, Pcf11, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrp11, Sptbn1, Dtx31, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, Paqr5, Ddhd1, Kdm6b, Ube2d1, Rhoc, LOC361100, Cx3cl1, Fbxo42, Daam1, Klhl5, Fbxo30, Syt12, Hercl, Dab2ip, Tacc2, Sipa1l1, Ets2, Zbtb43, Fhdcl, Pdlim5, Stxbp5, Hdac8, Sestd1, Acot2, Fam117b, Nrip1, Aldh3a2, Exosc8, Shmt1, Lrrc57, Rell2, Zfyve16, Cebpd, Tnrc6b, Nsun6, Dennd5a, Fbxw5, Slc25a25, Dtwd1, Hspb1, Homer2, Nhej1, Etnk1, Nol9, Med23, Hup133, Rnd1, Rplp1, Ankfy1, Pik3r4, Plcd1, Cxcl12, Lix1l, Ralbp1, Cald1, LOC302495, Cdcp1, Ralb, Pcyt1a, Skil, Trim25, Pigv, Mark3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, Il17re, Eif4e3, Dock8, Nolc1, Sult1a1, Ankrd17, Trim2, Nfib, Cd14, S100a10, LOC687105, Ccdc123, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, Cdh1, Rnf19a, Pdlim1, Csnk1g3, Hectd1, Taf51, Noc3l, Mfat5, RGD1562529, Atl3, Ptpn9, Nedd4l, Wipi1, Gnal, Tle1, Pprc1, Src, Clic1, Secisbp21, Rnf103, Ogt, Zcchc2, Clasp1, Gtpbp2, Ift172, Map3k6, Plce1, Nedd4, Cpne3, Prim2, Trafd1, Myef2, Znf532, Tmem50b, I16ra, Pdcd6ip, Tpm4, Rnasen, Lpin1, Flot1, Cds1, Tnfaip1, Mudeng, Cdc215, Arid2, Znf654, Nat8b, Snx12, Ap1g1, Itsn2, RGD1306820, Mesdc1, Tmco6, Fkbp5, Prpf8, Pdcd11, Runx1, Epn2, Arid4b, Slk, Prkx, Dnajb3, Phf17, Fam193a, Pvr12, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klhl24, Cox11, Tfip11, Rcan1, Nagk, Myst3, Rbm28, Anapc7, Ezh1, Cyp27a1, Irs2, Sdccag1, Foxp1, Dusp18, Hcca2, Fbx18, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, Twf1, Rp18, Prdx1, Phrf1, Pcm1 und Mapk12.

7. Verfahren zur Herstellung einer Zusammensetzung zur oralen Einnahme mit immunsuppressiver Wirkung, umfassend den Schritt des Hinzufügens von mRNA, deren Menge in Milch einer Ratte abnimmt, wenn der Ratte ein *Bifidobakterium* oral verabreicht wird, zu einer Grundlage für eine Zusammensetzung zur oralen Einnahme, wobei die mRNA ausgewählt ist aus der Gruppe bestehend aus Slc1a4, Trim16, Cacnale, Gal, Arnt2, Stk11ip, Sdc3, Tp53i11, Mpz, Dnmt31, Ache, Cln5, Asb7, Rtp3, Peli2, Caskin2, Cass4, Plvap, Hist1h1a, Co122al, Htr6, Trim42, Ccdc89, Atp10d, Oprk1, Cyct,
Pdzd2, Sh2d5, Tmem136, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbp1, Bnc1, Col18a1, Traf3, Sema7a, Rcn3, Ift122, Smc2, Cldn18, LOC679462, Pogk, Abhd15, Pias4, Alg8, Nkx2-4, Mmp2, Iqcf1, B9d2, Fam107a, Rarres1, Wbp2, Wdr93, Clqtnf5, Egf17, Slc32a1, Id3, Ccdc58, Sox21, Tnfsf12, Qrich2, RGD1308930, B3galnt2, Tob2, Creb311, Klk12, Dqx1, Ncoa2, Slfnl1, Elf2, Gsx2, Hhip, Grm2, Fam170a, Gpc2, Npvf, Tsen54, Igfbp1, Zdhhc19, Csrnp2, Dcst2, Krt1, Nanos1, Cdh13, Ebi3, Prr3, Rnf43, Btbd17, Trim26, Gata3, Cdkn1c, Hrc, Grasp, Galm, Samd14, Oprd1, Pim1, I12rb, Ptpn13, Mtus1, Fh13, Hdhd2, Farp1, Ptprs, Hand1, Rp137a, Tcf15, Neurod2, Usp20, Foxo3, Aldh1l2, Nov, RGD1566130, Slc34a2, Gzma, Iqgap3, Enpp2, Tm7sf2, Exo1, Slc9a1, Rp137,Pde12, Eefsec, Dmrta2, LOC498685, Brunol5, Tmem164, Flad1, Frmd4b, Marcksl1, Bhlhe22, Yiflb, Ttc38, Phyh, Mfsd6, Fam189b, Sned1, Cd3d, Slc25a29, Cd7, RGD1563281, Grb7, Ccdc147, Stat2, Ppp3ca, LOC688241, Shisa4, Atp8b1, Cd3g, Txn14b, Ranbp10, Dynlt3, Atp2b2, Trub1, Zhx3, Rexo4, Snapc2, Rad541, Vps37c, Hmga1, Furin, Rab15, Folr1, Fundc1, Pak3, Aqp2, Hapln2, Tmem37, Gjd3, Lambl, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpd1l, Ankrd37, Cox4i2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, Vegfb, Lpl, Chaf1a, Srrd, Steap1, Fkbpl1, Slc39a3, Bcan, Foxc2, Jph1, Ccl27, Pdia3, Gabrq, Surf6, Runx3, Abcb6, Cdk2, LOC687813, und Porcn.

8. Verfahren nach Anspruch 6 oder 7, wobei die Zusammensetzung zur oralen Einnahme ein Nahrungsmittel für Säuglinge oder ein Nahrungsmittel für Kleinkinder ist.

9. Verfahren nach Anspruch 8, wobei das Nahrungsmittel für Säuglinge oder das Nahrungsmittel für Kleinkinder Säuglingsanfangsnahrung oder Folgenahrung ist.

## Revendications

1. Procédé de criblage d'un régime alimentaire ou d'une substance permettant la production de lait maternel ayant une activité immunostimulante comprenant une étape consistant à identifier un régime alimentaire ou une substance qui augmente ou diminue la quantité d'ARNm présente dans le lait d'un mammifère en utilisant la corrélation du profil d'expression d'ARNm dans le lait et du régime alimentaire ingéré par le mammifère ou une substance contenue dans ce régime alimentaire en tant qu'indicateur,
les profils d'expression d'ARNm comprenant la quantité d'ARNm choisis dans le groupe formé par les ARNm suivants :
Cyp24a1, Dnm1, Dpysl2, Fam89a, Arhgap22, Fbx15,Btg2, Csnk1g1, Mll1, Eml4, Nelf, Aldhll1, Boc, Usp43_predicted, Rassf5, Man2a1, Srgn, Gbf1, RGD1562551, Lrba, P2ry2, Bcl2ll, Rbm14, RGD1307365, Tecpr1, Kctd9, Pkig, RGD1308139, Klhl35, Gab1, Arid5b, Mafk, Trim47, Ddx21, Tiparp, Hspalb, Nr3c2, Klf4, Dhx8, Myh14, Mcam, Dusp3, Acer2, L3mbtl3, Fcho2, Ash1l, Paxip1, Irgq, Plcb1, Sts, Zmym3, Anxa1, Rtn4ip1, Gngt1, Srcap, Otud4, RGD1307569, Adpgk, Plk3, Phf1, St14, N4bp1, Naaa, Fgf7, Lama2, Ahnak, Sec311, Mks1, Prodh, Baz1b, R3hdm1, Setd5, Rhebl1, Kdm5b, Fbxw8, Epb4.114a, Lpin2, Cxcl2, Nr2c2, Ckap4, Ankrd1, Ugcg, Fcgr2b, Sf3al, Parg, Heatr6, Ctdspl, Tp53bp1, Fam60a, Pik3r2, Arhgef5, Nek7, Rp2, vhl, Usp31, Camsap1, RGD1308759, Ldlrap1, Sp1, Mast4, Rela, Nhlrc3, Intu, Gcap14, Cdc42se2, Chd7, Rbms1, Invs, Kif2a, Exoc6, Zbtb1, Rnd3, Rb1cc1, Sphk1, Noxo1, Mllt6, Thumpd2, Ptpn4, Thex1, S100a8, Grk5, Trove2, Pcdh17, Med13, Fam82a2, Rab43, Clip1, Ankrd57, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargc1b, Med1, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5,Map6, Angptl4, Dnmt3b, Gzf1, Plcb4, Ap4m1, Stat3, Rkhd1, Dcp2, Rtn4, Tlr3, Tubb6, Casp8, Nin, Fyn, Tmem77, Maml3, Slc15a4, Sqrdl, Ppplr9b, Pafah1b1, Def8, Fam164a, Znf746, RGD1305235, Thada, Hip1, Pgrmc2, Sgk3, Bazla, Foxn3, Map3k12, Efhd2, Pogz, Pcf11, Mcart1, Sox4, Calcocol, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrpll, Sptbn1, Dtx31, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, Paqr5, Ddhdl, Kdm6b, Ube2d1, Rhoc, LOC361100, Cx3cl1, Fbxo42, Daam1, Klhl5, Fbxo30, Syt12, Herc1, Dab2ip, Tacc2, Sipa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Rel12, Zfyve16, Cebpd, Tnrc6b, Nsun6, Dennd5a, Fbxw5, Slc25a25, Dtwd1, Hspb1, Homer2, Nhej1, Etnk1, Nol9, Med23, Nup133, Rnd1, Rplp1, Ankfy1, Pik3r4, Plcd1, Cxcl12, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, I117re, Eif4e3, Dock8, Nolc1, Sult1a1, Ankrd17, Trim2, Nfib, Cd14, S100a10, LOC687105, Ccdc123, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, Cdh1, Rnf19a, Pdlim1, Csnklg3, Hectd1, Taf51, Noc31, Nfat5, RGD1562529, Atl3, Ptpn9, Nedd4l, Wipi1, Gnal, Tle1, Pprc1, Src, Clic1, Secisbp21, Rnf103, Ogt, Zcchc2, Clasp1, Gtpbp2, Ift172, Map3k6, Plce1, Nedd4, Cpne3, Prim2, Trafd1, Myef2, Znf532, Tmem50b, Il6ra, Pdcd6ip, Tpm4, Rnasen, Lpin1, Flot1, Cds1, Tnfaip1, Mudeng, Cdc215, Arid2, Znf654, Nat8b, Snx12, Ap1g1, Itsn2, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klhl24, Cox11, Tfip11, Rcan1, Nagk, Myst3, Rbm28, Anapc7, Ezh1, Cyp27a1, Irs2, Sdccag1, Foxp1, Dusp18, Hcca2, Fbxl8, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, Twf1, Rp18, Prdx1, Phrf1, Pcm1, Mapk12, Slc1a4, Trim16, Cacnale, Gal, Arnt2, Stk11ip, Sdc3, Tp53i11, Mpz, Dnmt3l, Ache, Cln5, Asb7, Rtp3, Peli2, Caskin2, Cass4, Plvap, Hist1h1a, Col22a1, Htr6, Trim42, Ccdc89, Atp10d, Oprk1, Cyct, Pdzd2, Sh2d5, Tmem136, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbp1, Bnc1, Col18a1, Traf3, Sema7a, Rcn3, Ift122, Smc2, Cldn18, LOC679462, Pogk, Abhd15, Pias4, Alg8, Nkx2-4, Mmp2, Iqcf1, B9d2, Fam107a, Rarres1, Wbp2, Wdr93, C1qtnf5, Egfl7, Slc32a1, Id3, Ccdc58, Sox21, Tnfsf12, Qrich2, RGD1308930, B3galnt2, Tob2, Creb311, Klk12, Dqx1, Ncoa2, Slfnl1, Elf2, Gsx2, Hhip, Grm2, Fam170a, Gpc2, Npvf, Tsen54, Igfbp1, Zdhhc19, Csrnp2, Dcst2, Krt1, Nanos1, Cdh13, Ebi3, Prr3, Rnf43, Btbd17, Trim26, Gata3, Cdkn1c, Hrc, Grasp, Galm, Samd14, Oprd1, Pim1, Il2rb, Ptpn13, Mtus1, Fhl3, Hdhd2, Farp1, Ptprs, Hand1, Rpl37a, Tcf15, Neurod2, Usp20, Foxo3, Aldh112, Nov, RGD1566130, Slc34a2, Gzma, Iqgap3, Enpp2, Tm7sf2, Exo1, Slc9a1, Rpl37,Pde12, Eefsec, Dmrta2, LOC498685, Brunol5, Tmem164, Flad1, Frmd4b, Marcksl1, Bhlhe22, Yiflb, Ttc38, Phyh, Mfsd6, Fam189b, Sned1,Cd3d, Slc25a29, Cd7, RGD1563281, Grb7, Ccdc147, Stat2, Ppp3ca, LOC688241, Shisa4, Atp8b1, Cd3g, Txnl4b, Ranbp10, Dynlt3, Atp2b2, Trub1, Zhx3, Rexo4, Snapc2, Rad54l, Vps37c, Hmga1, Furin, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpd1l, Ankrd37, Cox4i2, Snrnp70, Rps6ka5, Tomm20, Rassf4, Hes1, Ctdspl, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, Vegfb, Lpl, Chafla, Srrd, Steap1, Fkbp11, Slc39a3, Bcan, Foxc2, Jph1, Ccl27, Pdia3, Gabrq, Surf6, Runx3, Abcb6, Cdk2, LOC687813, et Porcn, et
étant précisé que lorsque l'une ou les deux conditions suivantes est(sont) satisfaite(s), il est établi que le régime alimentaire ou la substance permet la production de lait maternel ayant une activité immunostimulante :
(A) l'ARNm est un ARNm dont la quantité dans le lait chez le rat augmente lorsqu'une bifidobactérie est administrée oralement au rat et lorsque les profils d'expression d'ARNm dans le lait observés avant et après l'ingestion du régime alimentaire sont comparés, la quantité d'au moins un type d'ARNm dans le lait observée après l'ingestion est égale au moins à 1,5 fois la quantité observée avant l'ingestion,
(B) l'ARNm est un ARNm dont la quantité dans le lait chez le rat diminue lorsqu'une bifidobactérie est administrée oralement au rat et lorsque les profils d'expression d'ARNm dans le lait observés avant et après l'ingestion du régime alimentaire sont comparés, la quantité d'au moins un type d'ARNm dans le lait observée après l'ingestion est égale à au plus 0,67 fois la quantité observée avant l'ingestion.

2. Procédé de criblage d'un régime alimentaire ou d'une substance permettant la production de lait maternel ayant une activité immuno-suppressive comprenant une étape consistant à identifier un régime alimentaire ou une substance qui augmente ou diminue la quantité d'ARNm présente dans le lait d'un mammifère en utilisant la corrélation du profil d'expression d'ARNm dans le lait et le régime alimentaire ingéré par le mammifère ou une substance renfermée dans le régime alimentaire en tant qu'indicateur,
les profils d'expression d'ARNm comprenant la quantité d'ARNm choisis dans le groupe formé par les ARNm suivants :
Cyp24a1, Dnm1, Dpysl2, Fam89a, Arhgap22, Fbxl5,Btg2, Csnk1g1, Mll1, Eml4, Nelf, Aldhll1, Boc, Usp43_predicted, Rassf5, Man2a1, Srgn, Gbf1, RGD1562551, Lrba, P2ry2, Bcl2l1, Rbm14, RGD1307365, Tecpr1, Kctd9, Pkig, RGD1308139, Klhl35, Gab1, Arid5b, Mafk, Trim47, Ddx21, Tiparp, Hspa1b, Nr3c2, Klf4, Dhx8, Myh14, Mcam, Dusp3, Acer2, L3mbtl3, Fcho2, Ash1l, Paxip1, Irgq, Plcb1, Sts, Zmym3, Anxa1, Rtn4ip1, Gngt1, Srcap, Otud4, RGD1307569, Adpgk, Plk3, Phf1, St14, N4bp1, Naaa, Fgf7, Lama2, Ahnak, Sec3l1, Mks1, Prodh, Baz1b, R3hdm1, Setd5, Rhebl1, Kdm5b, Fbxw8, Epb4.1l4a, Lpin2, Cxcl2, Nr2c2, Ckap4, Ankrd1, Ugcg, Fcgr2b, Sf3a1, Parg, Heatr6, Ctdspl, Tp53bp1, Fam60a, Pik3r2, Arhgef5, Nek7, Rp2, Vhl, Usp31, Camsap1, RGD1308759, Ldlrap1, Sp1, Mast4, Rela, Nhlrc3, Intu, Gcap14, Cdc42se2, Chd7, Rbms1, Invs, Kif2a, Exoc6, Zbtb1, Rnd3, Rb1cc1, Sphk1, Noxo1, Mllt6, Thumpd2, Ptpn4, Thex1, S100a8, Grk5, Trove2, Pcdh17, Med13, Fam82a2, Rab43, Clip1, Ankrd57, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargc1b, Med1, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5,Map6, Angptl4, Dnmt3b, Gzf1, Plcb4, Ap4m1, Stat3, Rkhd1, Dcp2, Rtn4, Tlr3, Tubb6, Casp8, Nin, Fyn, Tmem77, Maml3, Slc15a4, Sqrdl, Ppplr9b, Pafah1b1, Def8, Fam164a, Znf746, RGD1305235, Thada, Hip1, Pgrmc2, Sgk3, Baz1a, Foxn3, Map3k12, Efhd2, Pogz, Pcf11, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrpll, Sptbn1, Dtx3l, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, Paqr5, Ddhd1, Kdm6b, Ube2d1, Rhoc, LOC361100, Cx3cl1, Fbxo42, Daam1, Klhl5, Fbxo30, Syt12, Herc1, Dab2ip, Tacc2, Sisa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Rell2, Zfyve16, Cebpd, Tnrc6b, Nsun6, Dennd5a, Fbxw5, Slc25a25, Dtwd1, Hspb1, Homer2, Nhej1, Etnk1, Nol9, Med23, Nup133, Rnd1, Rplp1, Ankfy1, Pik3r4, Plcd1, Cxcl12, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, I117re, Eif4e3, Dock8, Nolc1, Sult1a1, Ankrd17, Trim2, Nfib, Cd14, S100a10, LOC687105, Ccdc123, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, Cdh1, Rnf19a, Pdlim1, Csnk1g3, Hectd1, Taf5l, Noc3l, Nfat5, RGD1562529, Atl3, Ptpn9, Nedd4l, Wipi1, Gnal, Tle1, Pprc1, Src, Clic1, Secisbp2l, Rnf103, Ogt, Zcchc2, Clasp1, Gtpbp2, Ift172, Map3k6, Plce1, Nedd4, Cpne3, Prim2, Trafd1, Myef2, Znf532, Tmem50b, Il6ra, Pdcd6ip, Tpm4, Rnasen, Lpin1, Flot1, Cds1, Tnfaip1, Mudeng, Cdc215, Arid2, Znf654, Nat8b, Snx12, Ap1g1, Itsn2, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klhl24, Cox11, Tfip11, Rcan1, Nagk, Myst3, Rbm28, Anapc7, Ezh1, Cyp27a1, Irs2, Sdccag1, Foxp1, Dusp18, Hcca2, Fbx18, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, Twf1, Rp18, Prdx1, Phrf1, Pcm1, Mapk12, Slc1a4, Trim16, Cacna1e, Gal, Arnt2, Stk11ip, Sdc3, Tp53i11, Mpz, Dnmt31, Ache, Cln5, Asb7, Rtp3, Peli2, Caskin2, Cass4, Plvap, Hist1h1a, Col22a1, Htr6, Trim42, Ccdc89, Atp10d, Oprk1, Cyct, Pdzd2, Sh2d5, Tmcm136, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbp1, Bnc1, Col18a1, Traf3, Sema7a, Rcn3, Ift122, Smc2, C1dn18, LOC679462, Pogk, Abhd15, Pias4, Alg8, Nkx2-4, Mmp2, Iqcf1, B9d2, Fam107a, Rarres1, Wbp2, Wdr93, Clqtnf5, Egf17, Slc32a1, Id3, Ccdc58, Sox21, Tnfsf12, Qrich2, RGD1308930, B3galnt2, Tob2, Creb3l1, Klk12, Dqx1, Ncoa2, Slfnl1, Elf2, Gsx2, Hhip, Grm2, Fam170a, Gpc2, Npvf, Tsen54, Igfbp1, Zdhhc19, Csrnp2, Dcst2, Krt1, Nanos1, Cdh13, Ebi3, Prr3, Rnf43, Btbd17, Trim26, Gata3, Cdkn1c, Hrc, Grasp, Galm, Samdl4, Oprd1, Pim1, Il2rb, Ptpn13, Mtus1, Fhl3, Hdhd2, Farp1, Ptprs, Hand1, Rpl37a, Tcf15, Neurod2, Usp20, Foxo3, Aldh1l2, Nov, RGD1566130, Slc34a2, Gzma, Iqgap3, Enpp2, Tm7sf2, Exo1, Slc9a1, Rpl37,Pde12, Eefsec, Dmrta2, LOC498685, Brunol5, Tmem164, Flad1, Frmd4b, Marcksl1, Bhlhe22, Yiflb, Ttc38, Phyh, Mfsd6, Fam189b, Sned1, Cd3d, Slc25a29, Cd7, RGD1563281, Grb7, Ccdc147, Stat2, Ppp3ca, LOC688241, Shisa4, Atp8b1, Cd3g, Txnl4b, Ranbp10, Dynlt3, Atp2b2, Trub1, Zhx3, Rexo4, Snapc2, Rad54l, Vps37c, Hmga1, Furin, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpd1l, Ankrd37, Cox4i2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, Vegfb, Lpl, Chafla, Srrd, Steap1, Fkbp11, Slc39a3, Bcan, Foxc2, Jph1, Ccl27, Pdia3, Gabrq, Surf6, Runx3, Abcb6, Cdk2, LOC687813, et Porcn, et
étant précisé que lorsque l'une ou les deux conditions ci-dessous est(sont) satisfaite(s), il est établi que le régime alimentaire ou la substance permet la production de lait maternel ayant une activité immuno-suppressive :
(a) l'ARNm est un ARNm dont la quantité dans le lait chez le rat augmente lorsqu'une bifido bactérie est administrée au rat par voie orale et lorsque les profils d'expression d'ARNm dans le lait observés avant et après l'ingestion du régime alimentaire sont comparés, la quantité d'au moins un type d'ARNm dans le lait observée après l'ingestion est égale à au plus 0,67 fois la quantité observée avant l'ingestion,
(b) l'ARNm est un ARNm dont la quantité dans le lait chez le rat diminue lorsqu'une bifidobactérie est administrée au rat par voie orale et lorsque les profils d'expression d'ARNm dans le lait observés avant et après l'ingestion du régime alimentaire sont comparés, la quantité d'au moins un type d'ARNm dans le lait observée après l'ingestion est égale à au moins 1,5 fois la quantité observée avant l'ingestion.

3. Procédé conforme à la revendication 1 ou 2,
selon lequel le mammifère est un humain, un rat ou un bovin.

4. Procédé conforme à l'une quelconque des revendications 1 à 3, selon lequel l'ARNm dont la quantité dans le lait chez le rat augmente lorsqu'une bifidobactérie est administrée au rat par voie orale est choisi dans le groupe formé par les ARNm suivants : Cyp24a1 Dnm1, Dpysl2, Fam89a, Arhgap22, Fbxl5,Btg2, Csnk1g1, Ml11, Eml4, Nelf, Aldh1l1, Boc, Usp43_predicted, Rassf5, Man2a1, Srgn, Gbf1, RGD1562551, Lrba, P2ry2, Bel2l1, Rbm14, RGD1307365, Tecpr1, Kctd9, Pkig, RGD1308139, Klhl35, Gab1, Arid5b, Mafk, Trim47, Ddx21, Tiparp, Hspalb, Nr3c2, K1f4, Dhx8, Myh14, Mcam, Dusp3, Acer2, L3mbt13, Fcho2, Ash1l, Paxip1, Irgq, Plcb1, Sts, Zmym3, Anxa1, Rtn4ip1, Gngt1, Srcap, Otud4, RGD1307569, Adpgk, Plk3, Phf1, St14, N4bp1, Naaa, Fgf7, Lama2, Ahnak, Sec3l1, Mks1, Prodh, Baz1b, R3hdm1, Setd5, Rhebl1, Kdm5b, Fbxw8, Epb4.1l4a, Lpin2, Cxc12, Nr2c2, Ckap4, Ankrd1, Ugcg, Fcgr2b, Sf3a1, Parg, Heatr6, Ctdspl, Tp53bp1, Fam60a, Pik3r2, Arhgef5, Nek7, Rp2, Vhl, Usp31, Camsap1, RGD1308759, Ldlrap1, Sp1, Mast4, Rela, Nhlrc3, Intu, Gcap14, Cdc42se2, Chd7, Rbms1, Invs, Kif2a, Exoc6, Zbtb1, Rnd3, Rb1cc1, Sphk1, Noxo1, Mllt6, Thumpd2, Ptpn4, Thex1, S100a8, Grk5, Trove2, Pcdh17, Med13, Fam82a2, Rab43, Clip1, Ankrd57, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargc1b, Med1, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5,Map6, Angptl4, Dnmt3b, Gzf1, Plcb4, Ap4m1, Stat3, Rkhd1, Dcp2, Rtn4, Tlr3, Tubb6, Casp8, Nin, Fyn, Tmem77, Maml3, Slc15a4, Sqrdl, Ppplr9b, Pafah1b1, Def8, Fam164a, Znf746, RGD1305235, Thada, Hip1, Pgrmc2, Sgk3, Baz1a, Foxn3, Map3k12, Efhd2, Pogz, Pcf11, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrp11, Sptbn1, Dtx31, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgapl2, Dapk2, Nup160, Eea1, LOC363188, Paqr5, Ddhd1, Kdm6b, Ube2d1, Rhoc, LOC361100, Cx3cl1, Fbxo42, Daam1, Klhl5, Fbxo30, Syt12, Herc1, Dab2ip, Tacc2, Sipa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Rell2, Zfyve16, Cebpd, Tnrc6b, Nsun6, Dennd5a, Fbxw5, Slc25a25, Dtwd1, Hspb1, Homer2, Nhej1, Etnk1, Nol9, Med23, Nup133, Rnd1, Rplp1, Ankfyl, Pik3r4, Plcd1, Cxcl12, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, Il17re, Eif4e3, Dock8, Nolc1, Sult1a1, Ankrd17, Trim2, Nfib, Cd14, S100a10, LOC687105, Ccdc123, Pols, Aplf, Gulp1, Slc40a1, Tfdp2, Dyrk2, Anxa5, Nr3c1, RGD1306862, Rkhd2, Cdc42bpb, Numa1, Mocos, Mafb, Akap11, Diaph1, Znrf1, Sos1, Rhob, Pkp2, Srf, Cdh1, Rnf19a, Pdlim1, Csnklg3, Hectd1, Taf51, Noc3l, Nfat5, RGD1562529, Atl3, Ptpn9, Nedd4l, Wipi1, Gnal, Tle1, Pprcl, Src, Clic1, Secisbp2l, Rnf103, Ogt, Zcchc2, Clasp1, Gtpbp2, Ift172, Map3k6, Plce1, Nedd4, Cpne3, Prim2, Trafd1, Myef2, Znf532, Tmem50b, Il6ra, Pdcd6ip, Tpm4, Rnasen, Lpin1, Flot1, Cds1, Tnfaip1, Mudeng, Cdc215, Arid2, Znf654, Nat8b, Snx12, Ap1g1, Itsn2, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1, Setd2, Efr3a, Tnk1, Ncor1, Coq4, Klhl24, Cox11, Tfip11, Rcan1, Nagk, Myst3, Rbm28, Anapc7, Ezh1, Cyp27a1, Irs2, Sdccag1, Foxp1, Dusp18, Hcca2, Fbx18, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, Twf1, Rp18, Prdx1, Phrf1, Pcm1, et Mark12.

5. Procédé conforme à l'une quelconque des revendications 1 à 3, selon lequel l'ARNm dont la quantité dans le lait chez le rat diminue lorsqu'une bifidobactérie est administrée au rat par voie orale est choisi dans le groupe formé par les ARNm suivants : S1c1a4,
Trim16, Cacnale, Gal, Arnt2, Stkllip, Sdc3, Tp53i11, Mpz, Dnmt3l, Ache, Cln5, Asb7, Rtp3, Peli2, Caskin2, Cass4, Plvap, Hist1h1a, Col22a1, Htr6, Trim42, Ccdc89, Atp10d, Oprk1, Cyct, Pdzd2, Sh2d5, Tmem136, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbpl, Bnc1, Col18a1, Traf3, Sema7a, Rcn3, Ift122, Smc2, Cldn18, LOC679462, Pogk, Abhd15, Pias4, Alg8, Nkx2-4, Mmp2, Iqcf1, B9d2, Fam107a, Rarres1, Wbp2, Wdr93, C1qtnf5, Egfl7, Slc32a1, Id3, Ccdc58, Sox21, Tnfsf12, Qrich2, RGD1308930, B3galnt2, Tob2, Creb311, Klk12, Dqx1, Ncoa2, Slfnl1, Elf2, Gsx2, Hhip, Grm2, Fam170a, Gpc2, Npvf, Tsen54, Igfbp1, Zdhhc19, Csrnp2, Dcst2, Krt1, Nanos1, Cdh13, Ebi3, Prr3, Rnf43, Btbd17, Trim26, Gata3, Cdkn1c, Hrc, Grasp, Galm, Samd14, Oprd1, Pim1, I12rb, Ptpn13, Mtus1, Fhl3, Hdhd2, Farp1, Ptprs, Hand1, Rpl37a, Tcf15, Neurod2, Usp20, Foxo3, Aldh1l2, Nov, RGD1566130, Slc34a2, Gzma, Iqgap3, Enpp2, Tm7sf2, Exol, Slc9a1, Rpl37,Pde12, Eefsec, Dmrta2, LOC498685, Brunol5, Tmem164, Flad1, Frmd4b, Marcksl1, Bhlhe22, Yiflb, Ttc38, Phyh, Mfsd6, Fam189b, Sned1,Cd3d, Slc25a29, Cd7, RGD1563281, Grb7, Ccdc147, Stat2, Ppp3ca, LOC688241, Shisa4, Atp8b1, Cd3g, Txnl4b, Ranbp10, Dynlt3, Atp2b2, Trub1, Zhx3, Rexo4, Snapc2, Rad54l, Vps37c, Hmga1, Furin, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpd1l, Ankrd37, Cox4i2, Snrnp70, Rps6ka5, Tomm20, Rassf4, Hes1, Ctdspl, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, Vegfb, Lpl, Chafla, Srrd, Steap1, Fkbp11, Slc39a3, Bcan, Foxc2, Jph1, Ccl27, Pdia3, Gabrq, Surf6, Runx3, Abcb6, Cdk2, LOC687813, et Porcn.

6. Procédé de production d'une composition destinée à l'ingestion par voie orale ayant une activité immunostimulante, comprenant une étape consistant à ajouter un ARNm dont la quantité dans le lait chez le rat augmente lorsqu'une bifidobactérie est administrée au rat par voie orale à la base de la composition destinée à l'ingestion par voie orale, l'ARNm étant choisi dans le groupe formé par les ARNm suivants :
Cyp24a1, Dnm1, Dpysl2, Fam89a, Arhgap22, Fbxl5,Btg2, Csnk1g1, Mll1, Eml4, Nelf, Aldh1l1, Boc, Usp43_predicted, Rassf5, Man2a1, Srgn, Gbf1, RGD1562551, Lrba, P2ry2, Bcl211, Rbm14, RGD1307365, Tecpr1, Kctd9, Pkig, RGD1308139, Klhl35, Gab1, Arid5b, Mafk, Trim47, Ddx21, Tiparp, Hspalb, Nr3c2, Klf4, Dhx8, Myh14, Mcam, Dusp3, Acer2, L3mbtl3, Fcho2, Ash1l, Paxip1, Irgq, Plcb1, Sts, Zmym3, Anxa1, Rtn4ip1, Gngt1, Srcap, Otud4, RGD1307569, Adpgk, Plk3, Phf1, St14, N4bp1, Naaa, Fgf7, Lama2, Ahnak, Sec3l1, Mks1, Prodh, Baz1b, R3hdm1, Setd5, Rhebl1, Kdm5b, Fbxw8, Epb4.1l4a, Lpin2, Cxrl2, Nr2c2, Ckap4, Ankrd1, Ugcg, Fcgr2b, Sf3a1, Parg, Heatr6, Ctdspl, Tp53bp1, Fam60a, Pik3r2, Arhgef5, Nek7, Rp2, Vhl, Usp31, Camsap1, RGD1308759, Ldlrap1, Sp1, Mast4, Rela, Nhlrc3, Intu, Gcap14, Cdc42se2, Chd7, Rbms1, Invs, Kif2a, Exoc6, Zbtb1, Rnd3, Rb1cc1, Sphk1, Noxo1, Mllt6, Thumpd2, Ptpn4, Thex1, S100a8, Grk5, Trove2, Pcdh17, Med13, Fam82a2, Rab43, Clip1, Ankrd57, Crim1, Celsr2, Idh1, Lrrc61, Aff4, Arhgap1, Itpr3, Lhfp, Dnajc12, Garnl1, Gtf3c5, Hspb8, Chac1, Pof1b, Ptpre, Arl6ip1, Son, Ppargc1b, Med1, Zbtb39, Urb1, Wdr89, Josd1, Pank4, Ska2, Rrm1, Klf11, Phc1, Birc6, Cask, Mesdc2, St5, Smyd5, Heca, Plagl2, Strn4, Ftsjd1, Maml1, RGD1561817, Anxa8, Nup153, Anxa2, Trappc2, Gpr155, Zfhx3, Morc2, Lrsam1, Polg, RGD1311307, Kcnk4, Tmcc3, Pde4b, Chn1, Dusp16, Pex5,Map6, Angptl4, Dnmt3b, Gzf1, Plcb4, Ap4m1, Stat3, Rkhd1, Dcp2, Rtn4, Tlr3, Tubb6, Casp8, Nin, Fyn, Tmem77, Maml3, Slc15a4, Sqrdl, Ppplr9b, Pafah1b1, Def8, Fam164a, Znf746, RGD1305235, Thada, Hip1, Pgrmc2, Sgk3, Baz1a, Foxn3, Map3k12, Efhd2, Pogz, Pcf11, Mcart1, Sox4, Calcoco1, Myom1, Mtmr9, Akap13, Rhobtb1, Matr3, LOC684297, Rxrb, Mars2, Abcb8, Bik, Med22, Cryab, Cpt1a, Cnksr1, Kirrel3, Arl13a, Sept8, Rb1, Prmt2, Ccnb3, Zhx2, Pds5b, Hdac6, Prdm4, Smarcad1, Usp9x, Sdc1, Arhgap28, Rad17, Sorbs1, Tp63, Wdr48, Hnrpll, Sptbn1, Dtx3l, Cnnm2, Fbln5, Usp48, Tgm2, RGD1564776, RGD1563106, Taok1, Snx30, Utp6, Cytip, Rest, Eif4g3, Adnp2, Wdr1, Hiat1, Ddx58, RGD1359600, Arhgap12, Dapk2, Nup160, Eea1, LOC363188, Paqr5, Ddhd1, Kdm6b, Ube2d1, Rhoc, LOC361100, Cx3cl1, Fbxo42, Daam1, Klh15, Fbxo30, Syt12, Herc1, Dab2ip, Tacc2, Sipa1l1, Ets2, Zbtb43, Fhdc1, Pdlim5, Stxbp5, Hdac8, Sestd1, Acot2, Fam117b, Nrip1, Aldh3a2, Exosc8, Shmt1, Lrrc57, Rell2, Zfyve16, Cebpd, Tnrc6b, Nsun6, Dennd5a, Fbxw5, Slc25a25, Dtwd1, Hspb1, Homer2, Nhej1, Etnk1, Nol9, Med23, Nup133, Rnd1, Rplp1, Ankfy1, Pik3r4, Plcd1, Cxcl12, Lix1l, Ralbp1, Cald1, LOC302495, Cdcp1, Ralb, Pcyt1a, Skil, Trim25, Pigv, Mapk3, Pard6g, Klf7, Pxn, Dnaja4, Cnot1, Capn2, Birc3, Frmd8, Chd2, Prpf39, Taf5, Eif4enif1, Pkn2, Fam179b, Kdm2a, Gda, Ccdc21, I117re, Eif4e3, Dock8, Nolc1, Sult1a1, Ankrd17, Trim2, Nfib, Cd14, S100a10, LOC687105, Ccdc123, Pols, Aplf, Gulpl, Slc40a1, Tfdp2, Dyrk2, Anxa5, Nr3c1, RGD1306862, Rkhd2, Cdc42bpb, Numa1, Mocos, Mafb, Akapll, Diaphl, Znrf1, Sos1, Rhob, Pkp2, Srf, Cdh1, Rnf19a, Pdlim1, Csnklg3, Hectd1, Taf51, Noc31, Nfat5, RGD1562529, Atl3, Ptpn9, Nedd41, Wipi1, Gnal, Tle1, Pprc1, Src, Clic1, Secisbp21, Rnf103, Ogt, Zcchc2, Clasp1, Gtpbp2, Ift172, Map3k6, Plce1, Nedd4, Cpne3, Prim2, Trafd1, Myef2, Znf532, Tmem50b, I16ra, Pdcd6ip, Tpm4, Rnasen, Lpin1, Flot1, Cds1, Tnfaip1, Mudeng, Cdc215, Arid2, Znf654, Nat8b, Snx12, Aplg1, Itsn2, RGD1306820, Mesdc1, Tmco6, Fkbp5, Prpf8, Pdcd11, Runx1, Epn2, Arid4b, Slk, Prkx, Dnajb3, Phf17, Fam193a, Pvrl2, Tep1, Atxn7, Wwtr1, LOC313672, Mus81, C1d, Arid4a, Ehd1, Usp32, Carhsp1, Acss1, Ctcf, Bag3, Mtrr, Ywhah, Csrp1, Smc3, Sorbs2, RGD1309762, Ptpn1 Setd2, Efr3a, Tnk1, Ncor1, Coq4, Klhl24, Cox11, Tfip11, Rcan1, Nagk, Myst3, Rbm28, Anapc7, Ezh1, Cyp27a1, Irs2, Sdccag1, Foxp1, Dusp18, Hcca2, Fbxl8, Chd1, Tp53bp2, RGD1310552, Kif5b, Pcaf, Slc25a42, Ocln, RGD1559961, Kifap3, Wtip, Gapvd1, Ankrd42, Supt4h1, Haus1, Prdm2, Arvcf, Gtf2h1, Rassf6, Pcif1, Arap2, Ube2k, Twf1, Rpl8, Prix1, Phrf1, Pcm1, et Mapk12.

7. Procédé de production d'une composition destinée à l'ingestion par voie orale ayant une activité immunosuppressive comprenant une étape consistant à ajouter un ARNm dont la quantité dans le lait chez le rat diminue lorsqu'une bifidobactérie est administrée au rat par voie orale à la base de la composition destinée à l'ingestion par voie orale, l'ARNm étant choisi dans le groupe formé par les ARNm suivants :
Slc1a4, Trim16, Cacnale, Gal, Arnt2, Stk11ip, Sdc3, Tp53i11, Mpz, Dnmt31, Ache, Cln5, Asb7, Rtp3, Peli2, Caskin2, Cass4, Plvap, Hist1h1a, Col22a1, Htr6, Trim42, Ccdc89, Atp10d, Oprk1, Cyct, Pdzd2, Sh2d5, Tmem136, Gh1, Ttl, Pgbd5, Zfp583, Ankrd2, Avil, Mcm3ap, Mypn, Frmd4a, Rnf182, Kcng1, Paqr7, Pkp1, Csrnp1, Siae, Hoxb6, Pga5, Hoxd9, RGD1308541, Cyth3, Fbp1, Bnc1, Col18a1, Traf3, Sema7a, Rcn3, Ift122, Smc2, Cldn18, LOC679462, Pogk, Abhd15, Pias4, Alg8, Nkx2-4, Mmp2, Iqcf1, B9d2, Fam107a, Rarres1, Wbp2, Wdr93, C1qtnf5, Egfl7, Slc32a1, Id3, Ccdc58, Sox21, Tnfsf12, Qrich2, RGD1308930, B3galnt2, Tob2, Creb311, Klk12, Dqx1, Ncoa2, Slfnl1, Elf2, Gsx2, Hhip, Grm2, Fam170a, Gpc2, Npvf, Tsen54, Igfbp1, Zdhhcl9, Csrnp2, Dcst2, Krt1, Nanos1, Cdh13, Ebi3, Prr3, Rnf43, Btbd17, Trim26, Gata3, Cdkn1c, Hrc, Grasp, Galm, Samd14, Oprd1, Pim1, Il2rb, Ptpn13, Mtus1, Fhl3, Hdhd2, Farp1, Ptprs, Hand1, Rpl37a, Tcf15, Neurod2, Usp20, Foxo3, Aldh1l2, Nov, RGD1566130, Slc34a2, Gzma, Iqgap3, Enpp2, Tm7sf2, Exo1, Slc9a1, Rpl37,Pde12, Eefsec, Dmrta2, LOC498685, Brunol5, Tmem164, Flad1, Frmd4b, Marcksl1, Bhlhe22, Yiflb, Ttc38, Phyh, Mfsd6, Fam189b, Sned1, Cd3d, Slc25a29, Cd7, RGD1563281, Grb7, Ccdc147, Stat2, Ppp3ca, LOC688241, Shisa4, Atp8b1, Cd3g, Txnl4b, Ranhp10, Dynlt3, Atp2b2, Trub1, Zhx3, Rexo4, Snapc2, Rad541, Vps37c, Hmga1, Furin, Rab15, Folr1, Fundc1, Pak3, Aqp2, Hapln2, Tmem37, Gjd3, Lamb1, Cox5a, Cfd, Jak3, Magt1, Sc4mol, Uros, Calca, Abca7, Pm20d2, Dpf2, Tmprss4, Znf467, Btbd9, Rasgrf1, RGD1310270, Adam15, Kif15, Plekho2, Atp2c2, Rps25, Lipa, Cpeb2, Znf575, Arhgap26, LOC311352, Znrf4, Msh5, Tmed7, Gpd1l, Ankrd37, Cox4i2, Snrnp70, Rps6ka5, Tomm20, Rassf4, Hes1, Ctdsp1, Pomc, Inpp4b, Nfix, Ydjc, Cst3, Tesk2, Tcn2, RGD1309847, Artn, Wibg, Prpf4, LOC685179, Igsf3, Got1, Lcp2, Agrn, Cited2, Bat4, RGD1308059, Sft2d1, Ubac2, Scly, Wrb, Zfp161, Fads2, Fbxo8, Vegfb, Lpl, Chafla, Srrd, Steap1, Fkbp11, Slc39a3, Bcan, Foxc2, Jph1, Ccl27, Pdia3, Gabrq, Surf6, Runx3, Abcb6, Cdk2, LOC687813, et Porcn.

8. Procédé conforme à l'une des revendications 6 ou 7 selon lequel la composition destinée à l'ingestion par voie orale est un aliment destiné à des bébés ou à des enfants en bas âge.

9. Procédé conforme à la revendication 8,
selon lequel l'aliment pour bébé ou l'aliment pour enfant en bas âge est une formulation pour bébé ou une formulation pour enfant d'âge plus avancé.
